# EUROPEAN PATENT SPECIFICATION

(11) **EP 2 224 010 B1**
(45) Date of publication and mention of the grant of the patent: **20.06.2012**
(21) Application number: 10155532.4
(22) Date of filing: 05.04.2002
(51) Int. Cl.: C12N 15/85, C12N 15/11

(54) **Improved gene expression**
Gesteigerte Genexpression
Expression de gène modifiée

(30) Priority: 05.04.2001 US 281605 P; 15.06.2001 US 298675 P; 05.04.2001 GB 0108605; 17.04.2001 GB 0109335
(43) Date of publication of application: 01.09.2010
(62) Divisional of application: 02708511.7
(73) Proprietor: EMD Millipore Corporation, Billerica, MA 01821 (US)
(72) Inventor: Williams, Stephen Geraint, Keele, Staffordshire ST5 5SP (GB); Crombie, Robert Lachlan, Keele, Staffordshire ST5 5SP (GB)
(74) Representative: Harrison Goddard Foote

(56) References cited:
- WO-A-00/05393
- ORTIZ B D ET AL: "ADJACENT DNA ELEMENTS DOMINANTLY RESTRICT THE UBIQUITOUS ACTIVITY OF A NOVEL CHROMATIN-OPENING REGION TO SPECIFIC TISSUES" 15 August 1997 (1997-08-15), EMBO JOURNAL, OXFORD UNIVERSITY PRESS, SURREY, GB, VOL. 16, NR. 16, PAGE(S) 5037-5045 , XP000867213 ISSN: 0261-4189
- ZHUMA T ET AL: "HUMAN HMG BOX TRANSCRIPTION FACTOR HBP1: A ROLE IN HCD2 LCR FUNCTION" 15 November 1999 (1999-11-15), EMBO JOURNAL, OXFORD UNIVERSITY PRESS, SURREY, GB, VOL. 18, NR. 22, PAGE(S) 6396-6406 , XP000867214 ISSN: 0261-4189

## Description

### FIELD OF THE INVENTION

The present invention relates to a polynucleotide comprising a ubiquitous chromatin-opening element (UCOE) together with a selectable marker element. When operably linked to, and flanking, an expressible nucleic acid sequence, the combination of elements provides high and reproducible levels of gene expression. The present invention also relates to a vector comprising the polynucleotide sequence, a host cell comprising the vector and use of the polynucleotide, vector or host cell in therapy, or for applications involving protein expression in cell culture.

### BACKGROUND OF THE INVENTION

The current model of chromatin structure in higher eukaryotes postulates that genes are organised in "domains" (Dillon, N. & Grosveld, F. Chromatin domains as potential units of eukaryotic gene function. Curr. Opin. Genet. Dev. 4, 260-264 (1994); Higgs, D.R. Do LCRs open chromatin domains? Cell 95, 299-302 (1998)) Chromatin domains are envisaged to exist in either a condensed, "closed", transcriptionally silent state, or in a de-condensed, "open" and transcriptionally competent configuration. The establishment of an open chromatin structure characterised by increased DNasel sensitivity, DNA hypomethylation and histone hyperacetylation, is considered a prerequisite to the commencement of gene expression.

The open and closed nature of chromatin regions is reflected in the behaviour of transgenes that are randomly Integrated Into the host cell genome. Identical constructs give different patterns of tissue-specific and development stage-specific expression when integrated at different locations in the mouse genome (Palmiter, R.D. & Brinster, R.L. Ann. Ref. Genet. 20, 465-499 (1986); Allen, N.D. et al. Nature 333, 852-855 (1988); Bonnerot, C., Grimber, G., Briand, P. & Nicolas, J.F. Proc. Natl. Acad. Sci. USA 87:6331-6335 (1990)).

A variegated expression pattern within a given transgenic mouse tissue, known as position effect variegation (PEV), is also frequently observed (Kioussis, D. & Festensteln, R. Curr. Opin. Genet. Dev. 7, 614-619 (1997)). When exogenous genes are Integrated into the chromosome of mammalian cells cultures *In vitro,* many of the integration events result in rapid silencing of the transgene and the remainder give large variability In expression levels (Pikaart, M.J., Recillas-Targa, F. & Felsenfield, G. Genes Dev. 12, 2852-2862 (1998); Fussenegger, M., Bailey, J.E., Hauser, H. & Mueller, P.P Trends Biotech. 17, 35-42 (1999)), These position effects render transgene expression inefficient, with implication for both basic research and biotechnology applications.

The chromatin domain model of gene organisation suggests that genetic control elements that are able to establish and maintain a transcriptionally competent open chromatin structure should be associated with active regions of the genome.

Locus Control Regions (LCRs) are a class of transcriptional regulatory elements with long-range chromatin remodelling capability. LCRs are functionally defined in transgenic mice by their ability to confer site-of integration independent, transgene copy number-dependent, physiological levels of expression on a gene linked in *cis*., especially single copy transgenes Fraser, P. & Grosveld, F. Curr. Opin. Cell Biol. 10, 361-385 (1998); Li, Q., Harju, S. & Peterson, K.R. Trends Genet. 15: 403-408 (1999). Crucially, such expression is tissue-specific. LCRs are able to obstruct the spread of heterochromatin, prevent PEV (Kioussis, D. & Festenstein, R. Curr. Opin. Genet. Dev. 7, 614-619 (1997)) and consist of a series of DNase I hypersensitive (HS) sites which can be located either 5' or 3' of the genes that they regulate (LI, Q., Harju, S. & Peterson, K.R. Trends Genet. 15: 403-408 (1999)).

LCRs appear to be comprised of two separate, although not necessarily independent components. First, the establishment of an 'open chromatin domain', and second a dominant transcriptional activation capacity to confer transgene copy number dependent expression (Fraser, P. & Grosveld, F. Curr. Opin. Cell Biol. 10, 361-365 (1998). The molecular mechanisms by which LCRs exert their function remain a point of contention (Higgs, D.R. Cell 95, 299-302 (1998); Bulger, M. & Groudine, M. Genes Dev. 13, 2465-2477 (1999); Grosveld, F. Curr. Opin. Genet. Dev. 9 152-157 (1999); Bender, M.A., Bulger, M., Close, J. & Groudine, M.. Mol. Cell 5, 387-393 (2000).

The generation of cultured mammalian cell lines producing high levels of a therapeutic protein product is a major developing industry. Chromatin position effects make it a difficult, time consuming and expensive process. The most commonly used approach to the production of such mammalian "cell factories" relies on gene amplification induced by a combination of a drug resistance gene (e.g., DHFR, glutamine synthetase (Kaufman RJ. Methods Enzymol 185, 537-566 1990)), and the maintenance of stringent selective pressure. The use of vectors containing LCRs from highly expressed gene domains, using cells derived from the appropriate tissue, greatly simplifies the procedure, giving a large proportion of clonal cell lines showing stable high levels of expression (Needham M, Gooding C, Hudson K, Antoniou M, Grosfeld F and Hollis M. Nucleic Acids Res 20, 997-1003 (1992); Needham M, Egerton M, Millest A, Evans S, Popplewell M, Cerillo G, McPheat J, Monk A, Jack A, Johnstone D and Hollis M. Protein Expr Purif 6,124-131 (1995).

However, the tissue-specificity of LCRs, although useful in some circumstances, is also a major limitation for many applications, for instance where no LCR is known for the tissue In which expression is required, or where expression in many, or all, tissues is required.

Our co-pending patent applications PCT/GB99/02357 (WO 00/053S3), US 09/358082, GB 0022995.5 and US 60/252,048 incorporated by reference herein, describe elements that are responsible, in their natural chromosomal context, for establishing an open chromatin structure across a locus that consists exclusively of ubiquitously expressed, housekeeping genes. These elements are not derived from an LCR and comprise extended methylation-free CpG islands. We have used the term Ubiquitous Chromatin Opening Element (UCOE) to describe such elements.

In mammalian DNA, the dinucleotide CpG is recognised by a DNA methyltransferase enzyme that methylates cytosine to 5-methylcytosine. However, 5-methylcytosine is unstable and is converted to thymine. As a result, CpG dinucleotides occur far less frequently than one would expect by chance. Some sections of genomic DNA nevertheless do have a frequency of CpG that is closer to that expected, and these sequences are known as "CpG islands". As used herein a "CpG island" is defined as a sequence of DNA, of at least 200bp, that has a GC content of at least 60% and an observed / expected CpG content ratio of at least 0.6 (i.e. a CpG dinucleotide content of at least 60% of that which would be expected by chance) (Gardiner-Green M and Frommer M. J Mol Biol 196, 261-282 (1987); Rice P, Longden I and Bleasby A Trends Genet 16, 276-277 (2000).

Methylation-free CpG islands are well-known in the art (Bird et al (1985) Cell 40: 91-99, Tazi and Bird (1990) Cell 60: 909-920) and may be defined as CpG islands where a substantial proportion of the cytosine residues are not methylated and which usually extend over the 5' ends of two closely spaced (0.1-3 kb) divergently transcribed genes. These regions of DNA are reported to remain hypomethylated in all tissues throughout development (Wise and Pravtcheva (1999) Genomics 60: 258-271). They are often associated with the 5' ends of ubiquitously expressed genes, as well as an estimated 40% of genes showing a tissue-restricted expression profile (Antequera, F. & Bird, A. Proc. Natl. Acad. Sci. USA 90, 1195-11999 (1993); Cross, S.H. & Bird, A.P. Curr. Opin, Genet. Dev. 5, 309-314 (1995) and are known to be localised regions of active chromatin (Tazi, J. & Bird, A. Cell 60, 909-920 (1990).

An 'extended' methylation-free CpG island is a methylation-free CpG island that extends across a region encompassing more than one transcriptional start site and/or extends for more than 300bp and preferably more than 500bp. The borders of the extended methylation-free CpG island are functionally defined through the use of PCR over the region in combination with restriction endonuclease enzymes whose ability to digest (cut) DNA at their recognition sequence is sensitive to the methylation status of any CpG residues that are present. One such enzyme is *Hpa*II, which recognises and digests at the site CCGG, which is commonly found within CpG islands, but only if the central CG residues are not methylated. Therefore, PCR conducted with Hpall-digested DNA and over a region harbouring *Hpa*II sites, does not give an amplification product due to *Hpa*II digestion if the DNA is *unmethylated*. The PCR will *only* give an amplified product if the DNA is *methylated*. Therefore, beyond the methylation-free region Hpall will not digest the DNA a PCR amplified product will be observed thereby defining the boundaries of the "extended methylation-free CpG island".

We have demonstrated (WO 00/05393) that regions spanning methylation-free CpG islands encompassing dual, divergently transcribed promoters from the human TATA binding protein (TBP)/proteosome component-B1 (*PSMBI*) and heterogeneous nuclear ribonucleoprotein A2/B1 (hnRNPA2)/heterochromatin protein 1Hsγ (*HP1*^{*Hs*γ}) gene loci give reproducible, physiological levels of gene expression and that they are able to prevent a variegated expression pattern and silencing that normally occurs with transgene integration within centromeric heterochromatin.

As used herein, the term "reproducible expression" means that the polynucleotide of the invention will direct expression of the expressible gene at substantially the same level of expression irrespective of its chromatin environment and preferably irrespective of the cell type or tissue type in which the polynucleotide of the invention may be. Those of skill In the art will recognize that substantially the same level of expression of the operably-linked expressible gene is achieved, irrespective of the chromatin environment of the claimed polynucleotide, and preferably irrespective of the cell type, assuming that the cell is capable of active gene expression.

We have shown (WO 00/05393) that methylation-free CpG islands associated with actively transcribing promoters possess the ability to remodel chromatin and are thus thought to be a prime determinant in establishing and maintaining an open domain at housekeeping gene loci.

UCOEs confer an increased proportion of productive gene delivery events with improvements in the level and stability of transgene expression. This has important research and biotechnological applications including the generation of transgenic animals and recombinant protein products in cultured cells. We have shown (WO 00/05393) beneficial effects of UCOEs on expression of the CMV-EGFP reporter construct and with the secreted, pharmaceutically valuable protein erythropoietin. The properties of UCOEs also suggest utility in gene therapy, the effectiveness of which is often limited by a low frequency of productive gene delivery events and an inadequate level and duration of expression (Verma, I.M. & Somia, N. Nature 389: 239-242 (1997).

Given these significant implications and wide ranging applications, there is a ' desire to further optimise transgene expression levels. There is a need to further increase the levels of expression obtainable by the use of a UCOE alone, particularly in the fields of *in vivo* gene therapy and for *in vitro* production of recombinant proteins.

The expression of a nucleic acid operably linked to a 5' UCOE may surprisingly be further increased by the presence of a selectable element 3' to the expressed nucleic acid, so that the expressible nucleic acid sequence is flanked by a 5' UCOE and a 3' selectable marker.

A selectable element that performs more than one function in a vector, such as providing a selectable marker as well as increasing expression of an operably linked gene, allows construction of more compact and efficient expression vectors.

Mei, Kothary and Wall (Mei, Q, Kothary R and Wall L. Exp Cell Research 260, 304-312 (2000) disclose constructs comprising an expressible gene (β-globin) operably linked to an LCR and a *pgk* / puromycin resistance element. However, this work teaches that it is the combination of an expressible gene, and LCR and a *tk* / neomycin resistance element that is important in imposing position effects on gene expression, with the *pgk* / puromycin resistance element being used as a negative control. This paper teaches away from any beneficial effect being gained from the use of a *pgk* /puromycin resistance element. The paper does not disclose constructs comprising an extended unmethylated CpG island (or UCOE), an expressible gene and a *pgk* / puromycin resistance element, since the constructs comprise LCRs. Similarly, the paper does not disclose an expressible gene operably linked to a promoter with which it is not naturally linked, also operably linked to a *pgk* / puromycin resistance element, since in each case the β-globin gene is expressed under control of its endogenous promoter.

Artelt et al compare the influence of neomycin and puromycin resistance genes on cis-linked genes in eukaryotic expression vectors (Artelt P, Grannemann R, Stocking C, Friel J, Bartsch J and Hauser H Gene 99, 249-254 (1991). They conclude that neomycin resistance genes may have a silencing effect on linked genes, but that "the gene conferring resistance to puromycin from *Streptomyces alboniger* does not influence adjacent promoters". Accordingly, there is nothing in this paper that discloses or suggests the importance of the position or spacing use of resistance genes as disclosed in the present application.

Our co-pending patent applications PCT/GB99/02357 (WO 00/05393), US 09/358082, GB 0022995.5 and US 60/252,048 disclose polynucleotides and vectors comprising extended, methylation-free CpG islands operably linked to expressible nucleic acids with antibiotic resistance genes. However, in the examples disclosed, the antibiotic gene is not adjacent and 3' to the expressible nucleic acid. The surprising contribution of such an adjacent selectable marker is likewise not disclosed or implied.

### STATEMENTS OF THE INVENTION

The present invention discloses that the influence of extended, unmethylated CpG islands (UCOEs) to upregulate expression of operably linked nucleic acid sequences may be further increased by the presence of a selectable element providing that said selectable marker is situated 3' of the expressible nucleic acid sequence and adjacent to it.

The terms 5' and 3' are herein used with respect to the sense strand of the expressible nucleic acid sequence. Hence the 5' end of said sequence corresponds to the start of transcription, which proceeds in a 3' direction.

As used herein, the term "operably linked" refers to a relationship of operability between elements in the polynucleotides of the invention. "Operably linked" is a term, well known to those of skill in the art, that describes a functional relationship between cis-acting DNA sequences. The exact structural relationship may or may not be relevant and differs for different types of elements. For a promoter, it implies an essentially adjacent (usually within less than 100bp) position 5' to the open reading frame that it drives. In the case of extended methylation-free CpG islands, it appears that a regional effect on chromatin structure is responsible for increasing the level and consistency of gene expression. By way of example, the element comprising an extended methylation-free CpG-island is positioned immediately 5' of the expressible gene. However, "operably-linked" embraces the possibility of being positioned elsewhere, as long as a clear functional effect can be demonstrated.

In particular, the flanking of an expressible gene with a UCOE at the 5' end and a selectable element at the other results in an increase in expression of approximately two-fold. In some cases the increase is greater than five-fold over that obtained with a single UCOE alone.

According to the present invention, there is provided an isolated polynucleotide that enables increased levels of expression of an operably linked gene to be obtained as compared to those obtainable using an operably-linked UCOE or extended methylation-free CpG island alone.

The isolated polynucleotide comprises: an extended methylation-free CpG island, an expressible nucleic acid terminated by a polyadenylation signal and a selectable marker operably linked to a promoter, wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 2000bp of the proximal end of the selectable marker.

As used herein, "proximal end" means the end of the selectable marker gene (including its promoter) that is closest to the 3' end of the expressible nucleic acid, as marked by its polyadenylation signal. It is envisaged that the selectable marker might be in either orientation, so that the proximal end relative to the expressible nucleic acid might be at either the 5' promoter end of the selectable marker or the 3', termination of transcription end, taking 5' and 3' as being according to the sense strand of the selectable marker.

Preferably, the transcriptional start of the selectable marker is within 1500bp of the 3' end of the expressible nucleic acid sequence, as marked by its polyadenylation signal of the latter. More preferably, it is within 1000bp. Most preferably it is within 500bp.

In one aspect of the invention, the selectable element is an antibiotic resistance gene. Preferably it is an antibiotic resistance gene obtained from a *Streptomyces* species. More preferably, said antibiotic resistance gene is operably linked to a promoter of the phosphoglycerate kinase (*pgk*) gene. Most preferably, it is the promoter of the murine *pgk* gene (Adra, CN, Boer PH and McBurney, MW. Gene 60, 65-74 (1987). Alternatively, it may be another mammalian *pgk* promoter.

In a preferred embodiment, the antibiotic resistance gene is the puromycin resistance gene from a *Streptomyces* species. Most preferably, it is the puromycin N-acetyl transferase gene from *Streptomyces alboniger* (Vara JA, Portela A, Ortin J, Jimenez A. Nucleic Acids Res 14, 4617-4624 (1986)

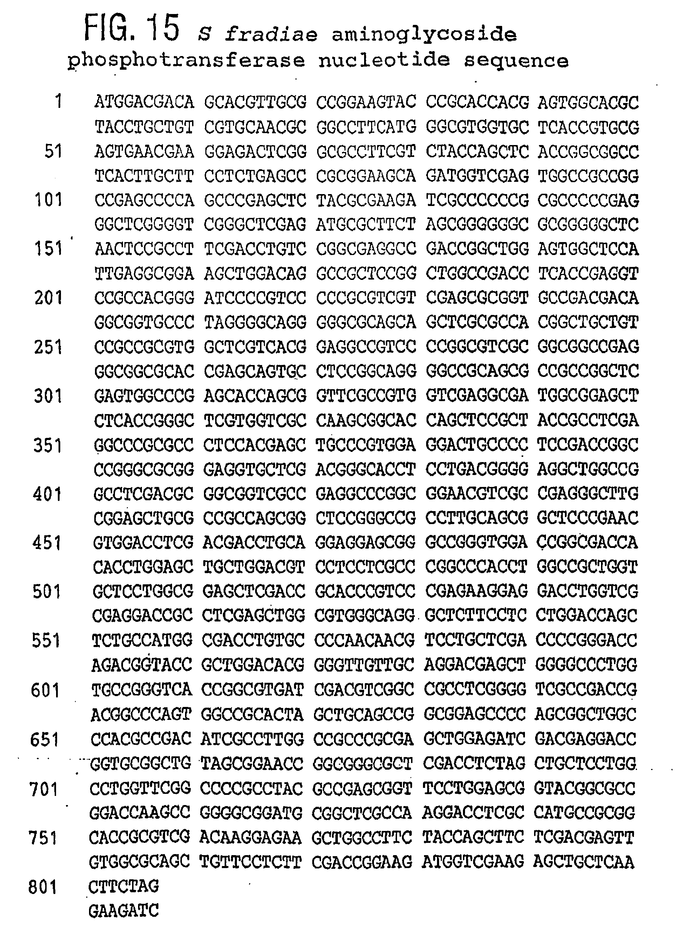

Alternatively, the antibiotic resistance gene is a modified form of the puromycin N-acetyl transferase gene from *Streptomyces alboniger*. Preferably this gene has been modified by manipulation of its codon usage, in a manner commonly done to adapt bacterial genes for expression in mammalian host cells. Such codon modification leaves the encoded amino acid sequence unchanged, with the result that the expressed enzyme is unchanged from the wild-type puromycin N-acetyl transferase. Most preferably, the modified gene has the sequence shown in Figure 15.

Alternatively, the antibiotic resistance gene is a neomycin resistance gene derived from a *Streptomyces* species. Preferably it is the aminoglycoside phosphotransferase gene from *Streptomyces fradiae* (Thompson CJ and Gray GS. Proc Natl Acad Sci USA 80, 5190-5194 (1983).

In an alternative embodiment, the antibiotic resistance gene is a hygromycin resistance gene. Preferably, it is the hygromycin phosphotransferase gene from *Streptomyces hygroscopicus.*

In a further alternative embodiment, the antibiotic resistance gene is a bleomycin resistance gene. Preferably, it is the bleomycin binding protein from *Streptomyces verticillus.* Alternatively, it is the bleomycin N-acetyltransferase from *Streptomyces verticillus.*

In another embodiment, the antibiotic resistance gene is a blasticidin resistance gene. Preferably, it is the blasticidin S-acetyltransferase gene from *Streptomyces verticillum.*

In another aspect of the invention, the antibiotic resistance gene is not obtained from a *Streptomyces* species. In one preferred embodiment it is the hygromycin resistance gene encoding aminocyclitol phosphotransferase from *Escherichia coli*.

In another preferred embodiment, it is the neomycin phosphotransferase gene from transposon Tn5, originally derived from *Klebsiella pneumonia*.

In an alternative aspect of the invention, the selectable marker is not an antibiotic resistance gene. Alternative selection mechanisms involve using genes encoding thymidylate synthase, thymidine kinase or dihydrofolate reductase. Such selection mechanisms are well-known to those of appropriate skill in the art. In a medium lacking methionine, a gene encoding glutamine synthetase may be used as a means of selection either in cells lacking an endogenous glutamine synthetase, or where use of an inhibitor, such as methionine sulphoxamine, has rendered it inactive (Kaufman RJ. Selection and coamplification of heterologous genes in mammalian cells. Methods Enzymol 185, 537-566 (1990).

In a further aspect, a screenable marker can be used. For instance, a gene encoding a fluorescent protein, such as the *Aequoria victoria* green fluorescent protein (GFP), or enhanced variants of it (EGFP), may be used as a selectable marker. Transfectants containing a polynucleotide according to the current invention, wherein the selectable marker encodes GFP, may be sorted by brightness of fluorescence on a FACS, by a process well-known in the art. Using the polynucleotide of the invention, and comparing with expressible constructs with the selectable marker situated either 5' to the UCOE, or 3' but remotely from the transgene (expressible nucleic acid), higher levels of expression of the transgene will be found for comparable levels of brightness. Selection of the brightest cells will, therefore, allow selection of cells with the highest level of transgene expression.

In one aspect of the invention, the extended methylation-free CpG island comprises a 16kb DNA fragment spanning the human hnRNP A2 gene with 5kb 5' and 1.5kb 3' flanking sequence. Preferably, the extended methylation-free CpG island comprises an 8kb DNA fragment spanning the human hnRNP A2 gene (WO 00/05393).

Alternatively, the extended methylation-free CpG island of the disposed polynucleotide is an 'artificial UCOE' as disclosed in our co-pending applications GB 0022995.5 and US 60/252,048, comprising the human β-actin CpG island/ promoter region or a fragment thereof. Preferably this fragment is within the size range of 100bp to 3.0 kb and spans the human β-actin CpG island/ promoter region or a fragment thereof. Preferably the artificial UCOE also comprises the human PDCD2 CpG island/ promoter region or a fragment thereof. More preferably the human PDCD2 CpG island/ promoter region comprises a fragment within the size range of 100bp to 3.0 kb. Further preferably, the extended methylation-free CpG island comprises a DNA fragment within the size range of 100bp to 3.0 kb spanning the human β-actin CpG island/ promoter region and a DNA fragment within the size range of 100bp to 3.0 kb spanning the human PDCD2 CpG island/promoter region.

Most preferably the claimed polynucleotide of this embodiment of the invention comprises an artificial UCOE comprising a 2.0 kb DNA fragment spanning the human β-actin CpG island/ promoter region and a 1.8 kb DNA fragment spanning the human PDCD2 CpG island/promoter region.

Also provided is a vector comprising the polynucleotide of any one of the previous embodiments. This vector may alternatively be either an episomal or an integrating vector. Depending on the intended use, episomal vectors may be desirable since they are self-replicating and so persist without the need for integration. Episomal vectors of this type are described in WO98/07876. Also preferred are non-replicating, non-integrating vectors.

Also provided is a vector so constructed as to deliver, when linearised and integrated into a chromosome, a polynucleotide comprising an extended methylation-free CpG island, an expressible nucleic acid terminated by a polyadenylation signal and a selectable marker operably linked to a promoter, wherein both the CpG island and the selectable marker are operably-linked to the expressible nucleic acid, and the components are positioned in the order: extended methylation-free CpG island, expressible nucleic acid, selectable marker, In the 5' to 3' orientation with respect to the sense strand of the expressible nucleic acid, and the polyadenylation signal at the 3' end of the expressible nucleic acid is within 2000bp of the proximal end of the selectable marker.

Preferably the vector is a plasmid. Alternatively, the vector may be a virus, such as an adenovirus, adeno-associated virus, a herpesvirus, vaccinia virus, lentivirus or other retrovirus.

Preferably said vector is an expression vector adapted for eukaryotic gene expression. Typically said adaptation includes, by example and not by way of limitation, the provision of transcription control sequences (promoter sequences) which mediate cell/tissue specific expression. Promoter and enhancer are terms well-known in the art and include the following features which are provided by example only, and not by way of limitation. Promoters are 5', cis-acting regulatory sequences directly linked to the initiation of transcription. Promoter elements include so-called TATA box and RNA polymerase initiation selection (RIS)-sequences that function to select a site of transcription initiation. These sequences also bind polypeptides that function, *inter alia*, to facilitate transcription initiation selection by RNA polymerase.

Enhancer elements are *cis* acting nucleic acid sequences often found 5' to the transcription initiation site of a gene (enhancers can also be found 3' to a gene sequence or even located in intronic sequences and are therefore position independent). Enhancers function to increase the rate of transcription of the gene to which the enhancer is linked. Enhancer activity is responsive to *trans* acting transcription factors (polypeptides) which have been shown to bind specifically to enhancer elements. The binding/activity of transcription factors is responsive to a number of environmental cues which Include, by way of example and not by way of limitation, intermediary metabolites (e.g. glucose), environmental effectors (e.g. heat,). (See Eukaryotic Transcription Factors, by David S Latchman, Academic Press Ltd, San Diego)

Adaptations also include the provision of selectable markers and autonomous replication sequences which both facilitate the maintenance of said vector in either the eukaryotic cell or prokaryotic host. Vectors that are maintained autonomously in eukaryotic cells are referred to as episomal vectors. Other adaptations which facilitate the expression of vector encoded genes include the provision of transcription termination/polyadenylation sequences. This also includes the provision of internal ribosome entry sites (IRES) which function to maximise expression of vector encoded genes arranged in bicistronic or multi-cistronic expression cassettes. These adaptations are well-known in the art. There is a significant amount of published literature with respect to expression vector construction and recombinant DNA techniques In general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol. III IRL Press, Oxford UK; DNA Cloning: F M Ausubel et al, Current Protocols in Molecular Biology, John Wiley & Sons, Inc.(1994).

In a preferred method of the invention said vector encodes, and thus said polypeptide is provided with, a secretion signal to facilitate purification of said polypeptide.

Alternatively, other preferred embodiments may include further refinements to facilitate purification of expressed recombinant protein, such as affinity tags or epitopes, or enzymatic cleavage sites.

Preferably the expressible nucleic acid is a therapeutic nucleic acid.

Alternatively, the expressible nucleic acid encodes a recombinant protein for expression in an *in vitro* cell culture system.

Alternatively, the expressible gene encodes a non-polypeptide product, such as RNA. Such RNA may be an antisense RNA capable of inhibiting expression of a particular gene at a post-transcriptional level, or may have an enzymatic (ribozyme) or other function, such as a ribosomal RNA.

One preferred embodiment is a vector comprising: an extended methylation-free CpG island, an expressible nucleic acid terminated by a polyadenylation signal and a selectable marker operably linked to a promoter, wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 2000bp of the proximal end of the selectable marker. Preferably, the the polyadenylation signal at the 3' end of the expressible nucleic acid is within 1500bp of the proximal end of the selectable marker. More preferably it is within 1000bp, most preferably, 500bp.

A preferred embodiment is a vector comprising: an extended methylation-free CpG island, a multiple cloning site, an antibiotic resistance gene obtained from a *Streptomyces* species, wherein both the CpG island and the selectable marker are operably-linked to the multiple cloning site, and the components are positioned in the order: extended methylation-free CpG island, multiple cloning site, selectable marker, in the 5' to 3' orientation with respect to the sense strand of the expressible nucleic acid, and the multiple cloning site is within 2000bp of the proximal end of the selectable marker.

More preferably, the multiple cloning site is further operably linked to a promoter. Further preferably the promoter is selected from CMV, EF-1α, RSV LTR or HIV2 LTR or combinations of sequences derived therefrom. More preferably the promoter is a CMV immediate/early promoter. Most preferably it is the mouse CMV immediate/early promoter. In a preferred embodiment, the vector comprises a CMV promoter, a multiple cloning site, a polyadenylation sequence and genes encoding selectable markers under suitable control elements.

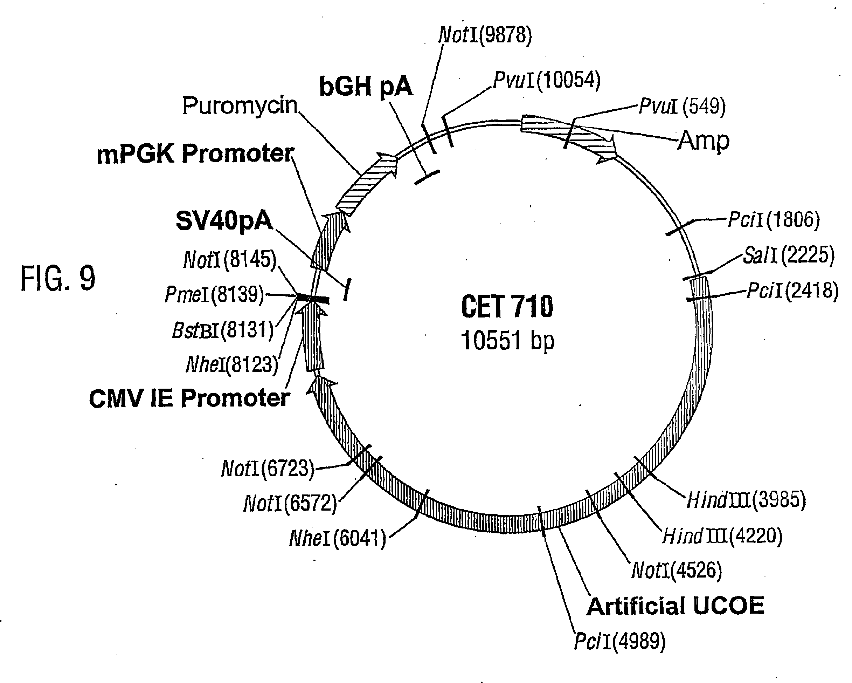

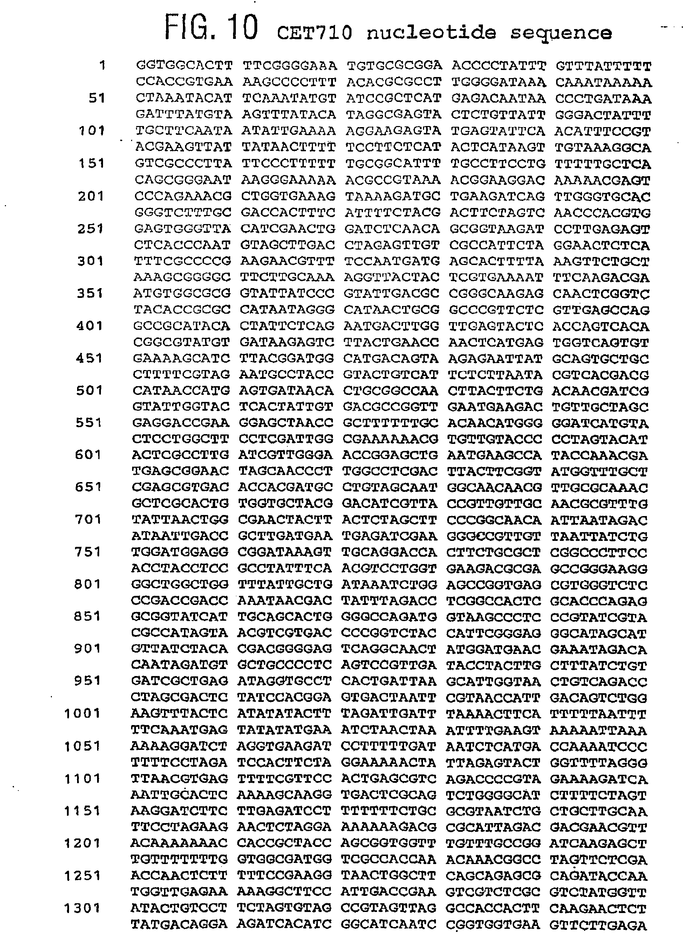

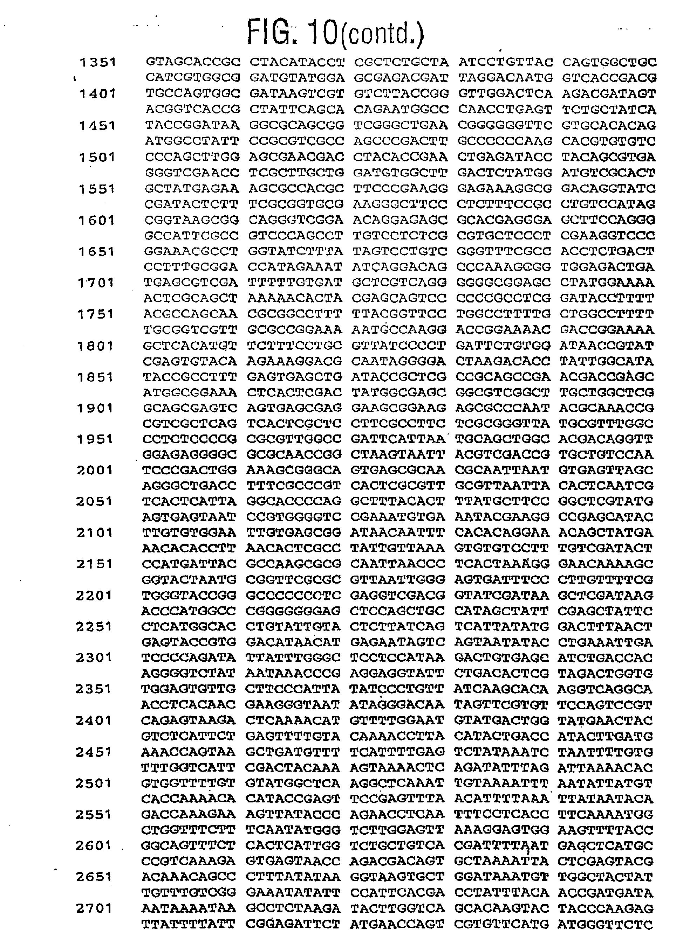

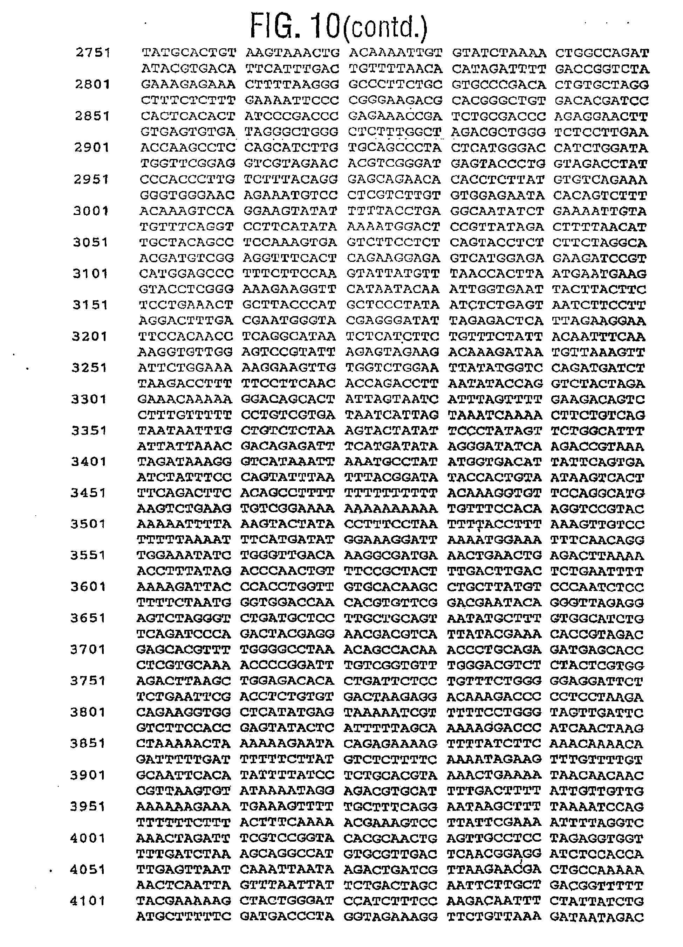

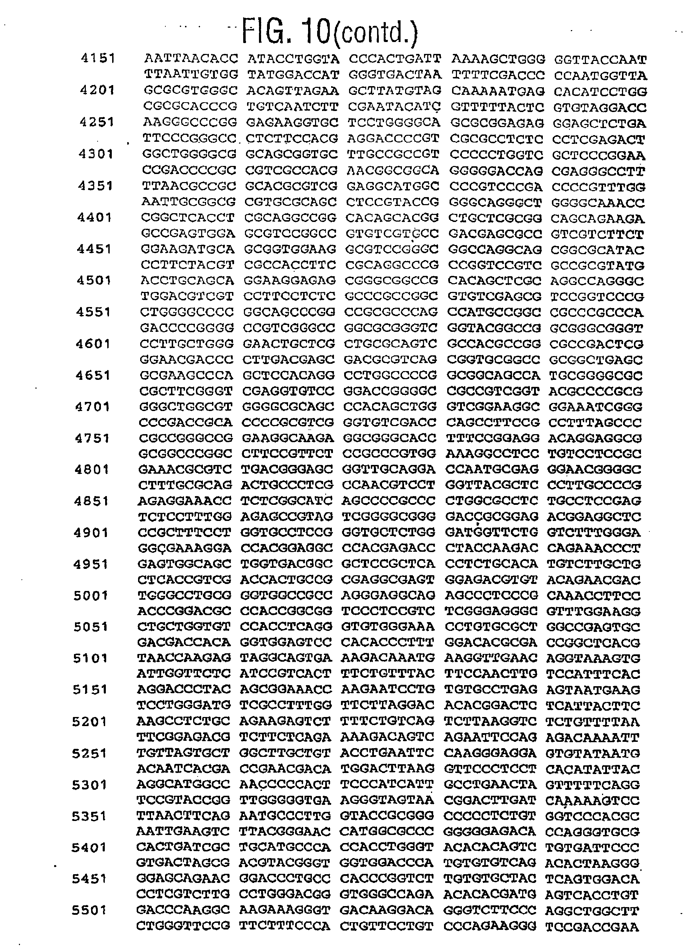

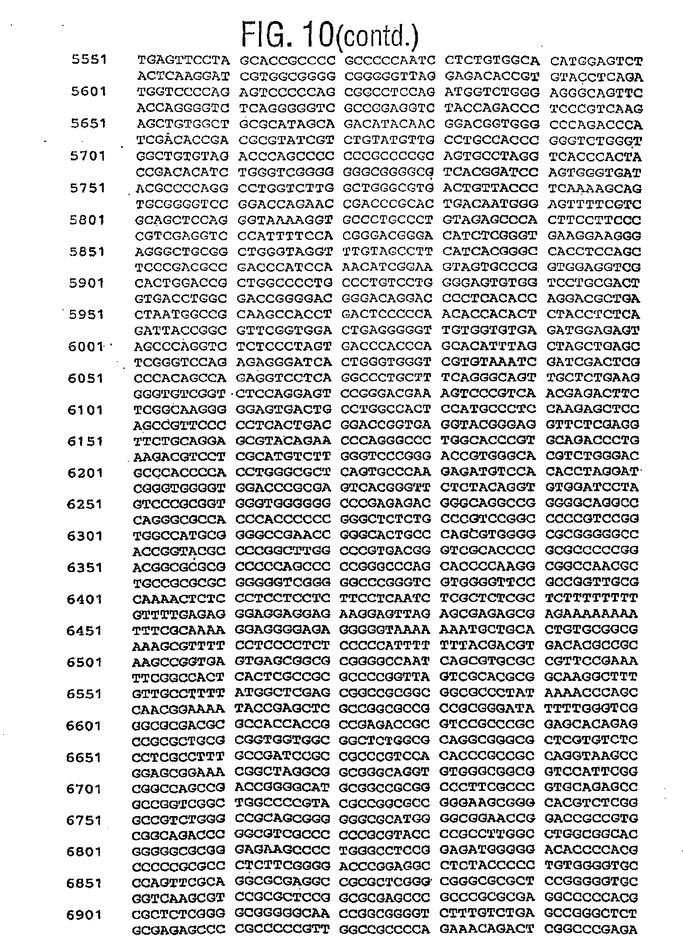

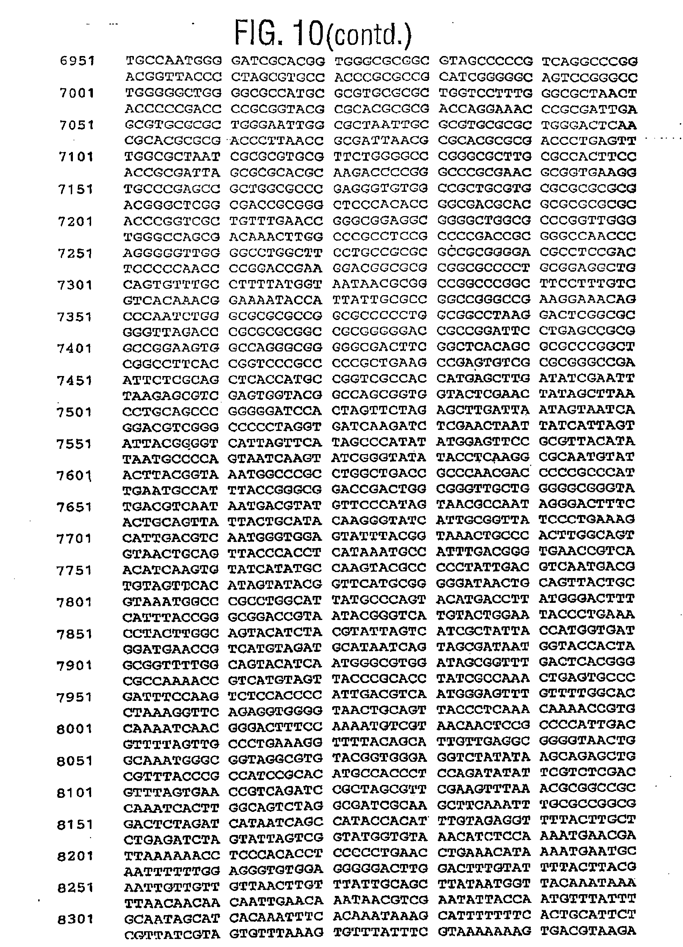

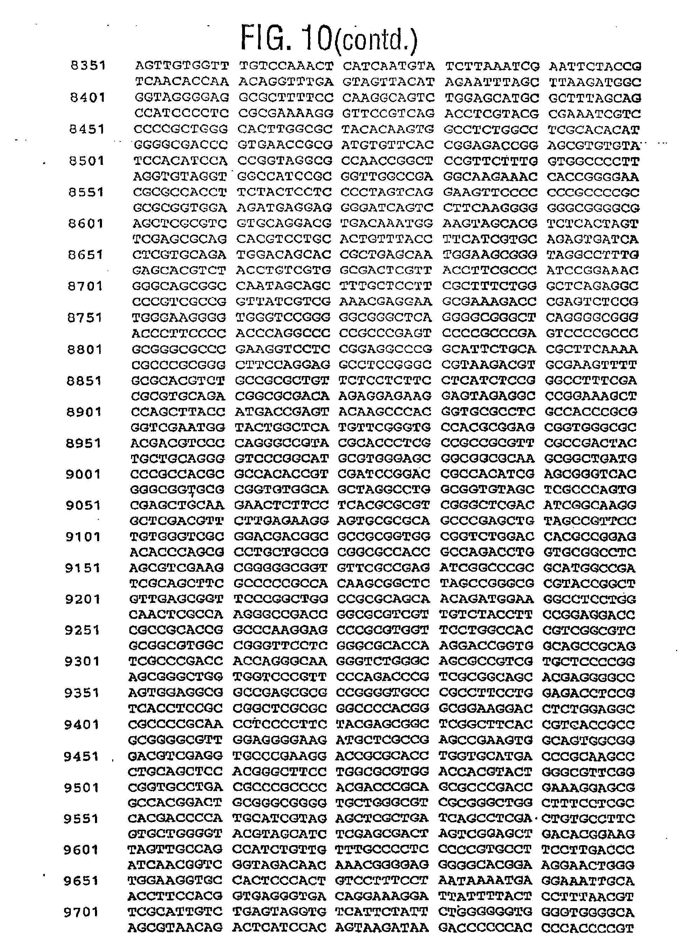

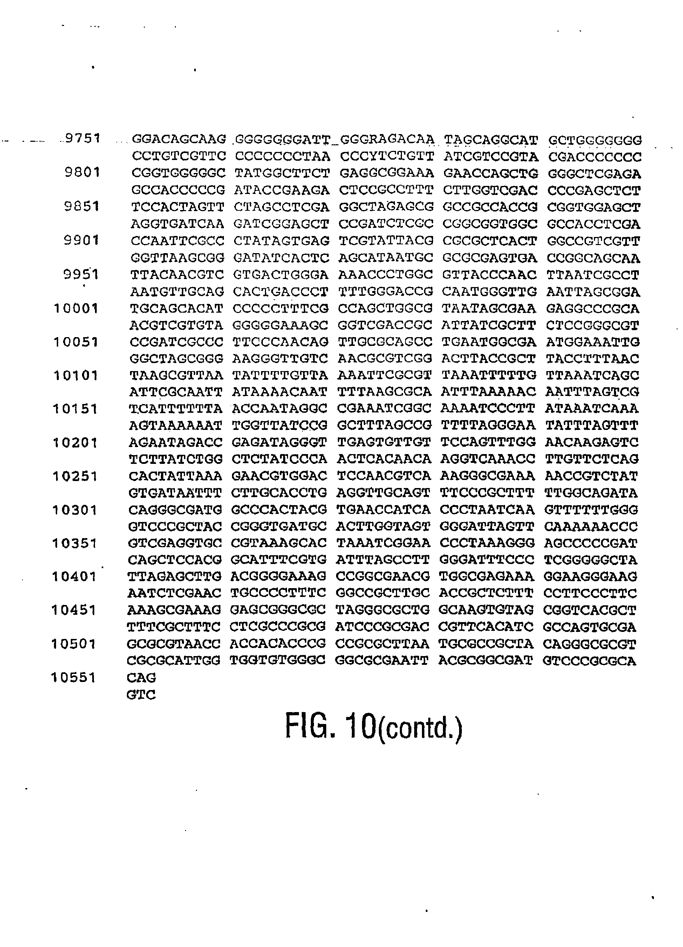

A preferred embodiment of the vector comprises nucleotides 1-10551 of the sequence of Figure 9. A most preferred embodiment is vector CET 710. Alternatively, the vector comprises nucleotides 1-13545 of the sequence of Figure 10, and is preferably vector CET 720.

Further preferred embodiment of vectors are:

CET 740 in which the puromycin resistance gene of CET 720 is replaced with the aminoglycoside phosphotransferase gene from *Streptomyces fradiae* (as listed In Figure 15) Also preferred are vectors having expressible nucleic acid sequences inserted into the multiple cloning site of CET 740, such as CET 741.

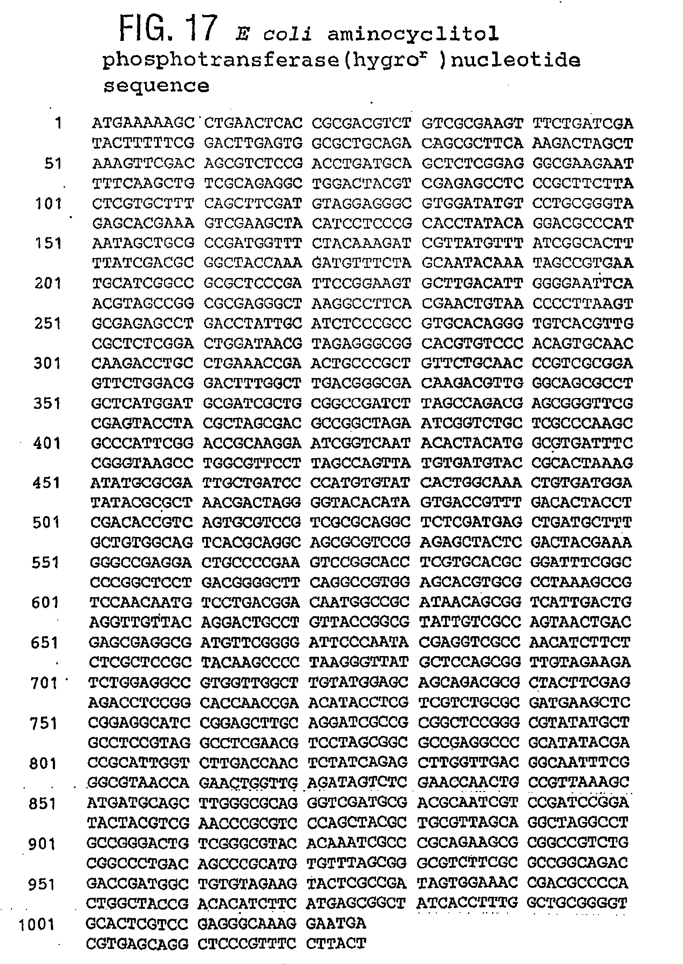

CET 760 in which the puromycin resistance gene of CET 720 is replaced with the aminocyclitol phosphotransferase from *Escherichia coli* (as listed in Figure 17). Also preferred are vectors having expressible nucleic acid sequences inserted into the multiple cloning site of CET 760, such as CET 761.

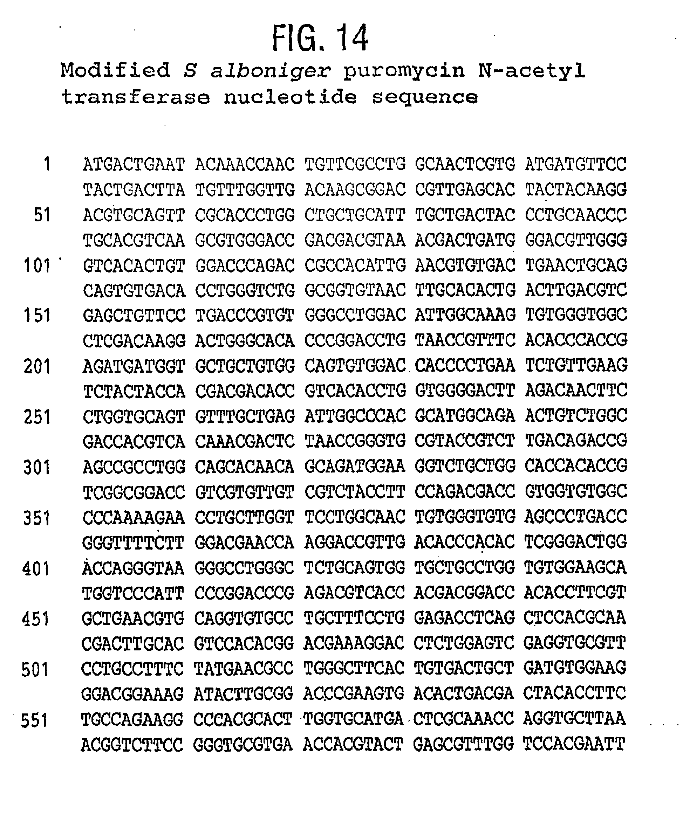

CET 780 in which the puromycin resistance gene of CET 720 is replaced with the modified form of the puromycin N-acetyl transferase gene from *Streptomyces alboniger* (as listed In Figure 14). Also preferred are vectors having expressible nucleic acid sequences Inserted into the multiple cloning site of CET 780, such as CET 781.

CET 820 in which the human IE CMV promoter, operably linked to the multicloning site In order to drive expression of expressible nucleic acid sequences inserted there, has been replaced with the murine IE CMV promoter. Also preferred are vectors having expressible nucleic acid sequences inserted into the multiple cloning site of CET 820, such as CET 821.

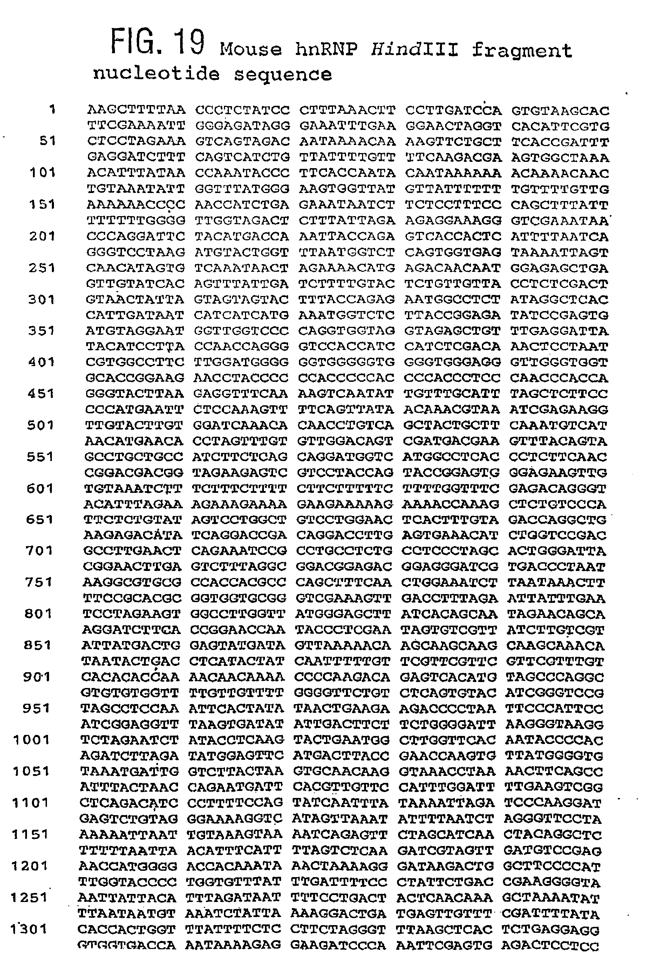

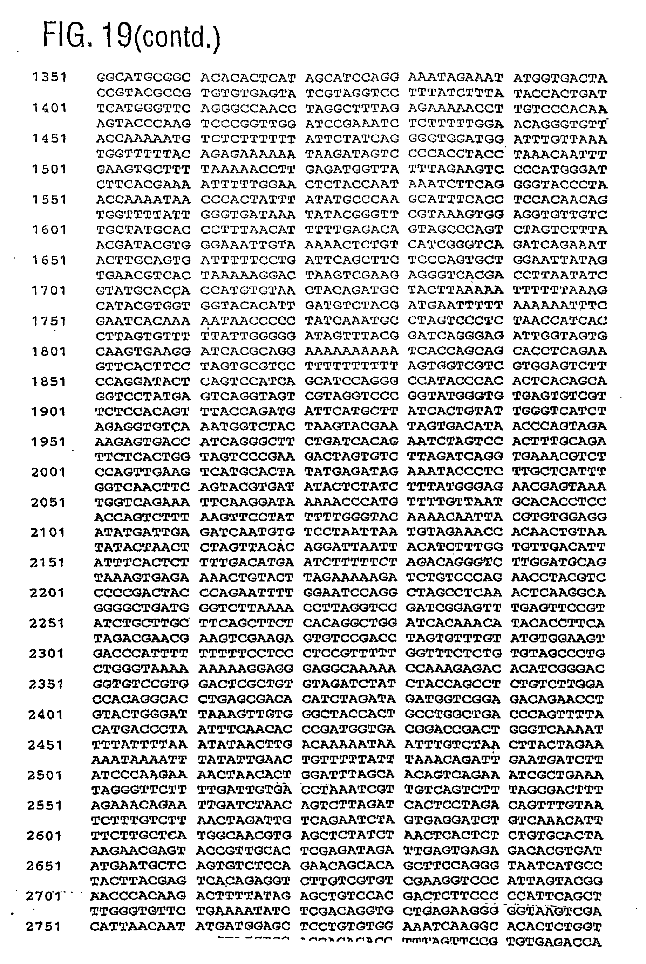

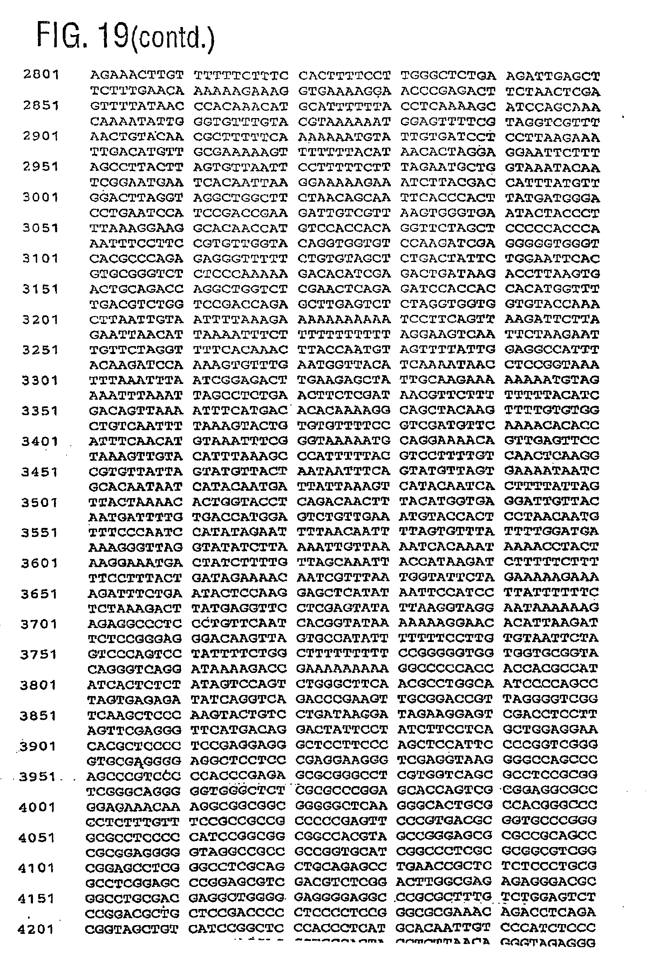

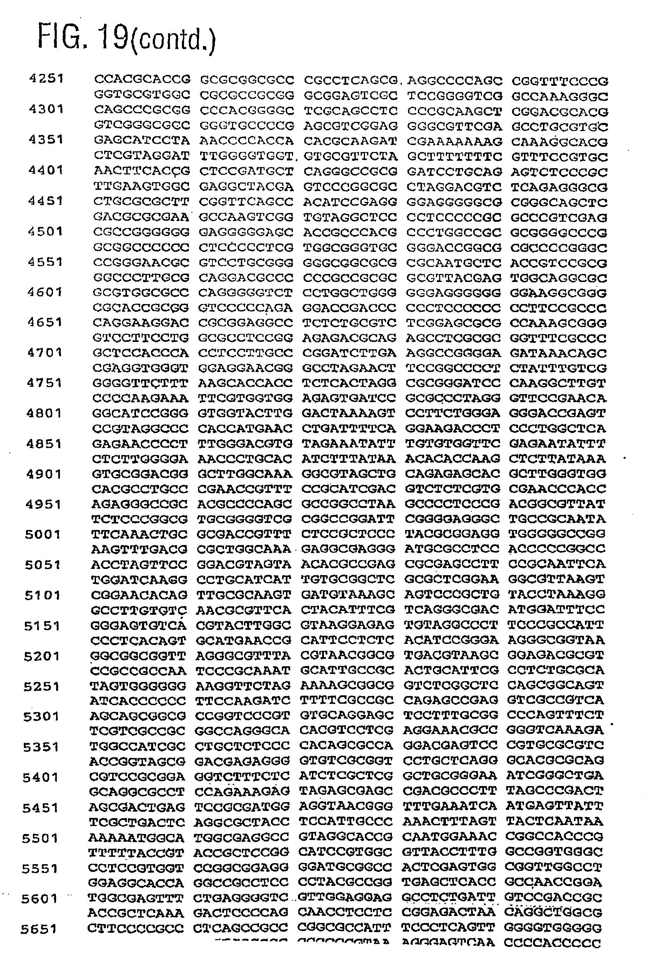

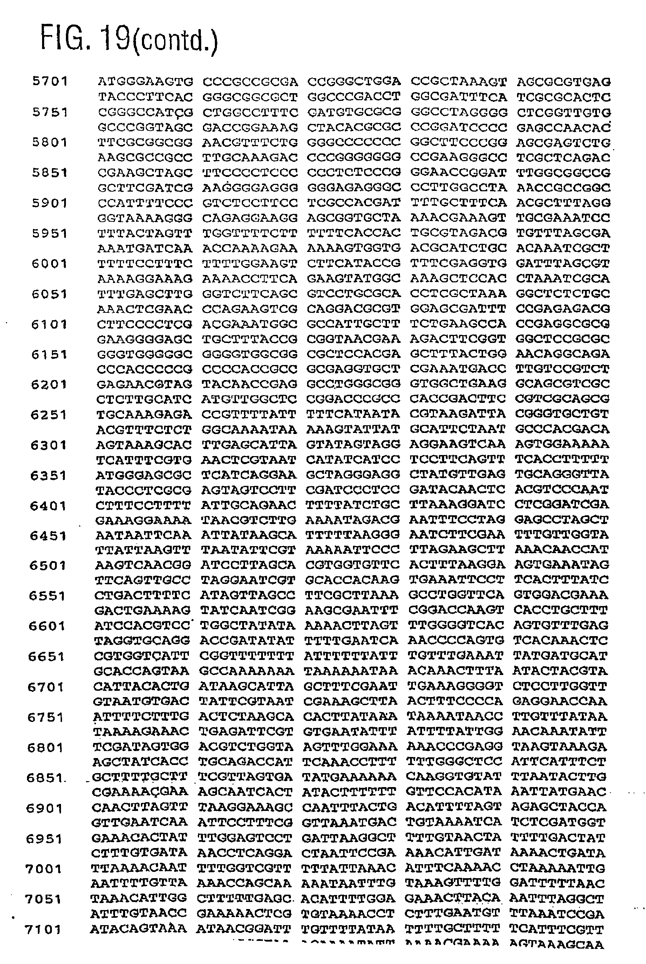

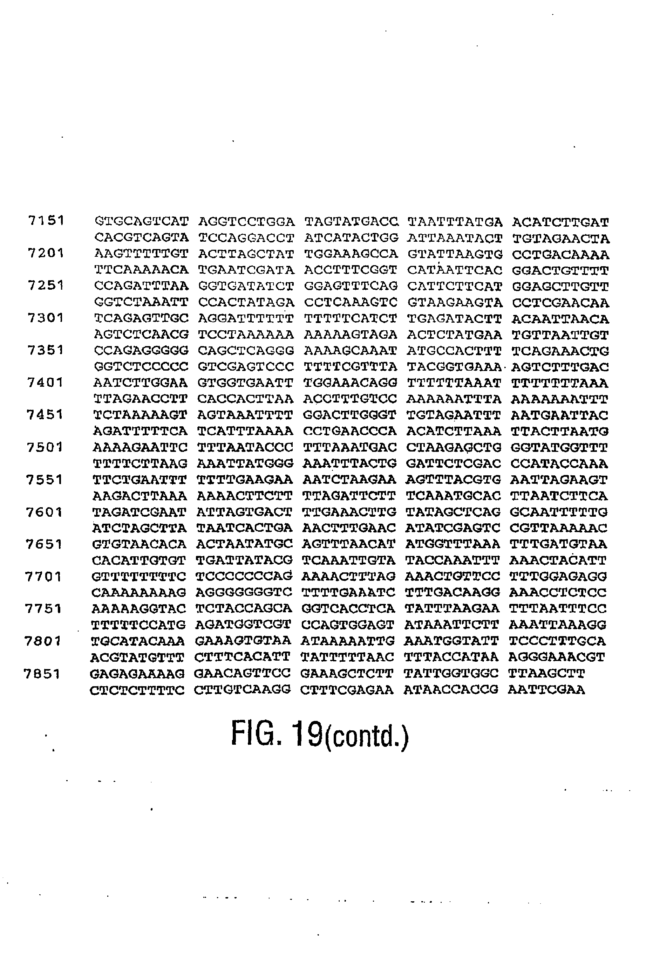

CET 823 in which the extended methylatlon-free CpG island comprising an 8kb DNA fragment spanning the human hnRNP A2 gene is replaced with the extended methylation-free CpG island comprising an 8kb fragment spanning the murine hnRNP A2 gene (as shown in the sequence of Figure 19). Also preferred are vectors having expressible nucleic acid sequences inserted into the multiple cloning site of CET 823, such as CET 824.

Also provided is host cell transfected with any of the embodiments of the disclosed vectors.

Alternatively said polynucleotide, vector or the host cell may be used in a cell culture system to obtain expression of a desired gene product. Suitable cell culture systems are well known in the art and are fully described In the body of literature known to those skilled in the art. There is provided a method for the production of a polypeptide-according to the Invention comprising:
i) providing a cell transformed/transfected with a nucleic acid molecule according to the invention;
ii) growing said cell in conditions conducive to the manufacture of said polypeptide; and
iii) purifying said polypeptide from said cell, or its growth environment.

In a preferred embodiment of the invention said nucleic acid molecule Is the vector according to the invention.

The present invention also provides the polynucleotide, vector or the host cell for use in therapy.

The present invention also provides use of the polynucleotide, vector or host cell in the manufacture of a composition for use.in gene therapy.

The present invention also provides a method of treatment, comprising administering to a patient in need of such treatment a pharmaceutically effective amount of the polynucleotide, vector or host cell of the present invention. Preferably the patient is suffering from a disease treatable by gene therapy.

The present invention also provides a pharmaceutical composition comprising the polynucleotide and/or the vector and/or host cell, optionally in admixture with a pharmaceutically acceptable carrier or diluent, for therapy to treat a disease or provide the cells of a particular tissue with an advantageous protein or function.

The polynucleotide, vector or host cell of the invention or the pharmaceutical composition may be administered via a route which includes systemic intramuscular, intravenous, aerosol, oral (solid or liquid form), topical, ocular, rectal, intraperitoneal and/or intrathecal and local direct injection.

The exact dosage regime will, of course, need to be determined by individual clinicians for individual patients and this, in turn, will be controlled by the exact nature of the protein expressed by the gene of interest and the type of tissue that is being targeted for treatment.

The dosage also will depend upon the disease Indication and the route of administration. The number of doses will depend upon the disease, and the efficacy data from clinical trials.

The amount of polynucleotide or vector DNA delivered for effective gene therapy according to the invention will preferably be in the range of between 50 ng -1000 µg of vector DNA/kg body weight; and more preferably in the range of between about 1-100 µg vector DNA/kg.

Although it is preferred according to the Invention to administer the polynucleotide, vector or host cell to a mammal for *in vivo* cell uptake, an ex *vivo* approach may be utilised whereby cells are removed from an animal, transduced with the polynucleotide or vector, and then re-implanted into the animal. The liver, for example, can be accessed by an *ex vivo* approach by removing hepatocytes from an animal, transducing the hepatocytes *in vitro* and re-implanting the transduced hepatocytes into the animal (e.g., as described for rabbits by Chowdhury et al., Science 254:1802-1805, 1991, or in humans by Wilson, Hum. Gene Ther. 3:179-222, 1992). Such methods also may be effective for delivery to various populations of cells in the circulatory or lymphatic systems, such as erythrocytes, T cells, B cells and haematopoietic stem cells.

Another aspect of the invention provides an isolated polynucleotide comprising a first promoter operably linked to an expressible gene to which it is not naturally operably linked and a selectable element, also operably linked and 3' to the expressible gene, comprising a *pgk* promoter and a puromycin resistance gene. The use of such a polynucleotide to obtain reproducible expression of said expressible gene in at least two tissue or cell types is also provided.

In another embodiment of the invention there is provided a non-human transgenic animal comprising an artificially introduced extended methylation-free CpG island element and an artificially introduced selectable marker element wherein both elements are operably-linked to an expressible gene situated between them and wherein reproducible expression of said expressible gene occurs in at least two tissue or cell types. Methods of making transgenic mice (Gordon et al., Proc. Natl. Acad. Scl. USA 77:7380 (1980); Harbers et al., Nature 293:540 (1981); Wagner et al., Proc. Natl. Acad. Sci. USA 78:5016 (1981); and Wagner et al., Proc. Natl. Acad. Sci. USA 78:6376 (1981), sheep pigs, chickens (see Hammer et al., Nature 316:680 (1985)), etc., are well-known in the art and are contemplated for use according to the invention.

Such transgenic animals containing the polynucleotide of the invention also may be used for long-term production of a protein of interest.

There is also provided a mammalian model for determining the efficacy of gene therapy using the polynucleotide, vector or host cell of the Invention. The mammalian model comprises a transgenic animal whose cells contain the vector of the present invention. Such animals permit testing prior to clinical trials in humans.

The present invention also provides the use of the polynucleotide of the present invention in producing transgenic plants.

The generation of transgenic plants that have increased yield, or increased resistance to disease, pests, drought or salt are well known to those skilled in the art. The present invention also provides for transgenic plant containing cells that contain the polynucleotide of the present invention. Some or all of the cells comprising the artificial UCOE may originate from plants.

The present invention also relates to the use of polynucleotide of the present invention in functional genomics applications. Functional genomics relates principally to the identification of genes specifically expressed in particular cell types or disease states and now provides thousands of novel gene sequences of potential Interest for drug discovery or gene therapy purposes. The major problem in using this information for the development of novel therapies lies in how to determine the functions of these genes. The polypeptides of the invention can be used in a number of functional genomic applications in order to determine the function of gene sequences. The functional genomic applications of the present invention include, but are not limited to:
(1) Using the polynucleotide of the present invention to achieve sustained expression of anti-sense versions of the gene sequences or ribozyme knockdown libraries, thereby determining the effects of inactivating the gene on cell phenotype.
(2) Using the polynucleotide of the present invention to prepare expression libraries for the gene sequences, such that delivery into cells will result in reliable, reproducible, sustained expression of the gene sequences. The resulting cells, expressing the gene sequences can be used in a variety of approaches to function determination and drug discovery. For example, raising neutralising antibodies to the gene product; rapid purification of the protein product of the gene itself for use in structural, functional or drug screening studies; or in cell-based drug screening.
(3) Using the polynucleotide of the present invention in approaches Involving mouse embryonic stem (ES) cells and transgenic mice. One of the most powerful functional genomics approaches involves random insertion into genes in mouse ES cells of constructs which only allow drug selection following insertion Into expressed genes, and which can readily be rescued for sequencing (G.Hicks et al., Nature Genetics, 16, 338-334). Transgenic mice with knockout mutations in genes with novel sequences can then readily be made to probe their function. At present this technology works well for the 10% of mouse genes which are well expressed in mouse ES cells. Incorporation of the polynucleotides of the present invention into the Integrating constructs will enable this technique to be extended to identify all genes expressed in mice.

### DETAILED DESCRIPTION OF THE INVENTION

The invention will now be described by way of example only and with reference to the accompanying figures wherein;

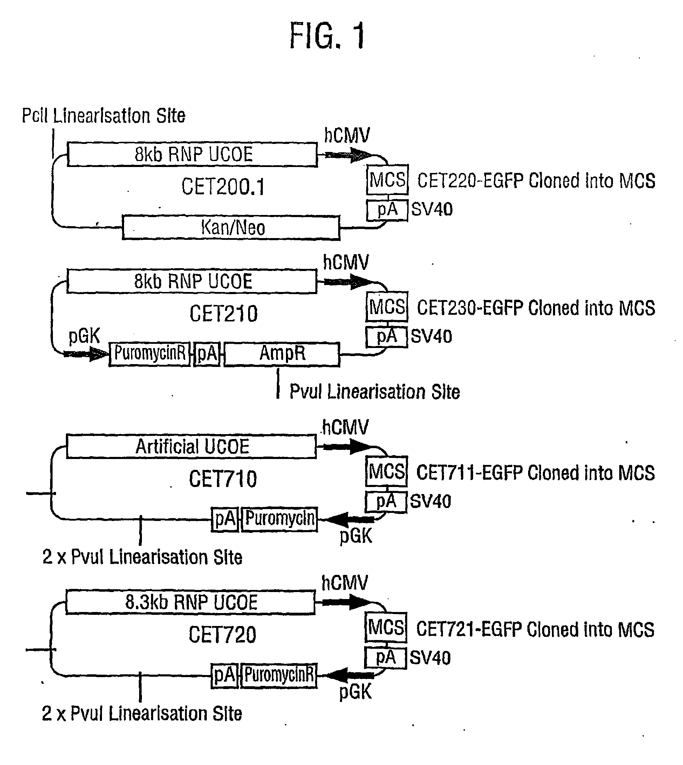

**Figure 1** shows maps of 'empty' vectors CET 200.1, 210, 710 and 720. Insertion of the Enhanced Green Fluorescent Protein (EGFP) gene into the multicloning site results in CET 230, 711 and 721, respectively. All vectors contain a CMV promoter from which inserted genes are expressed. However, in the case of CET 210 (and its EGFP-expressing derivative, CET230) although such an inserted gene would be flanked by a UCOE and a *pgk* / puromycin resistance element in the plasmid, the latter is not immediately adjacent. More importantly, it is separated by a *Pvu* I site used to linearise the plasmid before transfection. After integration into the host cell chromosome, this results in the gene no longer being flanked, since both the UCOE and the *pgk* / puromycin resistance element will integrate in the same side of the gene. In the case of CET 710 (and its EGFP-expressing derivative, CET 711) and CET 720 (and its EGFP-expressing derivative, CET 721), *Pvu* I linearisation results in the integration of the gene closely flanked by the UCOE on one side and the *pgk I* puromycin resistance element on the other. CET 210 (and CET 230) and CET 720 (and CET 721) carry hnRNP-derived UCOEs, while CET 710 (and CET 711) carry an 'artificial' β-actin /PDCD2 -derived UCOE.

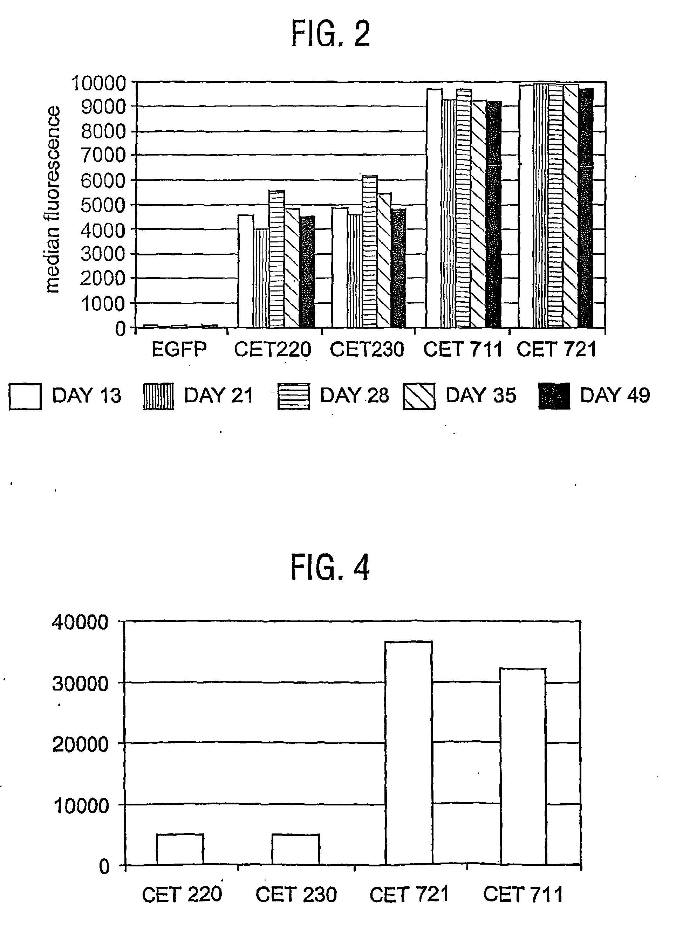

**Figure 2** shows expression of EGFP from various vectors transfected into CHO-K1 cells as measured by median fluorescence on FACS analysis measured on the indicated days post-transfection. 'EGFP' depicts cells transfected with a control (pEGFP) non-UCOE containing plasmid. CET220 shows cells transfected with a plasmid where the EGFP expression unit is operably linked to a hnRNP-derived UCOE but not to a *pgk* / puromycin resistance element. Instead a SV40 /neomycin resistance element is used.

The remaining cells are transfected with CET 230, 711 or 721, the structure of which are shown in Figure 1.
**Figure 3** shows the proportion of the populations of cells shown In **Figure 2** judged to be positive for expression on the indicated days post-transfection.
**Figure 4** shows the expression of EGFP in CHO-K1 cells transfected with vectors CET 220, 230, 721 and 711 as measured by median fluorescence corrected to allow comparison without exceeding the detection capacity of the FACScan. This clearly shows the comparative effect of placing the selectable marker (puro^{r}) either 5' (CET230) or 3' (CET721) to the expressible transgene (EGFP).
**Figure 5** shows the expression of EGFP in CHO-K1 cells transfected with vectors CET 701, 721, 704, 741, 705, 751, 706, 761, 708 and 781 as measured by median fluorescence corrected to allow comparison without exceeding the detection capacity of the FACScan.
**Figure 6** shows the expression levels of EGFP In CHO-K1 cells transfected with vectors comparing 5' human and murine hnRNP UCOEs with a 3' puromycin resistance gene.
**Figure 7** shows the effect of position of the Streptomyces neomycin resistance gene on EGFP expression. CET741 has the selectable marker 3' of the transgene, CET 745 has the marker 5' of the transgene and UCOE.

The UCOE is the human RNP UCOE in both cases.

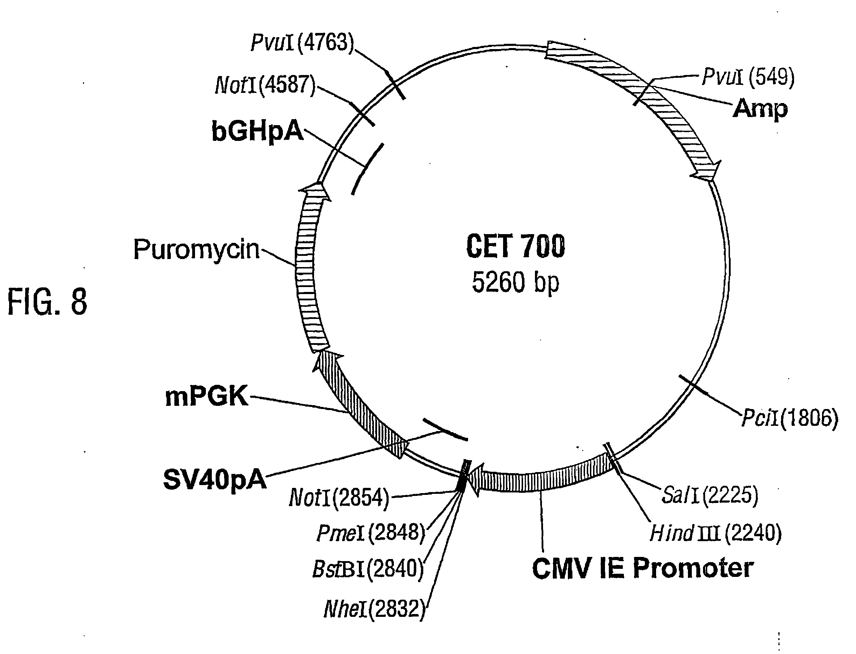

**Figure 8** shows a map of plasmid CET 700
**Figure 9** shows a map of plasmid CET 710
**Figure 10** shows the nucleotide sequence of CET710

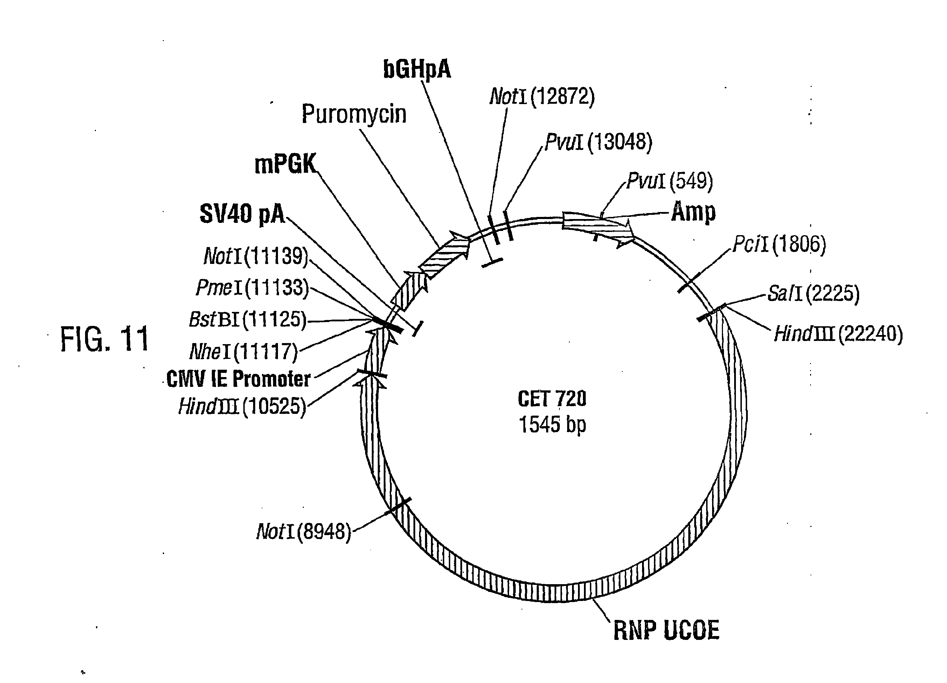

**Figure 11** shows a map of plasmid CET 720
**Figure 12** shows the nucleotide sequence of CET720

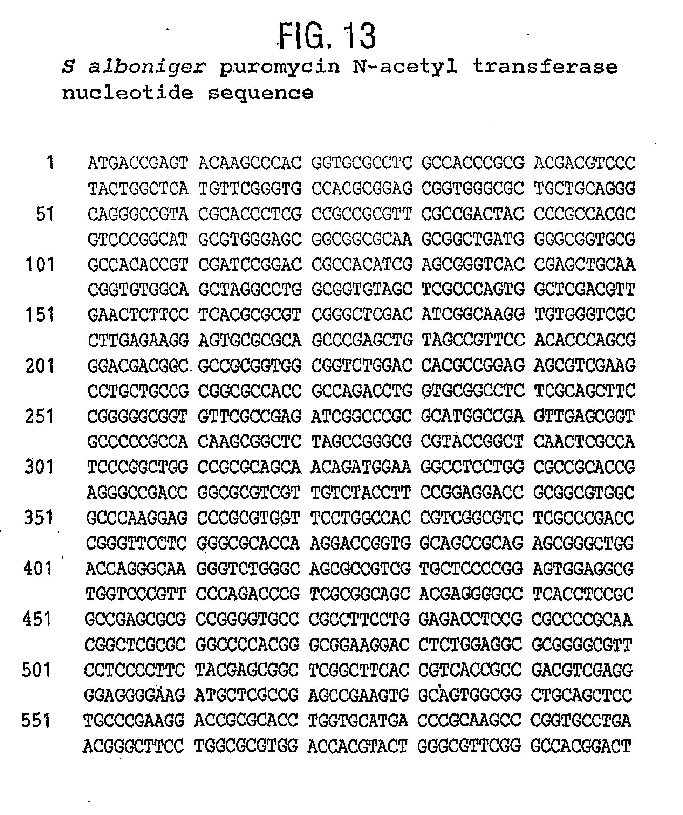

**Figure 13** shows the nucleotide sequence of the wild-type *S*. *alboniger* puromycin N-acetyl transferase gene.
**Figure 14** shows the nucleotide sequence of the modified *S*. *alboniger* puromycin N-acetyl transferase gene.
**Figure 15** shows the nucleotide sequence of the *S. fradiae* aminoglycoside phosphotransferase gene.

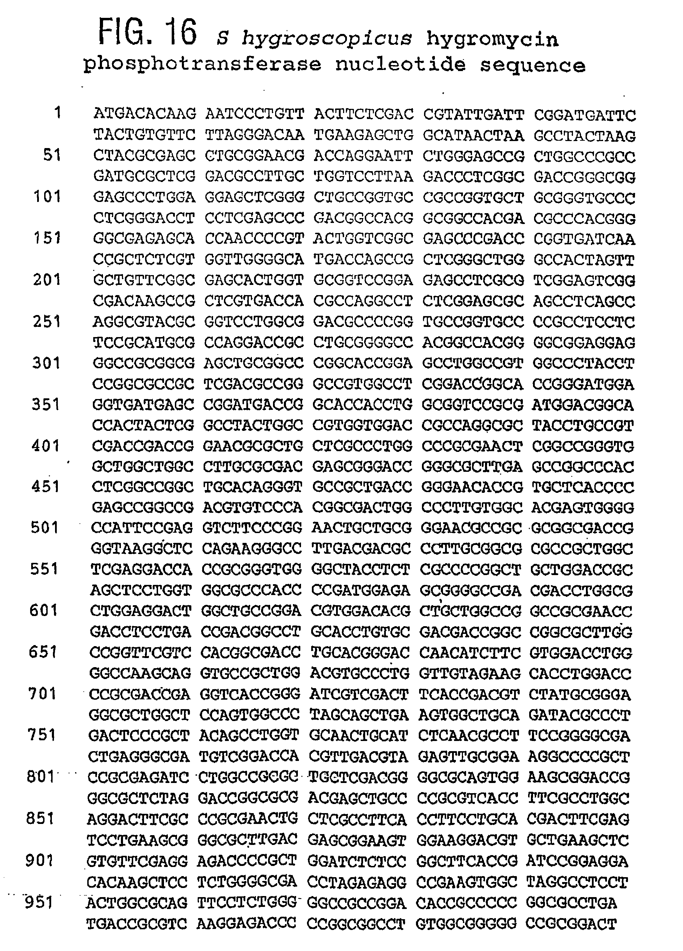

**Figure 16** shows the nucleotide sequence of the *S*. *hygroscopicus* hygromycin phosphotransferase gene.
**Figure 17** shows the nucleotide sequence of the *E*.*coli* aminocyclitol phosphotransferase (hygro^{r}) gene.

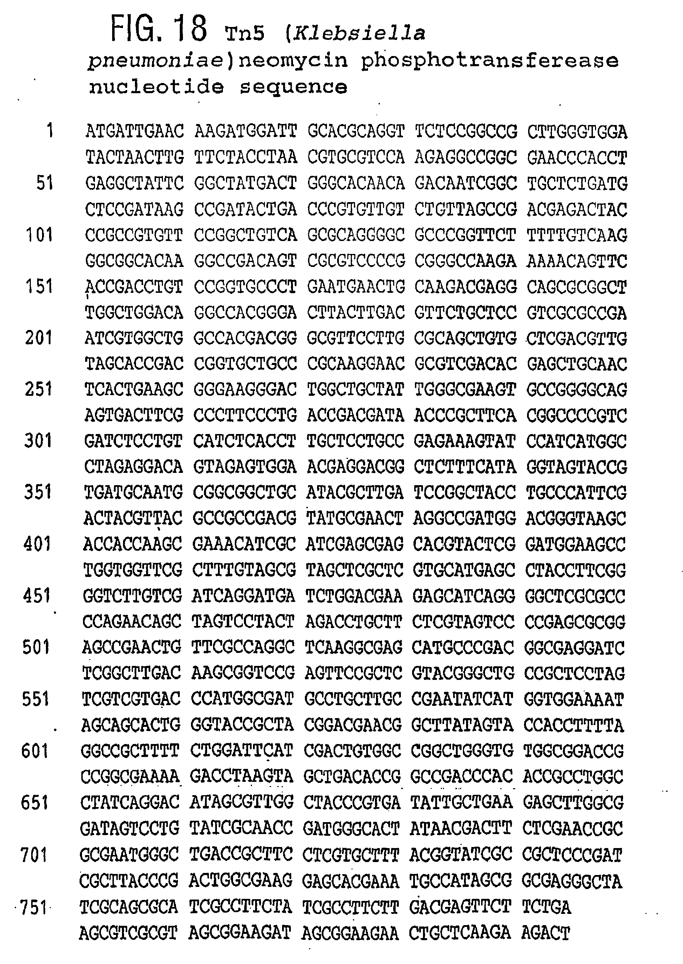

**Figure 18** shows the nucleotide sequence of the transposon Tn5 *(Klebsiella pneumoniae*) neomycin phosphotransferase gene.
**Figure 19** shows the nucleotide sequence of the mouse hnRNP A2 *Hind*III fragment.

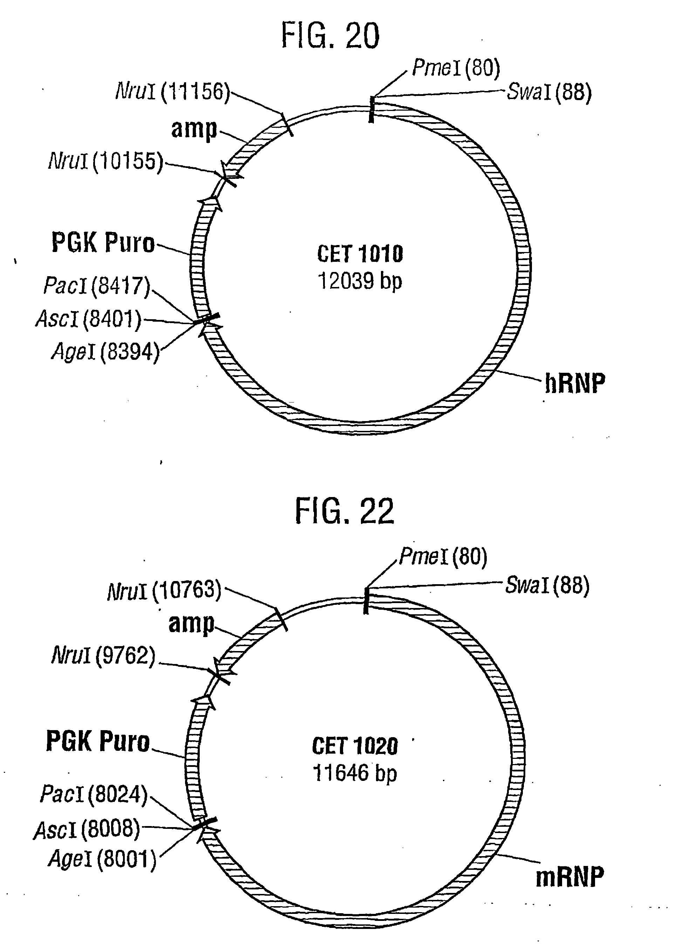

**Figure 20** shows a map of plasmid CET 1010
**Figure 21** shows the nucleotide sequence of CET1010
**Figure 22** shows a map of plasmid CET 1020
**Figure 23** shows the nucleotide sequence of CET1020
**Figure 24** shows a map of plasmid CET 1030
**Figure 25** shows the nucleotide sequence of CET1030

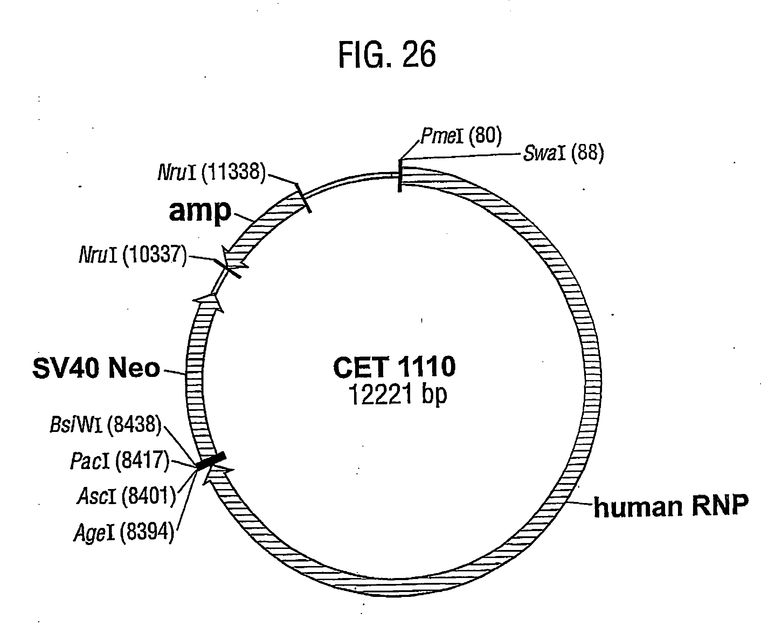

**Figure 26** shows a map of plasmid CET 1110
**Figure 27** shows the nucleotide sequence of CET1110

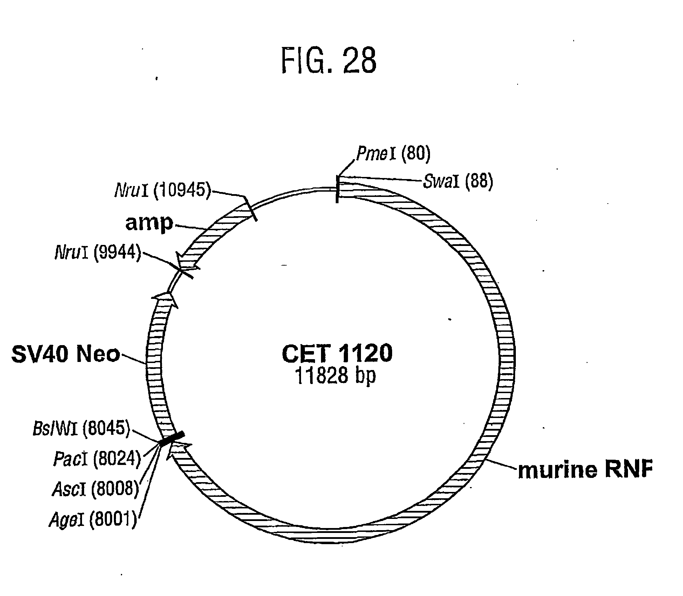

**Figure 28** shows a map of plasmid CET 1120
**Figure 29** shows the nucleotide sequence of CET1120

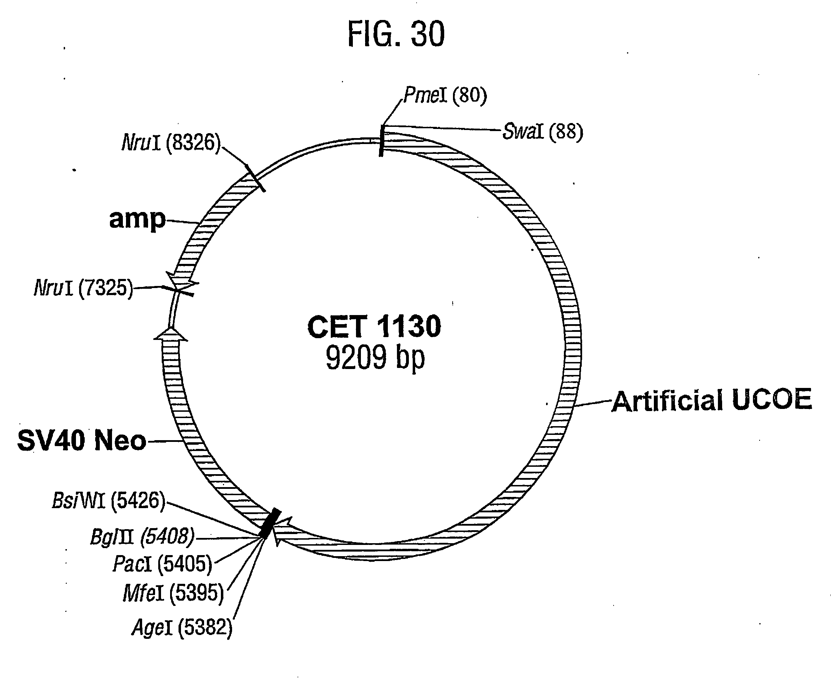

**Figure 30** shows a map of plasmid CET 1130
**Figure 31** shows the nucleotide sequence of CET1130

### EXAMPLES

### Example 1 Flanking of an expressible gene with UCOEs and selectable elements

### Materials and Methods

### Construction of PGK-Puro CET expression Vectors

### CET700

The CMV-MCS-SV40pA cassette was removed from CET31 (A CMV MCS pA SV40Neo based plasmid) as an *Ase*I/*Afl*II fragment, blunt end filled with T4 DNA polymerase and ligated into pPGK-Puro (mPGK promoter, Puromycin resistance gene, bGHpA in pBluescript) that had been digested with *Eco*RV.

### CET 720

CET20 (8.3kb hnRNPA2 fragment in pBluescript) was digested with *Hind*III to obtain the 8kb RNP UCOE and this was then ligated Into CET700 that had also been cut with *Hin*dIII.

### CET710

The Artificial UCOE was removed from CET 21 (Artificial UCOE in pBluescript) as an *Xba*I/*Cla*I fragment, blunt end filled with T4 DNA polymerase and ligated into CET700 that had been digested with *Hind*III and again blunt end filled with T4 DNA polymerase.

### CET 230

This vector was constructed by digesting pUC19 with *Nar*I an *Eco*RI to remove approximately 160bp, followed by blunting and religation. This removed one of the two *Pvu*I and *Pvu*II sites in the vector backbone. The CMV-EGFP-SV40pA cassette (with its MCS deleted) was excised from pEGFPN-1 (Clontech), as an *Ase*I/*Afl*II digest followed by blunt end filling, and then inserted into the pUC19 vector backbone that had been digested with *Nde*I and Eco109 I and again blunt end filled.

The PGK-Puro-bGpA cassette was then removed from pPGK-Puro as an *Eco*RI/*Xh*oI blunt end filled fragment and inserted into the unique *Pvu*II site of the above vector. Finally the 8.3kb hnRNPA2 fragment was inserted into the unique *Hind*III site of this vector as a *Hin*dIII fragment derived from CET20.

For clarity:
CET230 is the EGFP-expressing version of the 'empty' vector CET21 0
CET711 is the EGFP-expressing version of the 'empty' vector CET710
CET721 is the EGFP-expressing version of the 'empty' vector CET720

Vectors based on CET 720 with different antibiotic resistant genes and with alternative promoters or UCOEs can be constructed in the following manner. The PGK promoter (bp11384-11894) and the bghpA (bp 12567-12893) can be removed from CET 720 by restriction digestion. These elements can be inserted into the pBluescript backbone such that restriction sites are available for the insertion of any resistance gene sequences (derived by PCR or restriction digestion) between the PGK promoter and the bghpA in such a manner as to allow expression of that gene. The CMV-MCS-SV40pA expression cassette can also be removed from CET 720 (bp 10533-19380) and inserted 5' to the PGK promoter in the above vector; alternatively the mCMV-MCS-SV40pA expression cassette can be placed in the same position (CET 801, 821, 824-EGFP expression versions). The hnRNPA2 UCOE can be removed from CET 720 (bp 2240-10525) by restriction digestion and inserted 5' to the CMV expression cassette in the above vectors, alternatively other UCOEs (e.g. murine hnRNPA2) can be inserted into the same position (CET 824-EGFP expression version).

### For clarity:

CET741 is the EGFP-expressing version of the 'empty' vector CET740 and comprises a 5' human RNP UCOE and a 3' *S fradiae* neo^{r} gene.

CET761 is the EGFP-expressing version of the 'empty' vector CET760 and comprises a 5' human RNP UCOE and a 3' *E.coli* aminocyclitol phosphotransferase (hygro^{r}) gene.

CET781 Is the EGFP-expressing version of the 'empty' vector CET780 and comprises a 5' human RNP UCOE and a 3' modified *S. alboniger* puromycin N-acetyl transferase gene.

CET821 is the EGFP-expressing version of the 'empty' vector CET820 and comprises a 5' human RNP UCOE and a 3' wild-type *S*. *alboniger* puromycin N-acetyl transferase gene. Expression of the EGFP transgene is driven by the murine (rather than human) CMV IE promoter.

CET824 is the EGFP-expressing version of the 'empty' vector CET823 and comprises a 5' murine (rather than human) RNP UCOE and a 3' wild-type S. *alboniger* puromycin N-acetyl transferase gene.

### pCIA vectors

This is a series of vectors that easily allow the construction of UCOE expression vectors with the final optimal configuration (UCOE-expression cassette-resistance cassette) when integrated Into the chromosome.

CET 900 is an empty cloning vector in which pairs of rare restriction sites flank the MCS. CET 901 and CET 902 contain the hCMV and mCMV promoters respectively, an MCS and the SV40pA. The same pairs of rare restriction sites also flank these cassettes.

The CET 1000 series of vectors contain various combinations of UCOEs and resistance expression cassettes. They also contain the same rare restriction sites as the CET 900 series at a position 3' to the UCOE and 5' to the resistance cassette. The vectors also contain linearisation sites 5' to the UCOE and 3' to the resistance cassette.

Expression cassettes for any transgene can therefore be constructed in the CET 900 series and then easily be transferred into the CET 1000 series such that the ultimate configuration when integrated into the chromosome is the desired UCOE-expression cassette-resistance cassette.

As described above the antibiotic gene can be exchanged within the CET 1000 series by restriction digestion or PCR.

### Transfection

CHO K1 cells where transfected and selected according to standard methods and as described in the co-pending applications incorporated by reference.

### Results

With particular reference to Figure 2, comparison of cells transfected with CET 721 and CET 230 shows a consistently higher level of expression obtained with CET 721. These two vectors are similar in that both carry an 8kb hnRNP-derived UCOE operably-linked to the CMV promoter driven EGFP gene and both carry the *pgk*/puromycin resistance gene element. However, following linearisation with *Pvu* I, integration of CET 230 into the host cell chromosome results in the elements being positioned in the order: pgkIPuro, hnRNP UCOE, EGFP gene. The same process with CET 721 results in the EGFP gene being flanked by the UCOE and the *pgk*/Puro. The levels of expression obtained with CET 230 are not significantly higher than those obtained with CET 220, a vector carrying no *pgk*/Puro element but with the same UCOE and promoter driving EGFP expression. All UCOE carrying vectors show greatly increased expression compared with the basic EGFP expression plasmid.

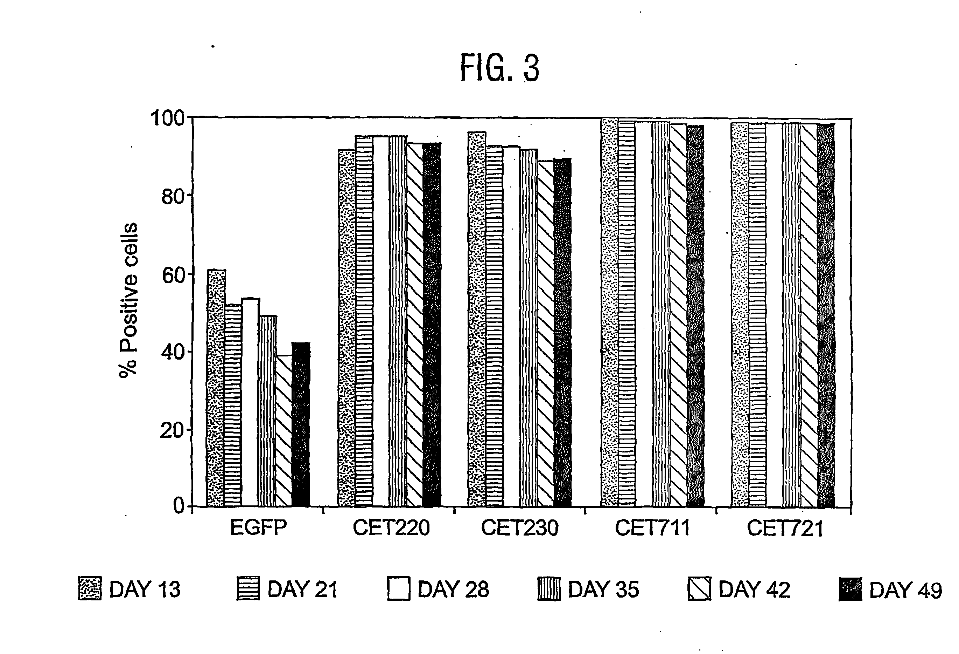

Figure 3 shows that increased expression as expressed by median fluorescence is also reflected in an increased proportion of cells within the transfected population judged to be positive, in terms of expression, at all time points following transfection. This is a measure of the lack of position effects, since random integration of the construct would normally result in a range of expression levels within the (non-clonal) population of transfected cells. This is overcome by the combination of 5' UCOE and 3' selectable element, resulting in a homogenous, highly-expressing population.

The levels of expression in some of the pools of cells in Figure 2 are so high that the fluorescence produced has exceeded the capacity of the detector. In Figure 4, measurements have been corrected to the linear region of the detector's response to allow comparison between constructs. This shows that the combination of UCOE and 3' flanking selectable element used in CET 721 has produced an approximately 7-fold Increase in levels of expression of EGFP as compared with that obtained with the UCOE alone (CET220) or that obtained with the selectable element (puro^{r}) placed 5' to the UCOE. It is clear that flanking the expressed transgene with the UCOE and selectable marker Is required to obtain the boost in expression.

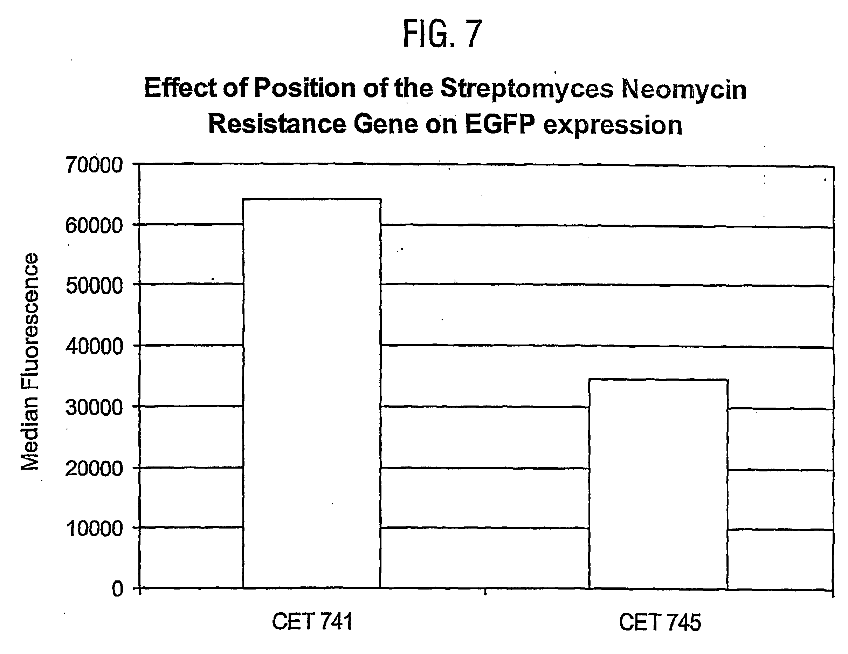

This effect is not restricted to a particular selectable marker. Figure 7 compares expression of EGFP operable linked to a 5' human RNP UCOE and either a 5' (CET745) or 3' (CET741) placed *S*. *fradiae* neomycin resistance gene. There is almost a doubling of the already high expression level.

### Example 2 Effectiveness of other 3' flanking selectable markers Results

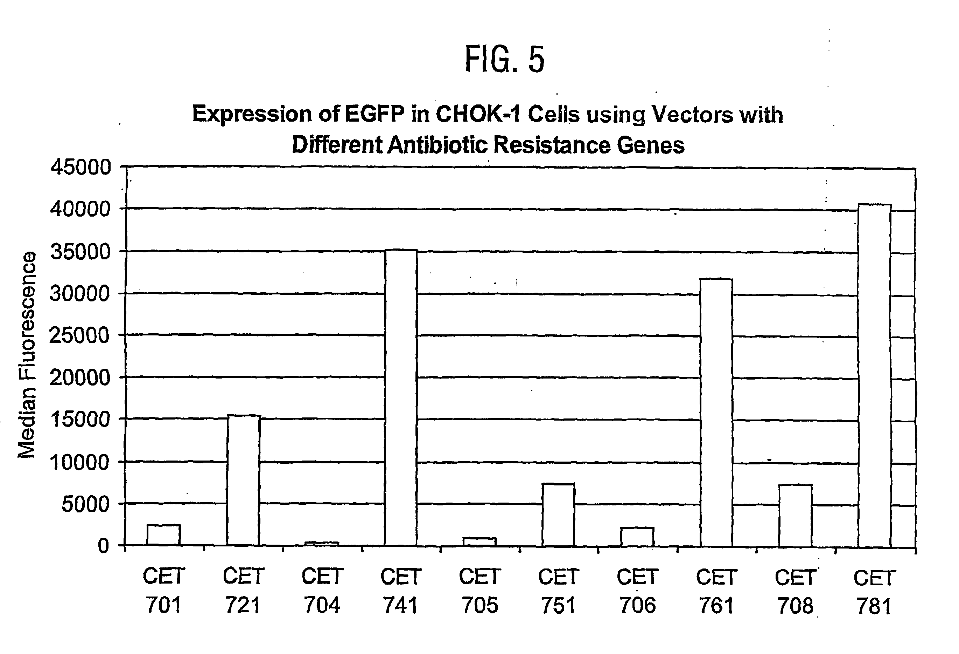

Figure 5 shows the effect of flanking the EGFP transgene with a 5' human RNP UCOE and various 3' flanking antibiotic resistance genes. CET701 is a control containing no UCOE, but with the wild-type *S alboniger* puro^{r}. CET 721 has both the 5' UCOE and 3' puro^{r}. CET704 contains the *S fradiae* neo^{r} but no UCOE, CET741 has both. CET705 contains the *S hygroscopicus* hygro^{r} but no UCOE, CET751 has both. CET706 has the *E coli* hygro^{r} but no UCOE, CET761 has both. CET708 has the codon-modified puro^{r} but no UCOE, CET781 has both. In all cases the boosting effect of the 3' flanking resistance gene is evident.

### Example 3 Combination of other UCOEs and Puro selectable element

### Results

As shown in Figures 2 and 3, expression from a comparable plasmid carrying an artificially constructed UCOE (CET 711) was comparable to that obtained with the RNP UCOE both in terms of median fluorescence and proportion of positive cells. This demonstrates that the phenomenon of amplification of the effect of a UCOE by a second flanking CpG-rich element is a general one, not confined to a particular combination of the RNP UCOE and the *pgk*/Puro element. The comparison of CET 711 and CET 721 expression in Figure 4 indicates a slightly lower level of expression was obtained with CET 711, but this was still at least 6-fold higher than that obtained with a UCOE alone.

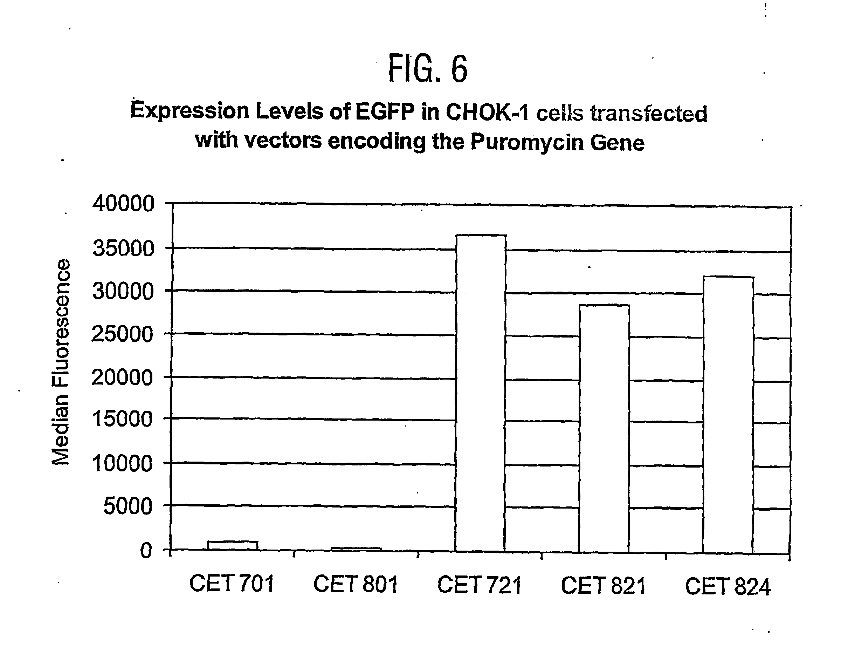

Figure 6 shows the comparable effect obtained with either a human hnRNP UCOE using the murine CMV promoter to drive expression (CET821) and the murine equivalent (CET824). CET721 comprises the human hnRNP UCOE and uses the human CMV promoter.

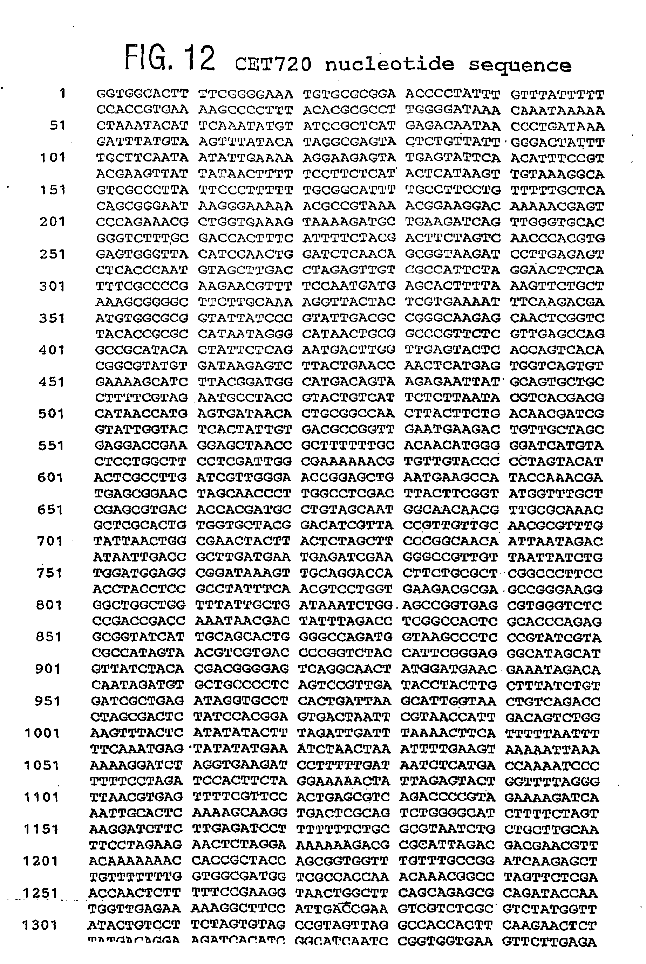

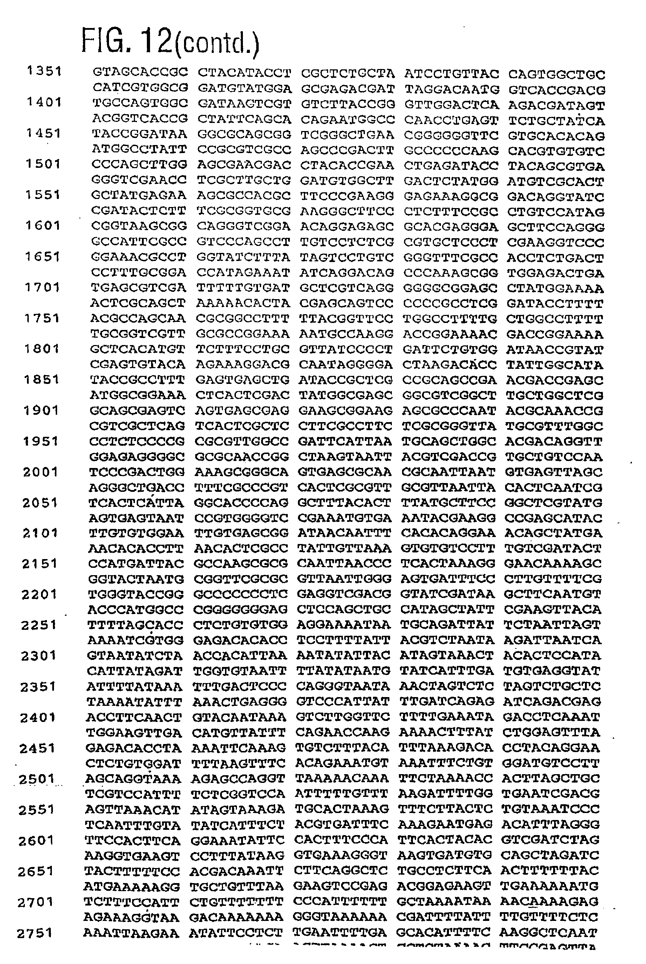

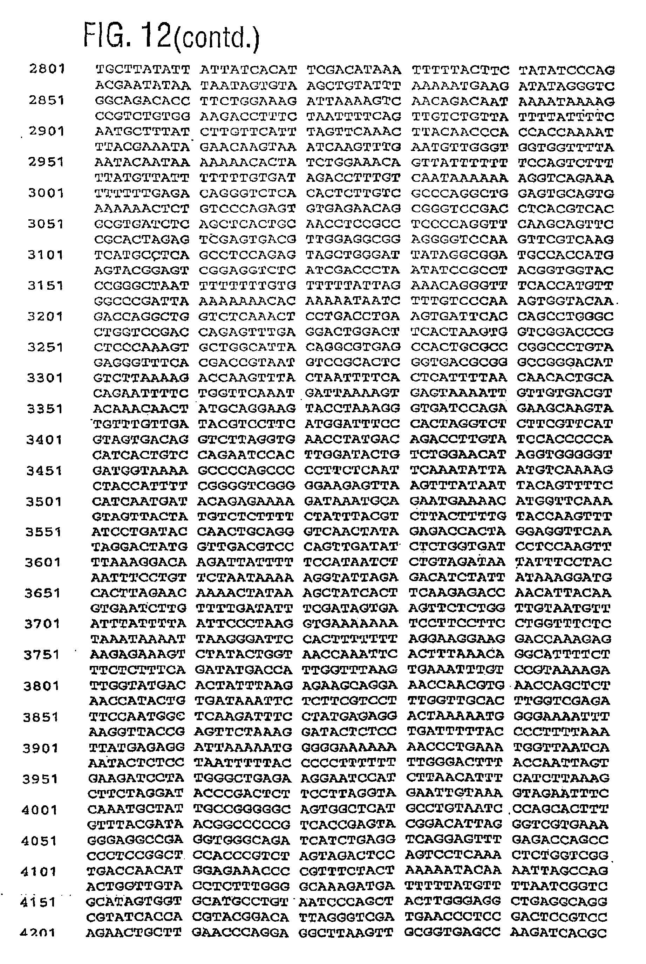

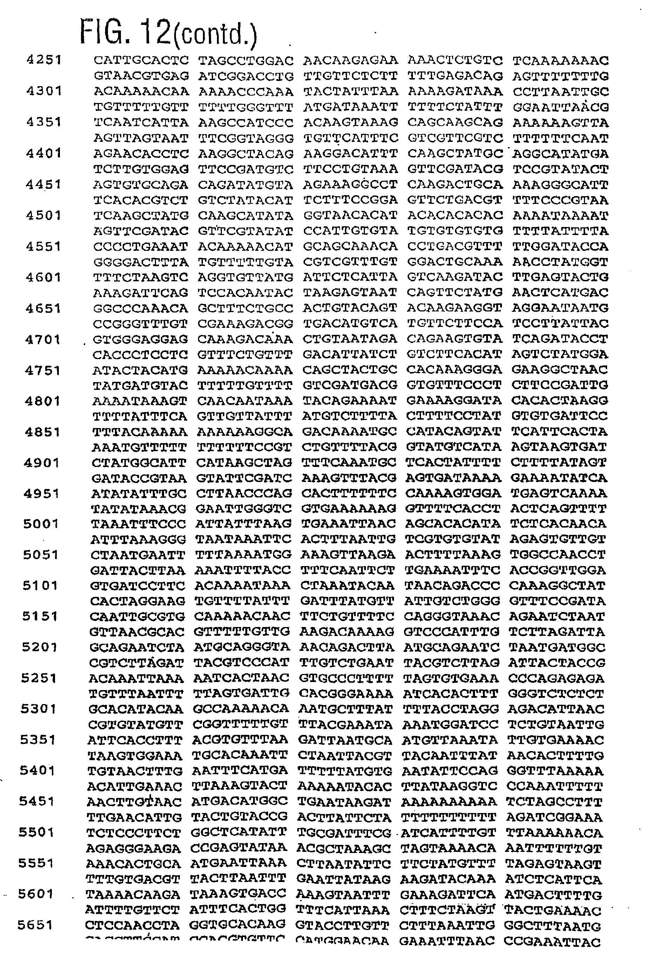

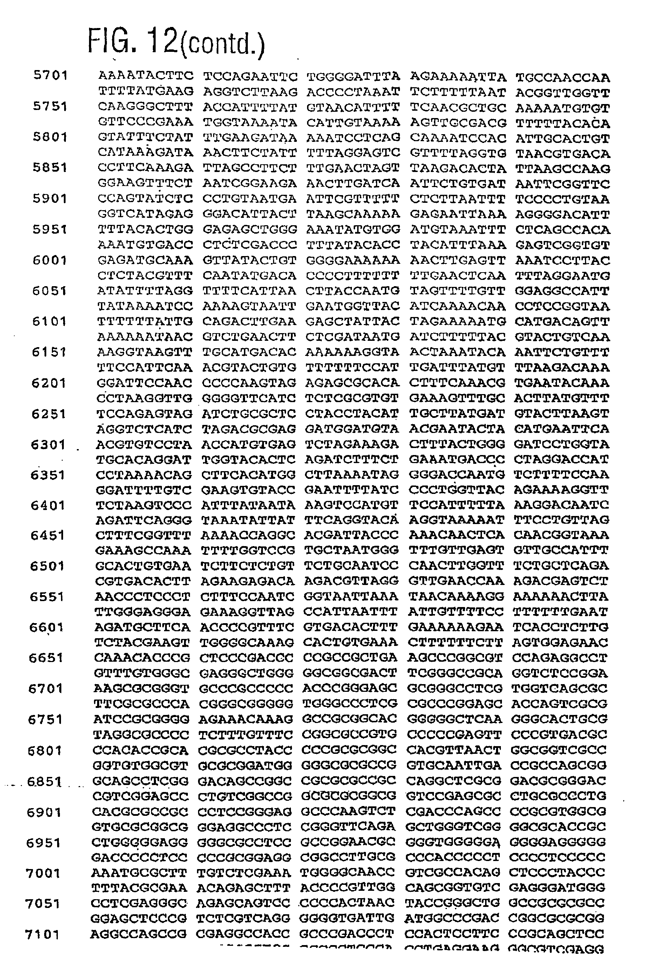

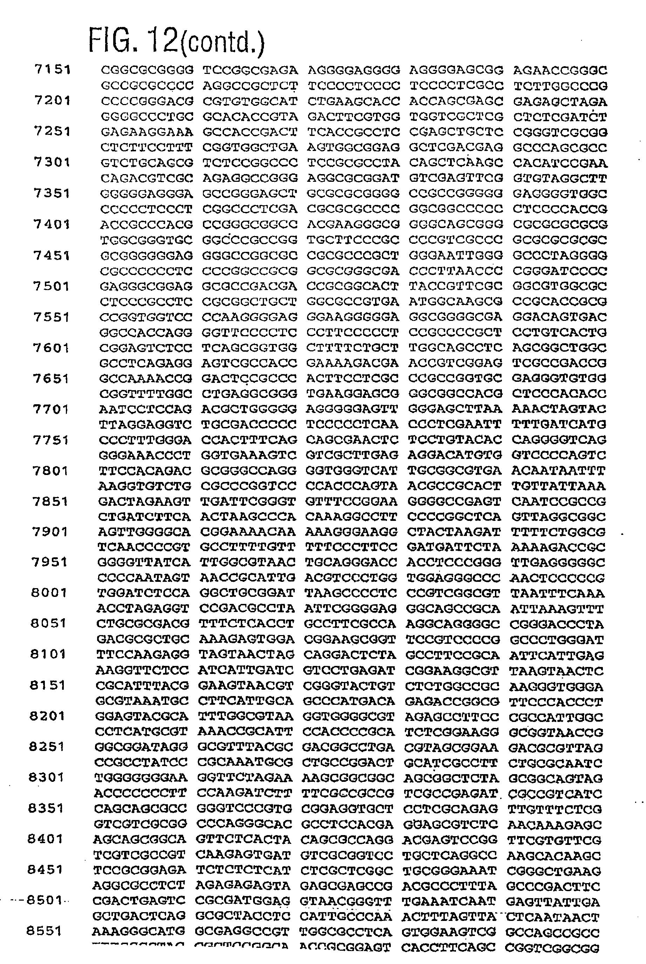

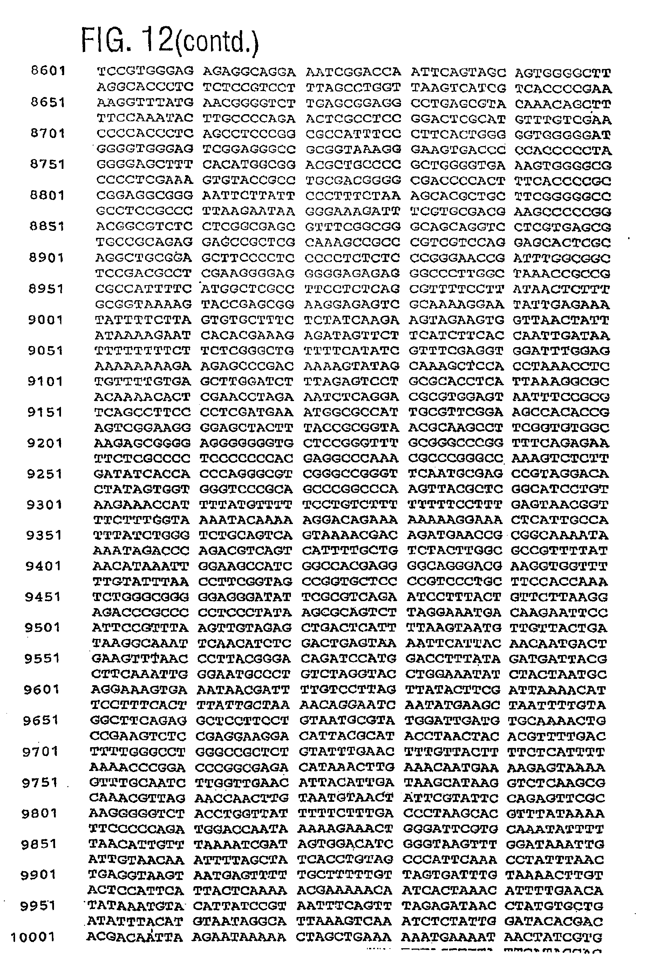

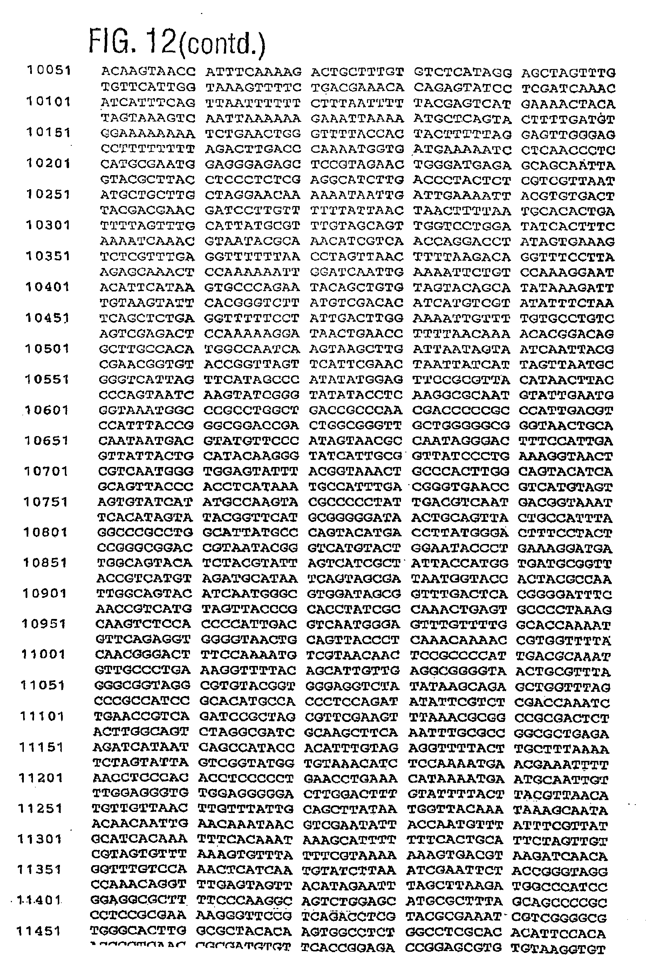

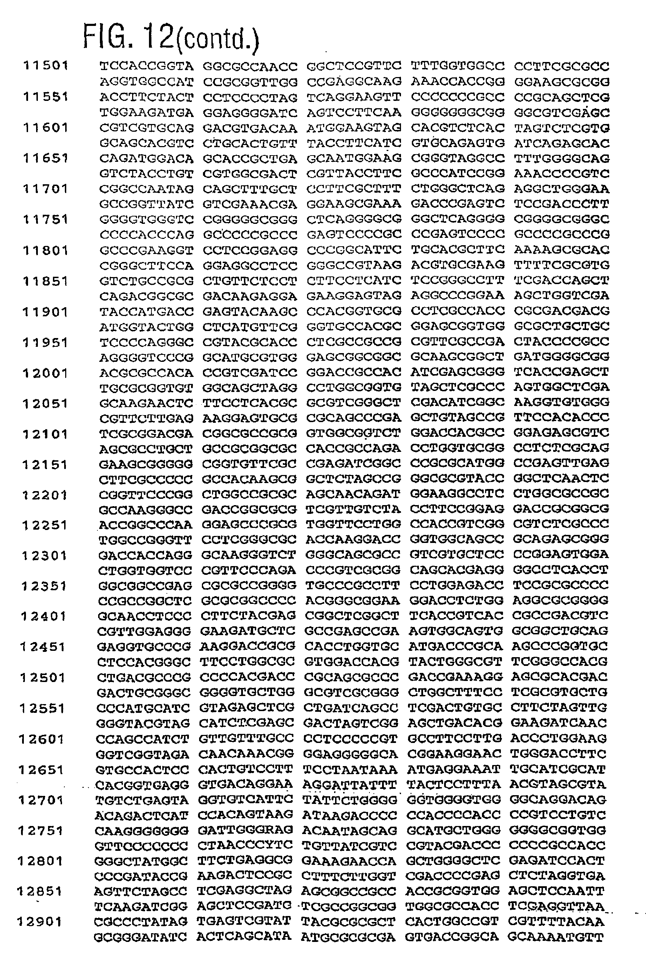

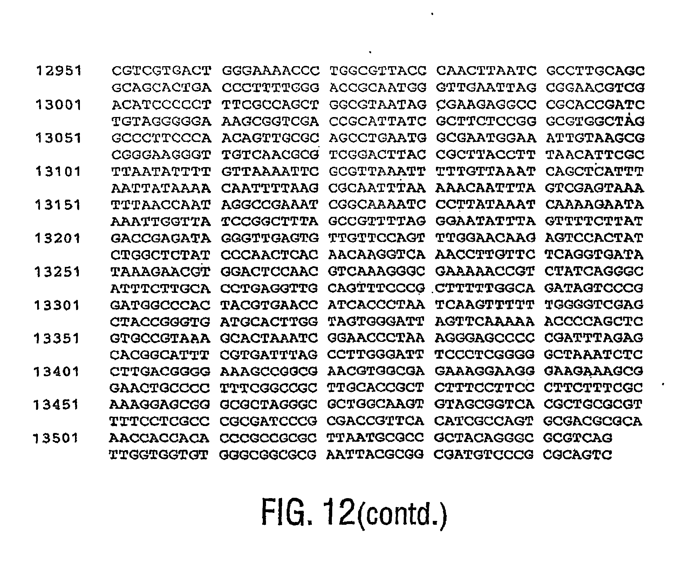

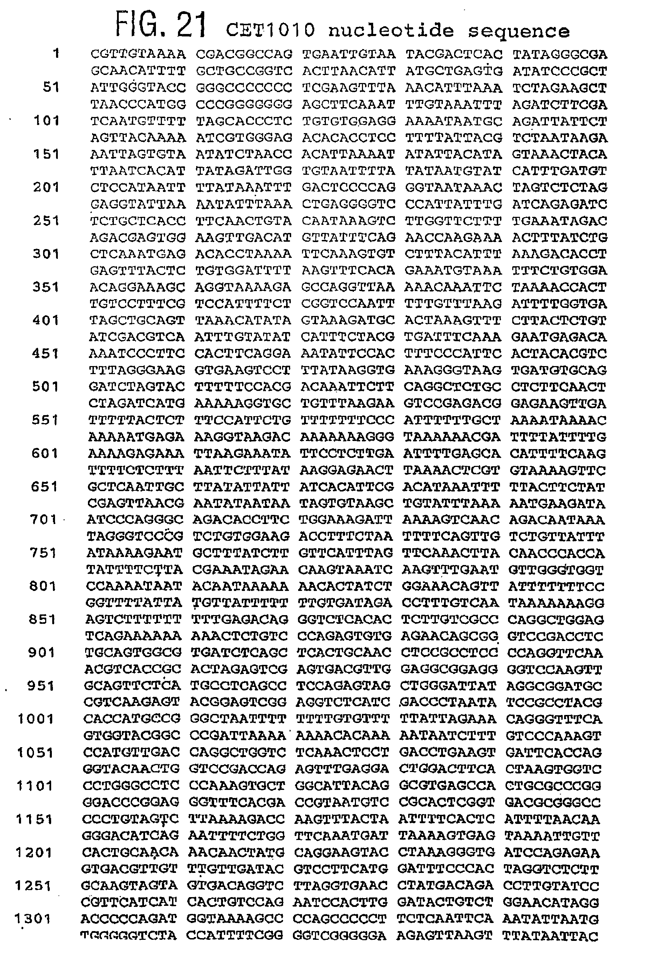

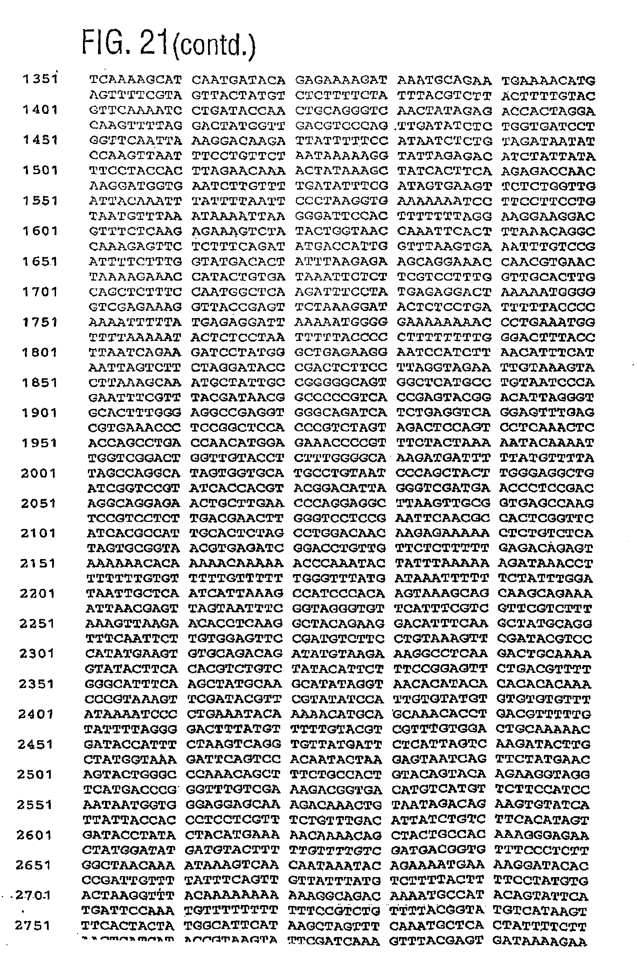

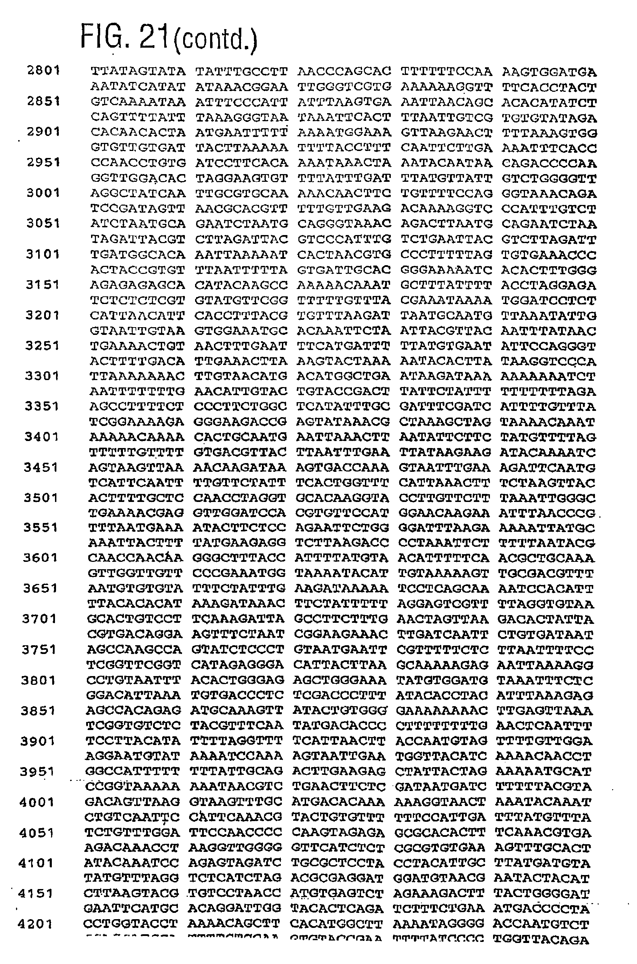

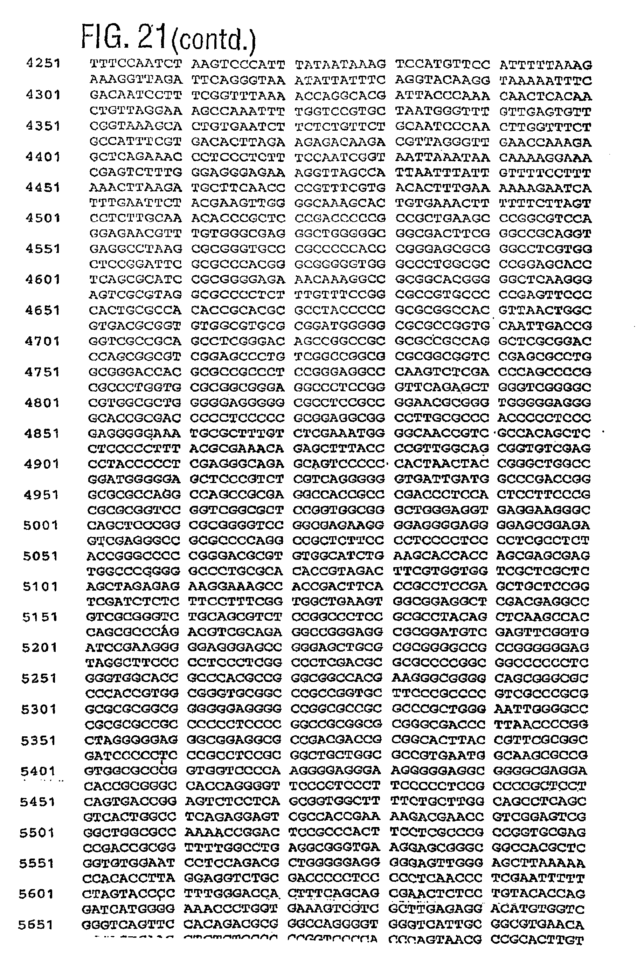

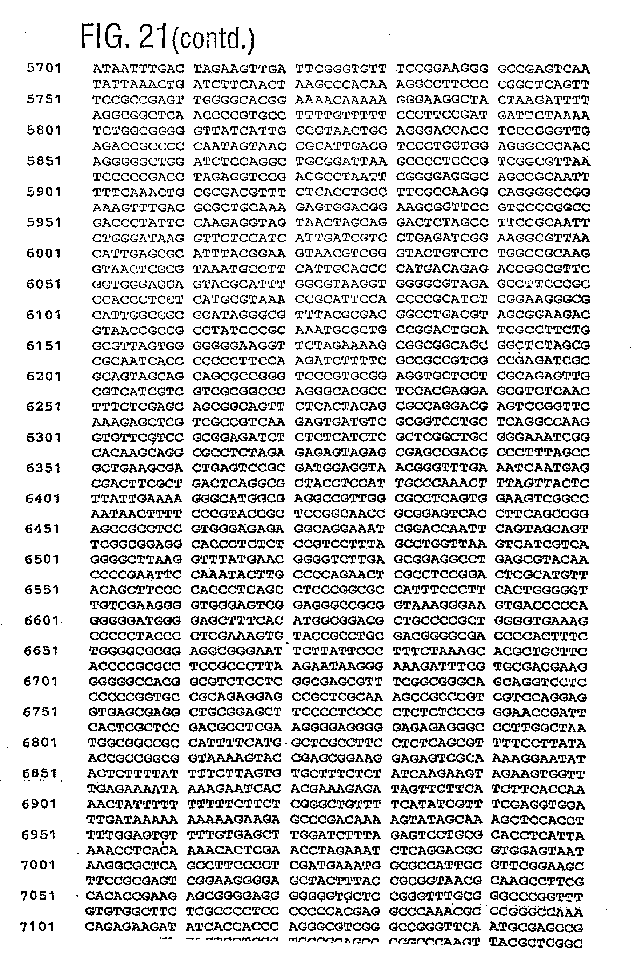

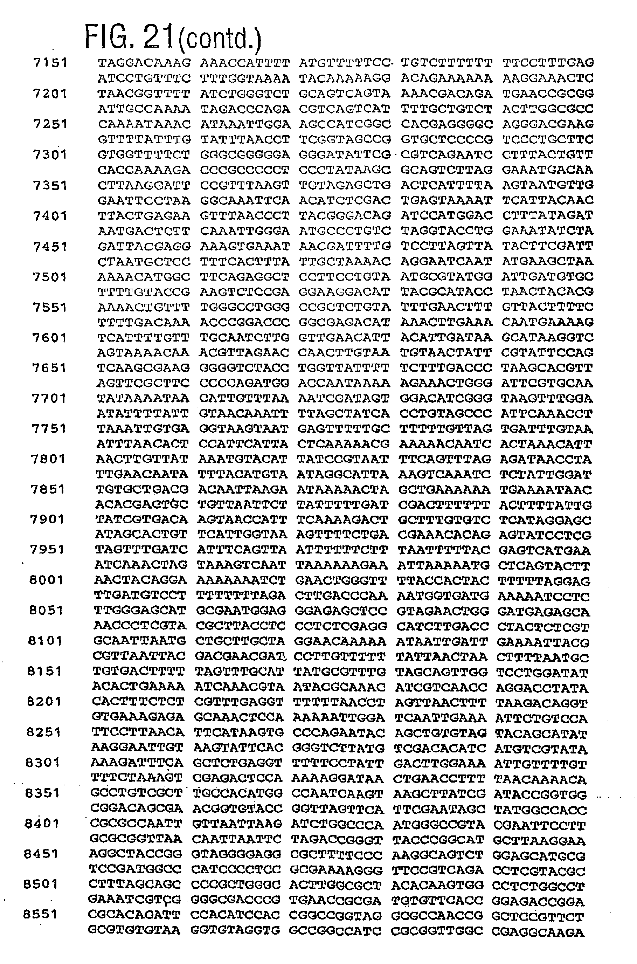

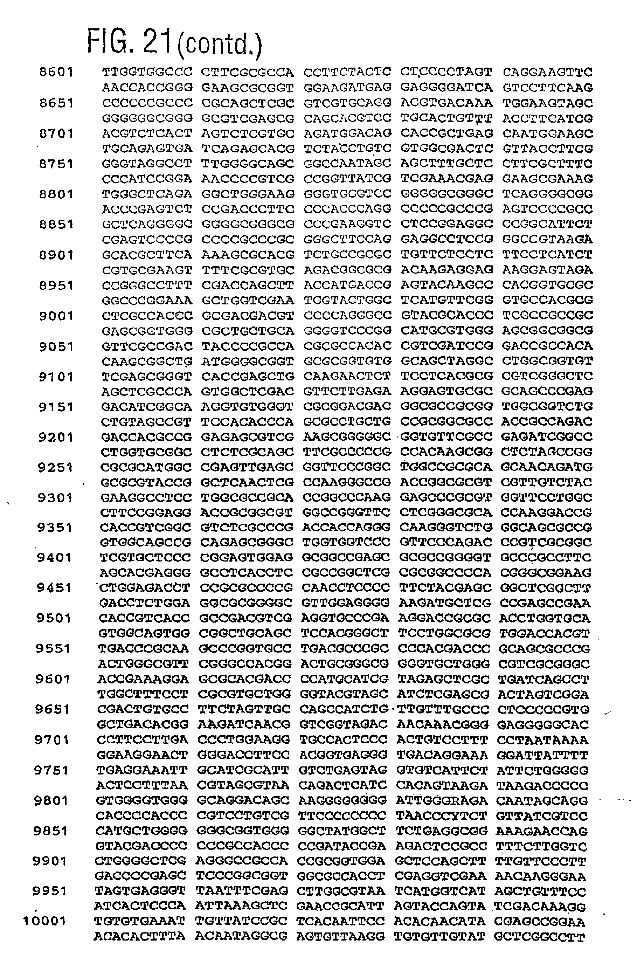

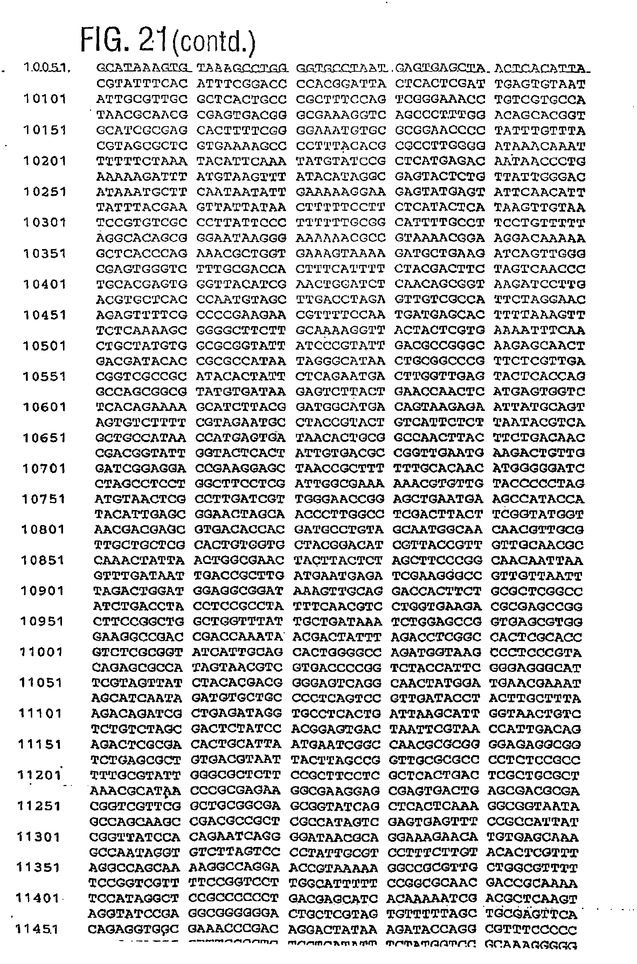

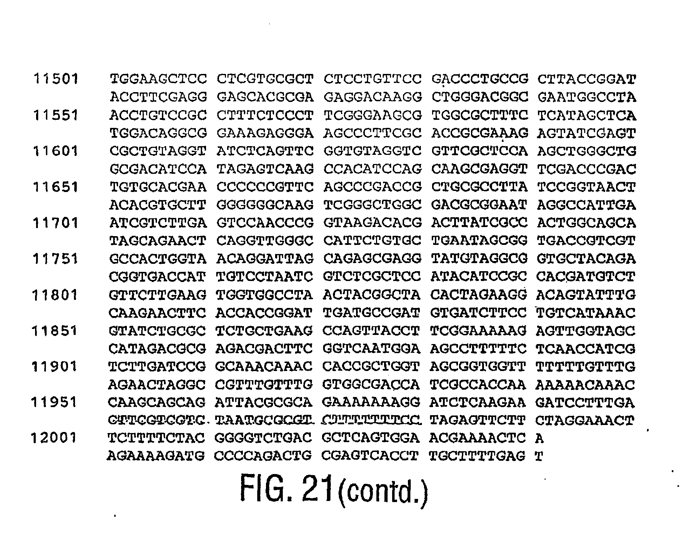

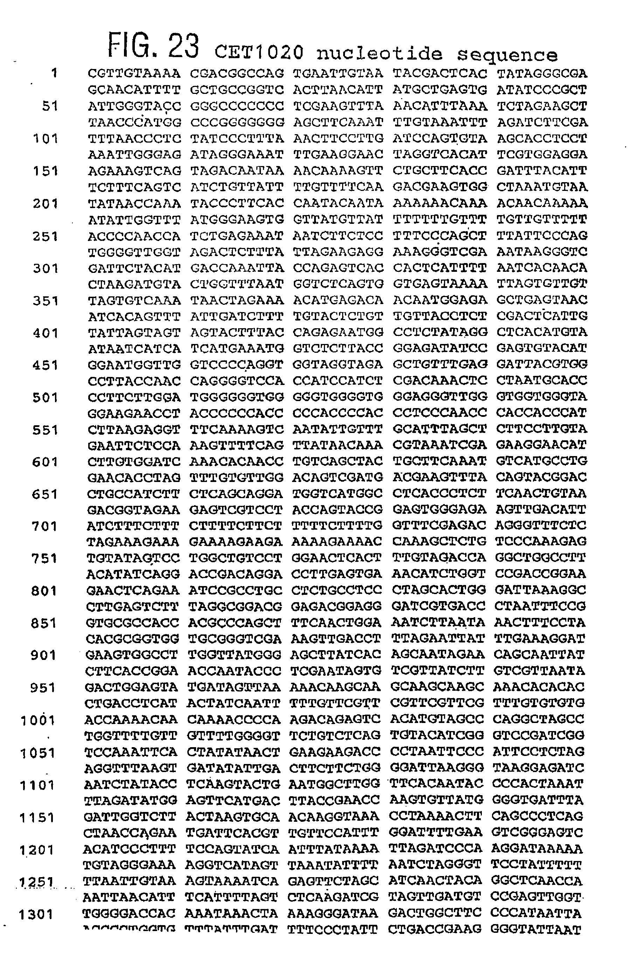

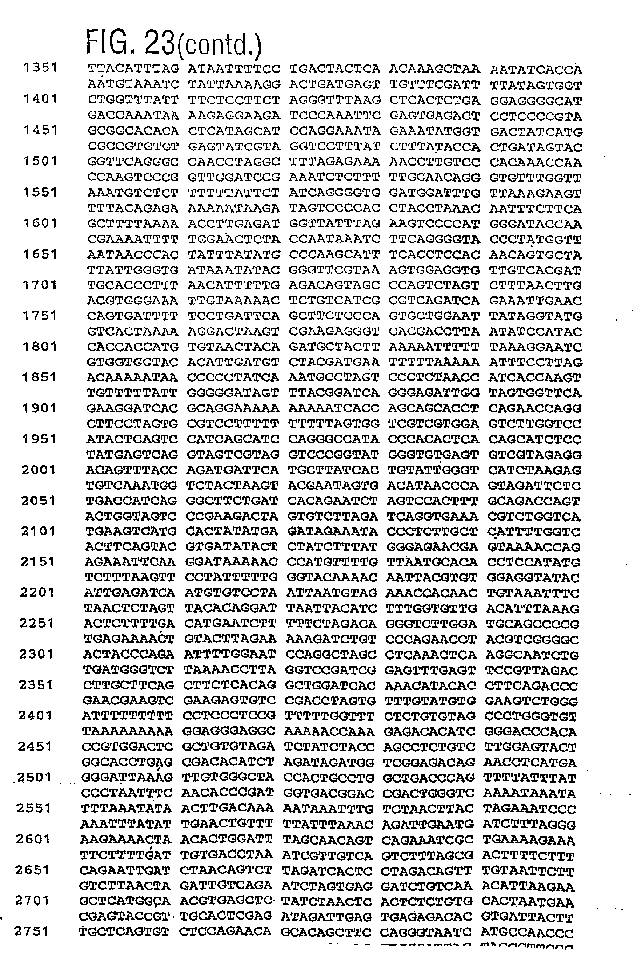

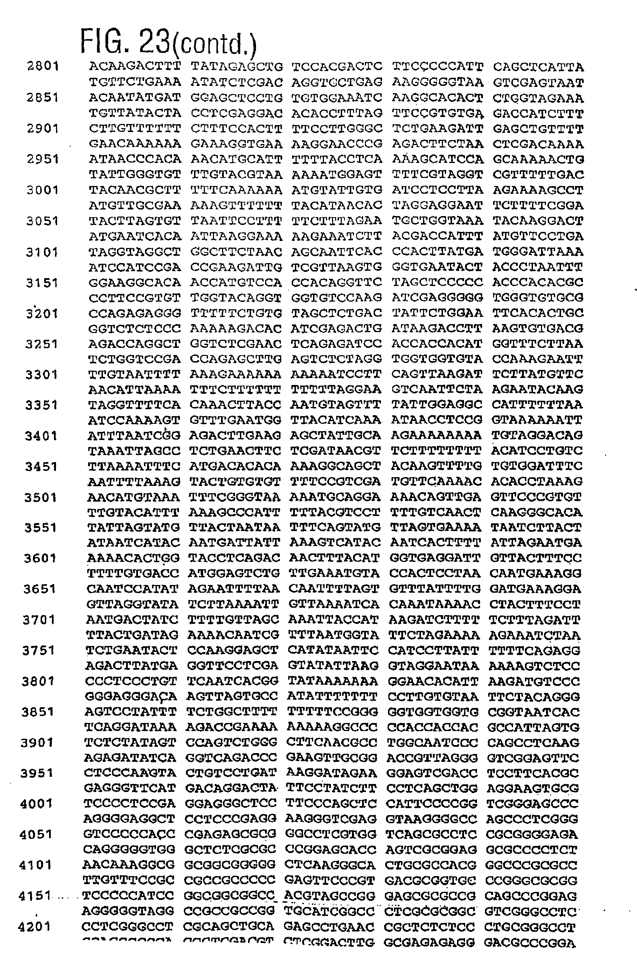

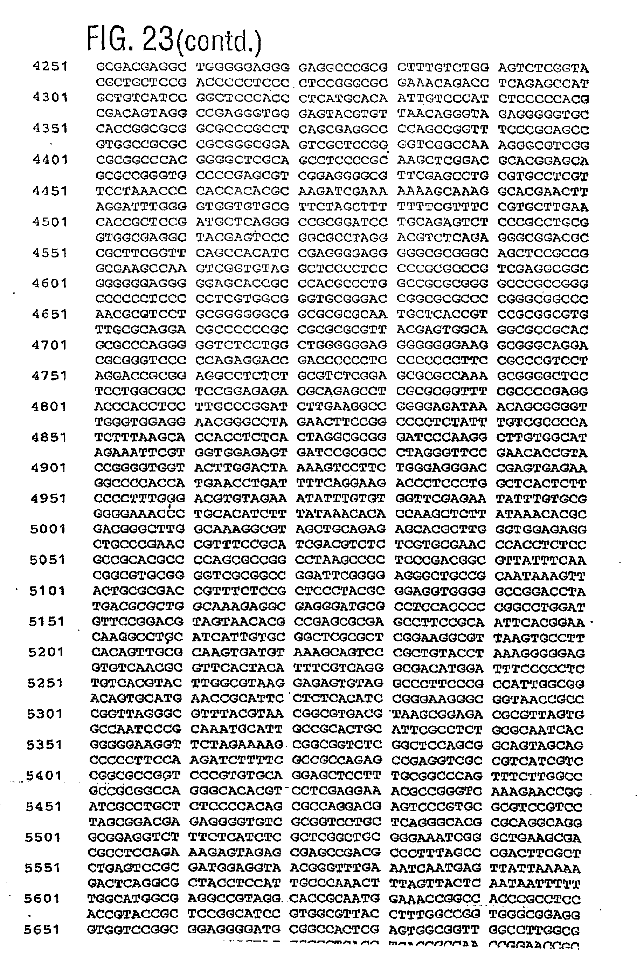

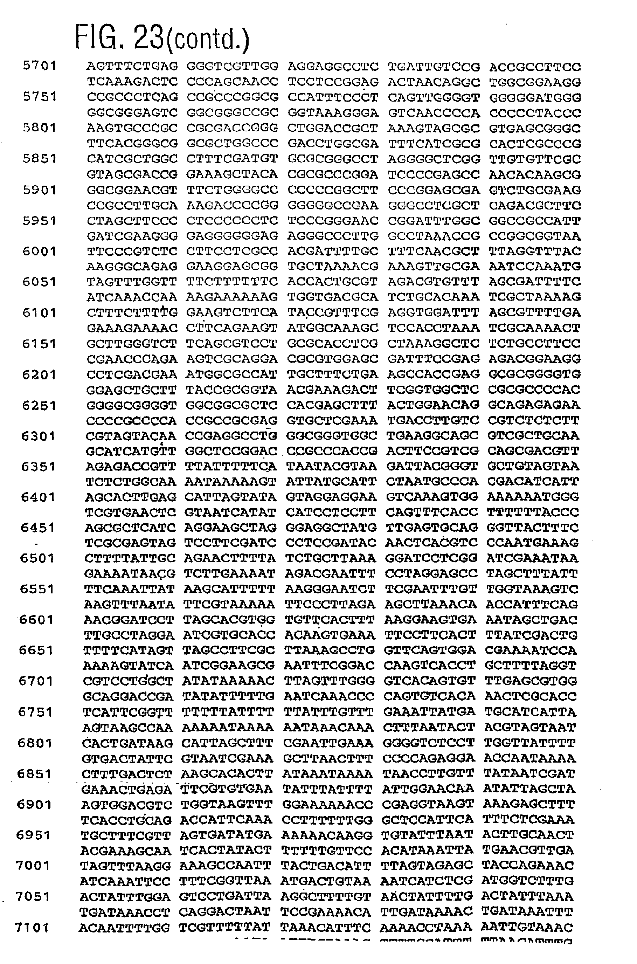

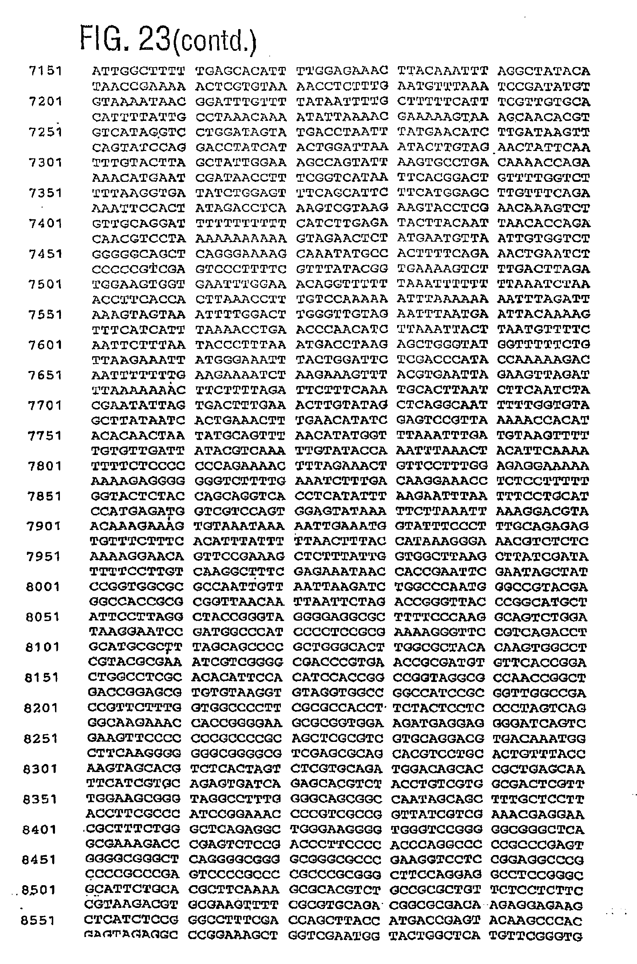

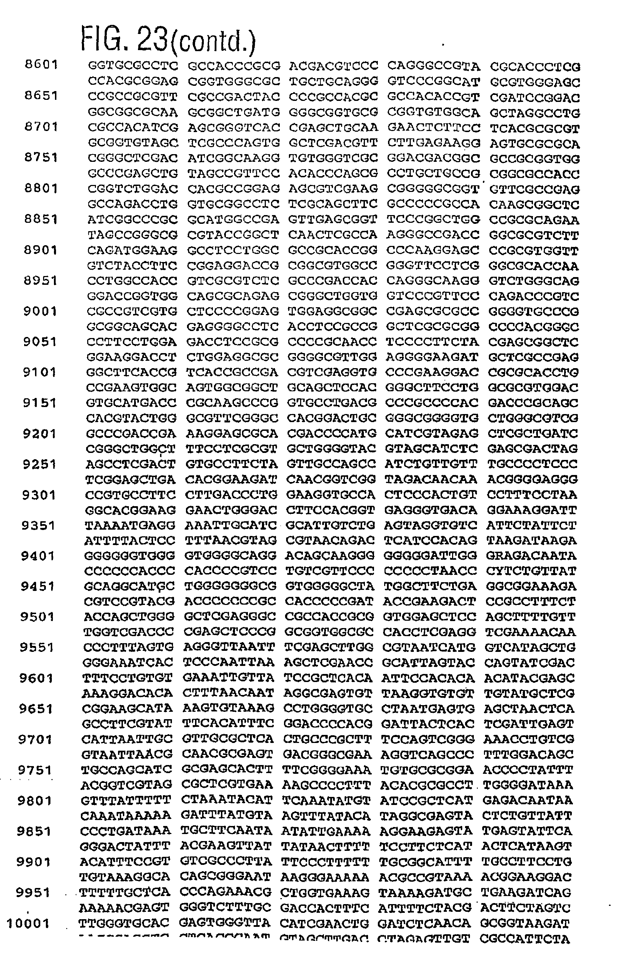

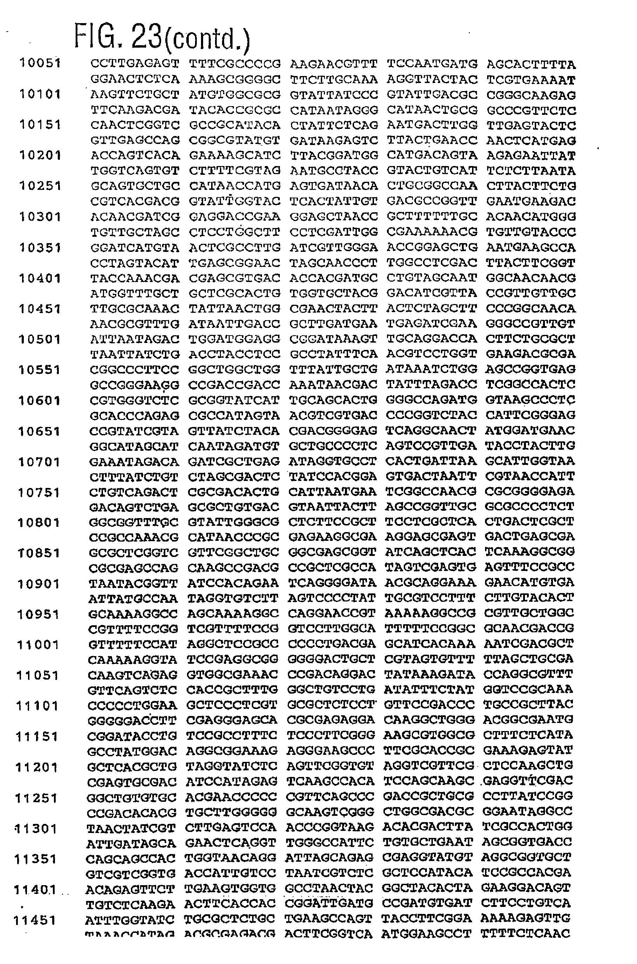

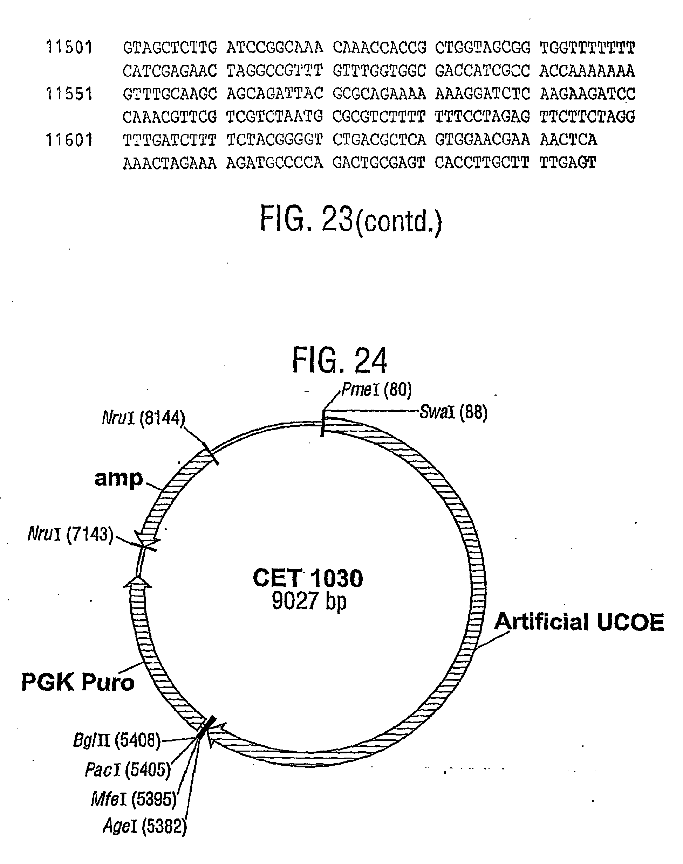

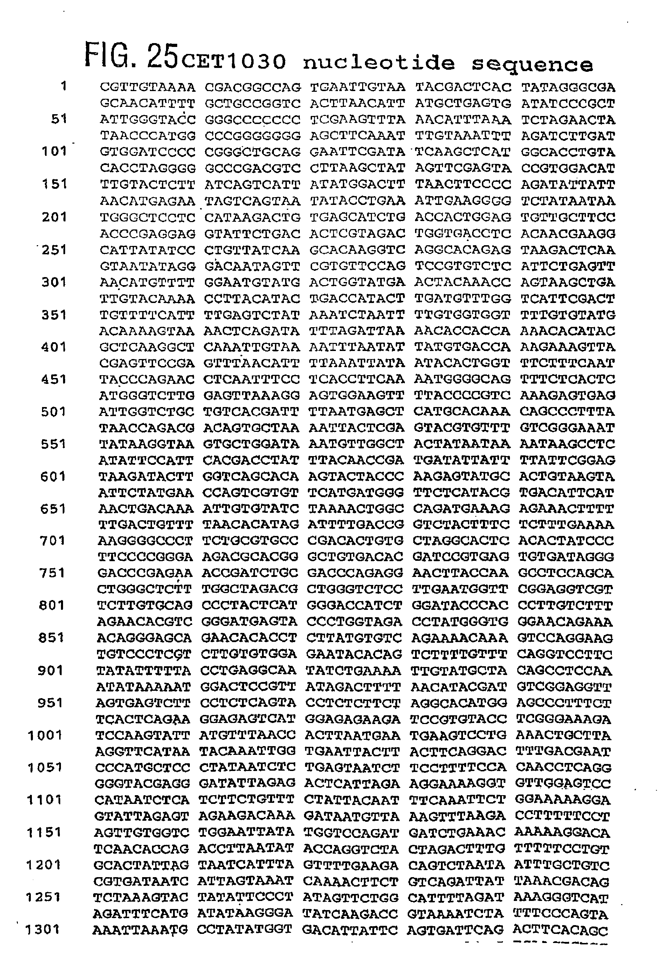

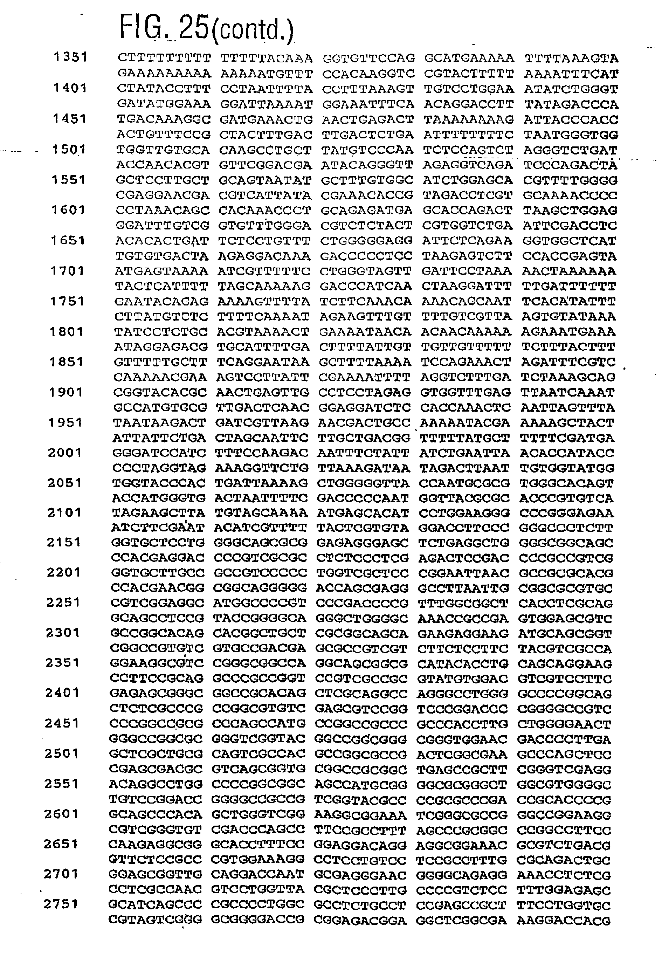

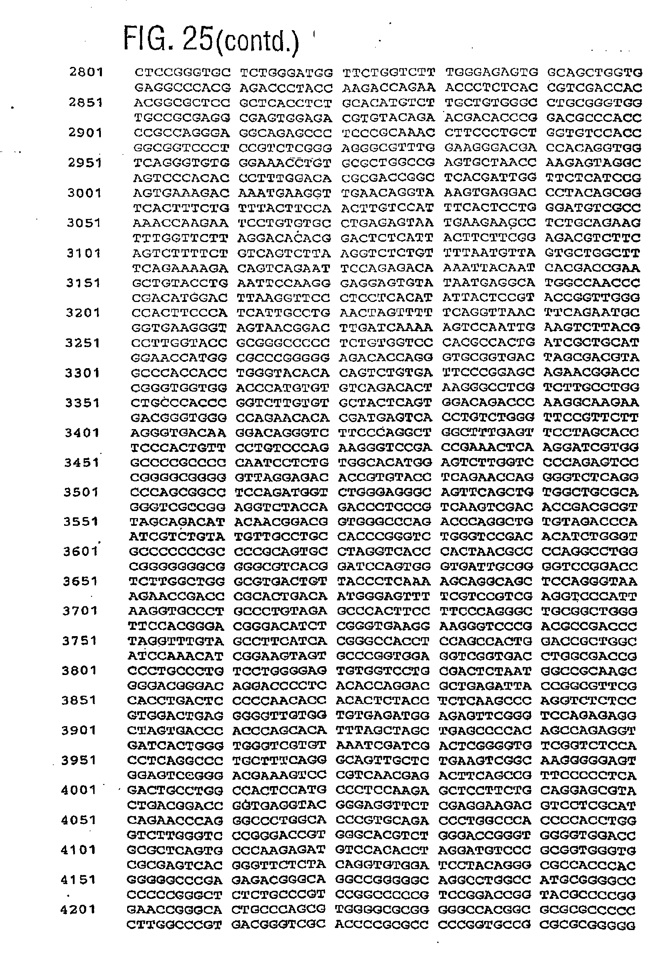

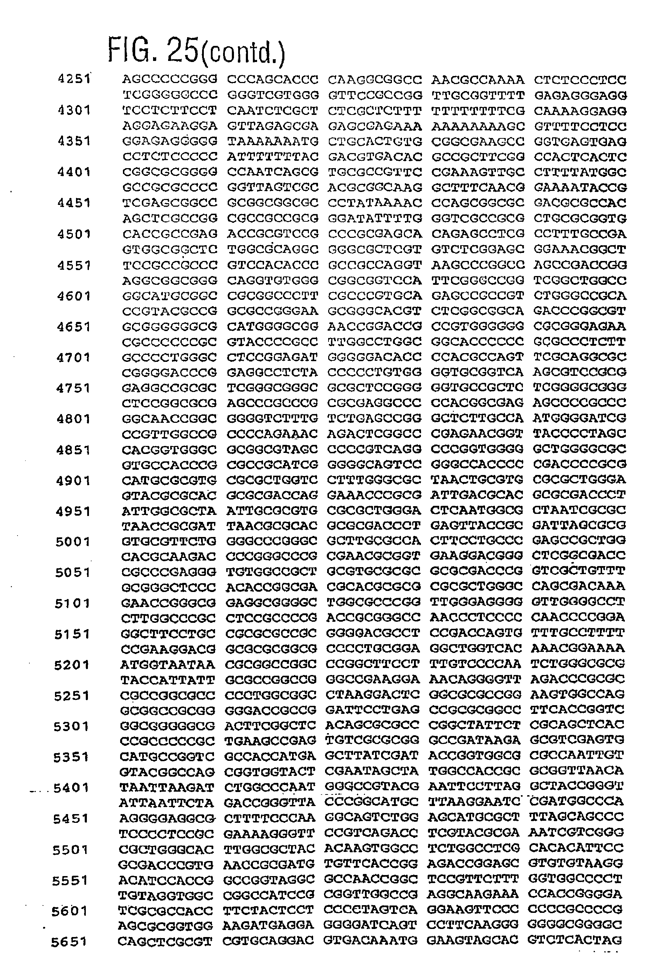

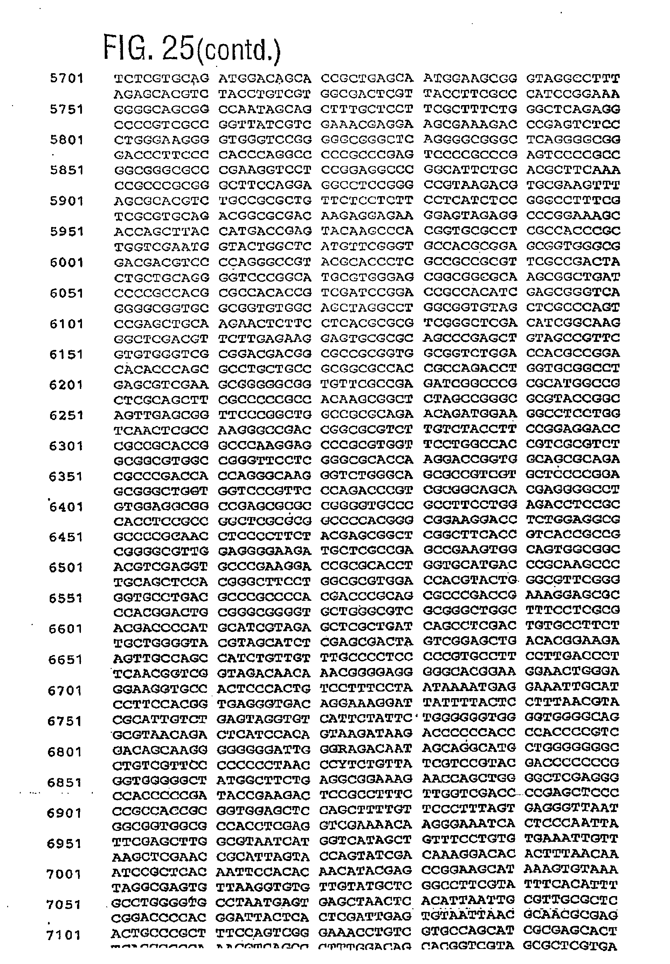

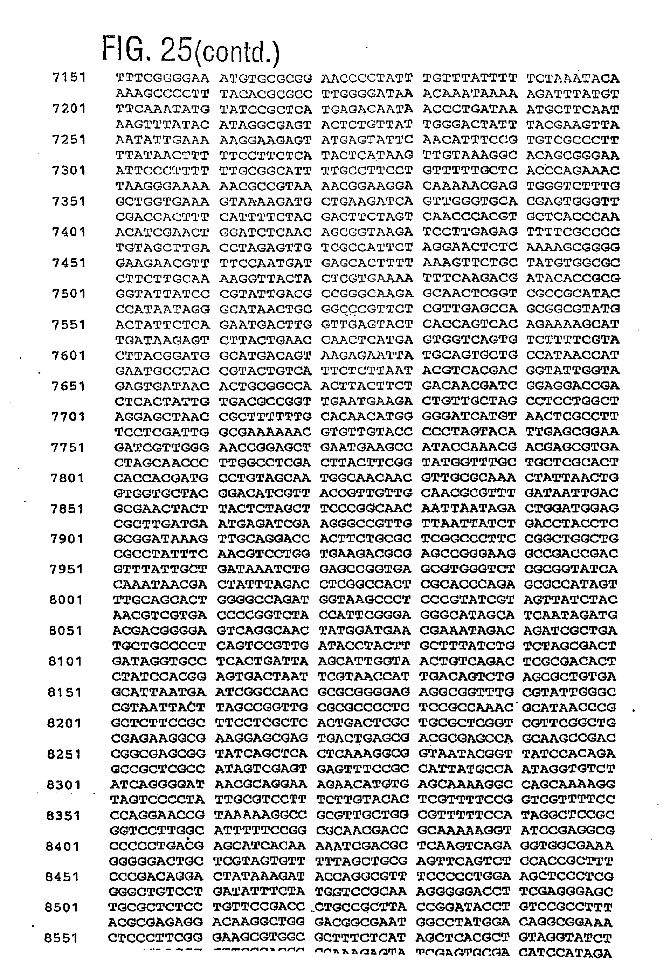

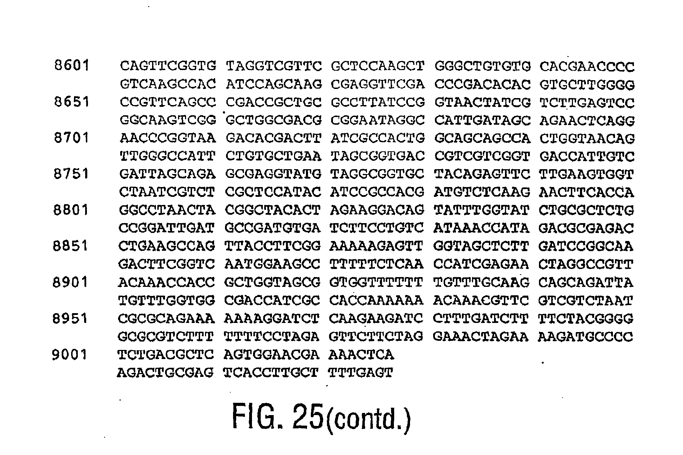

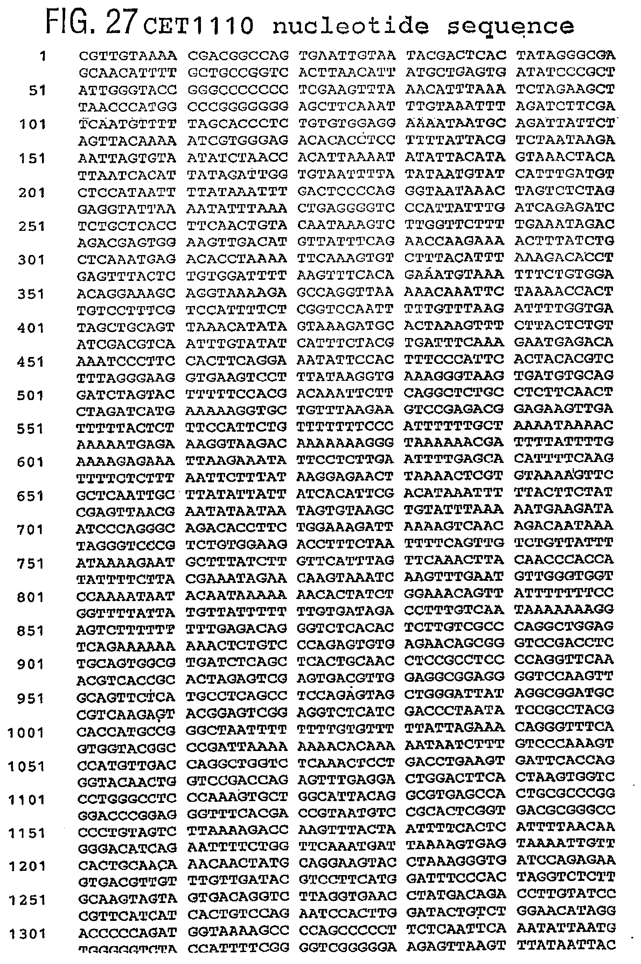

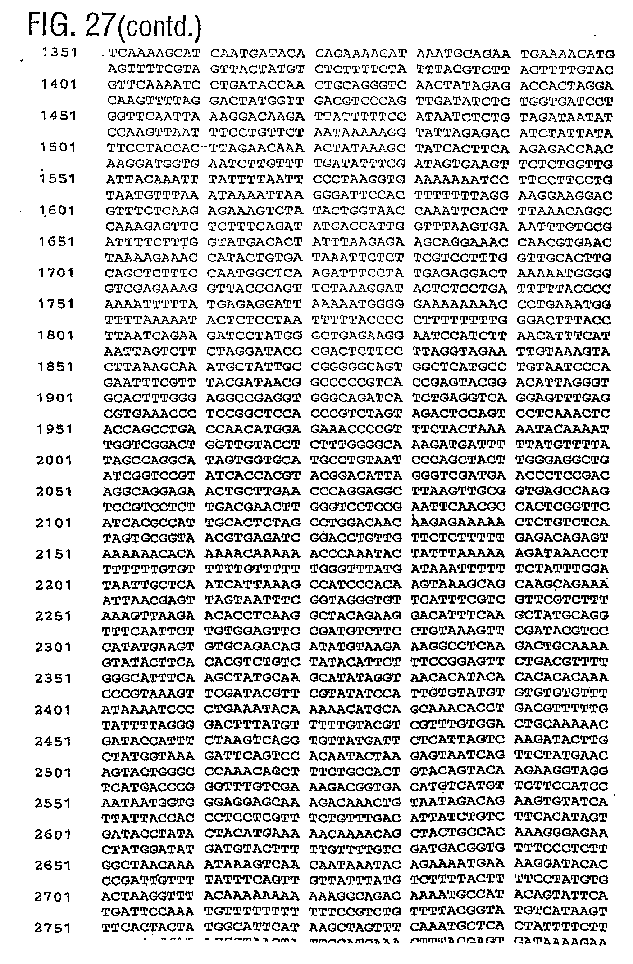

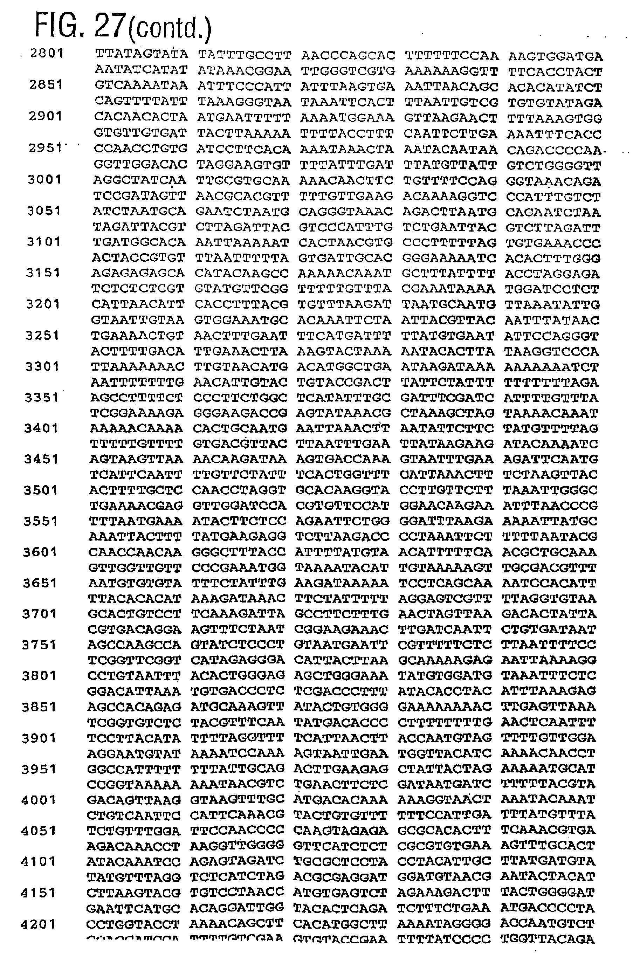

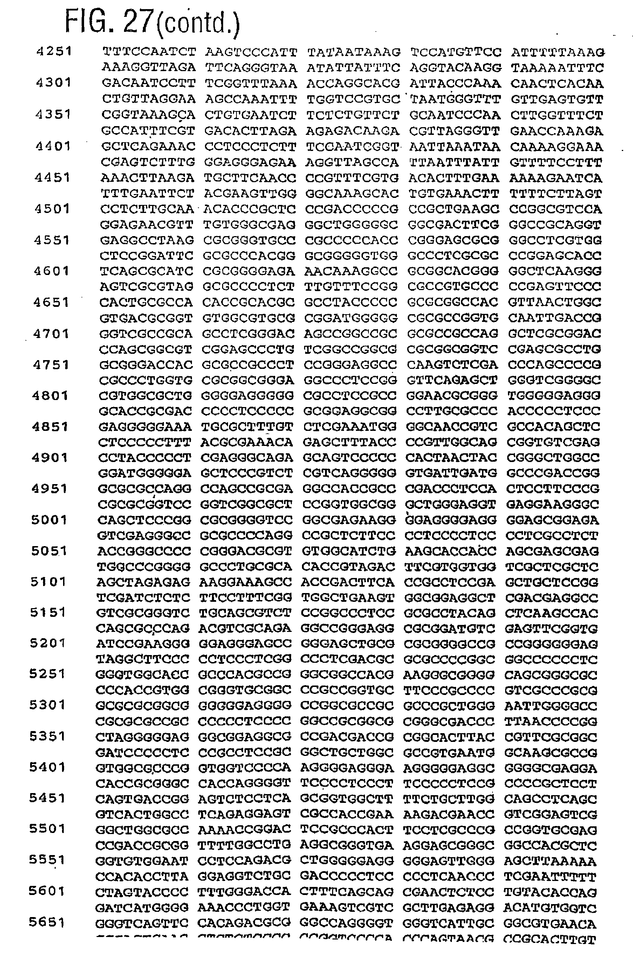

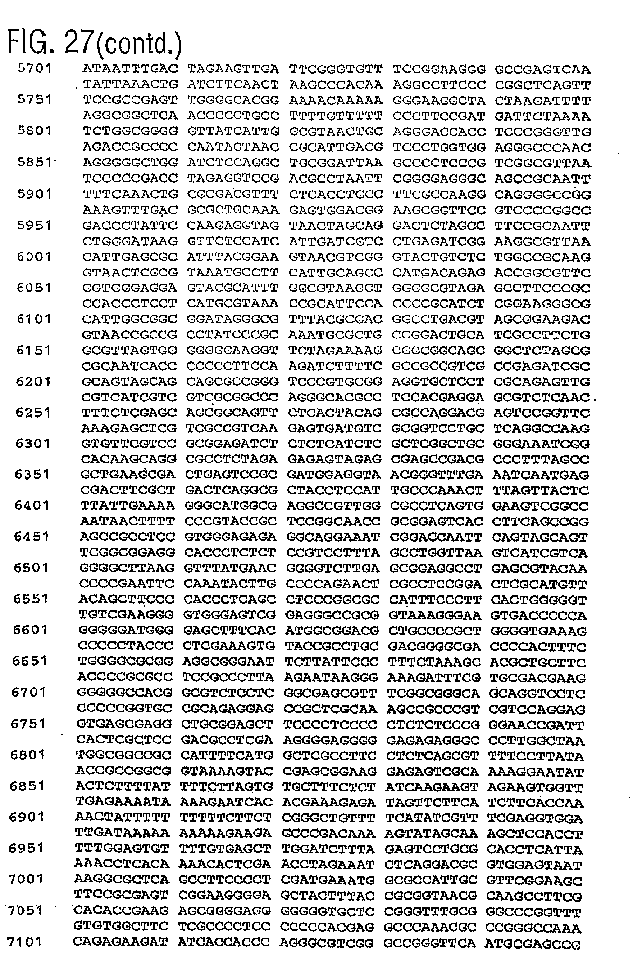

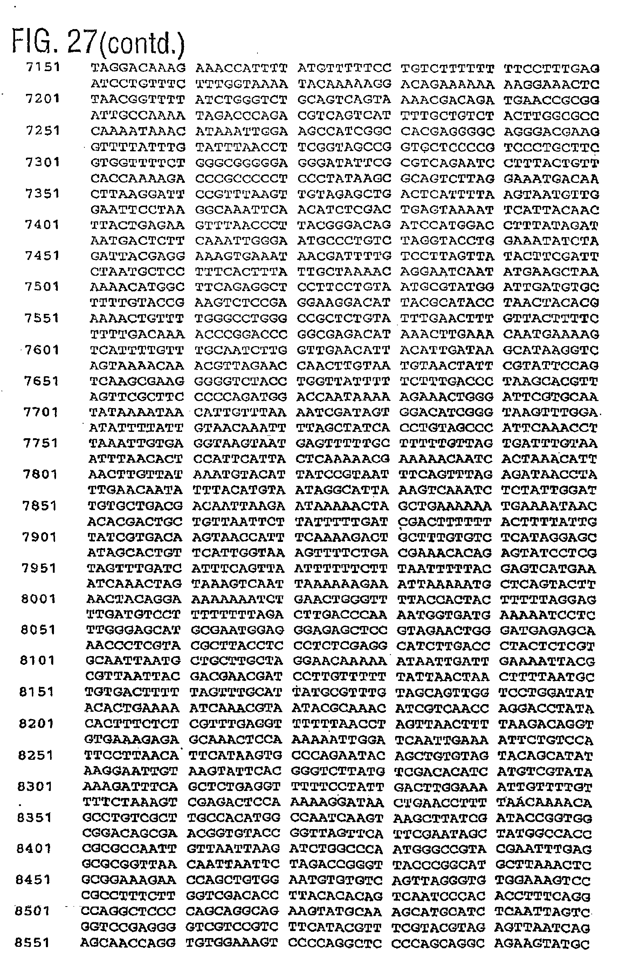

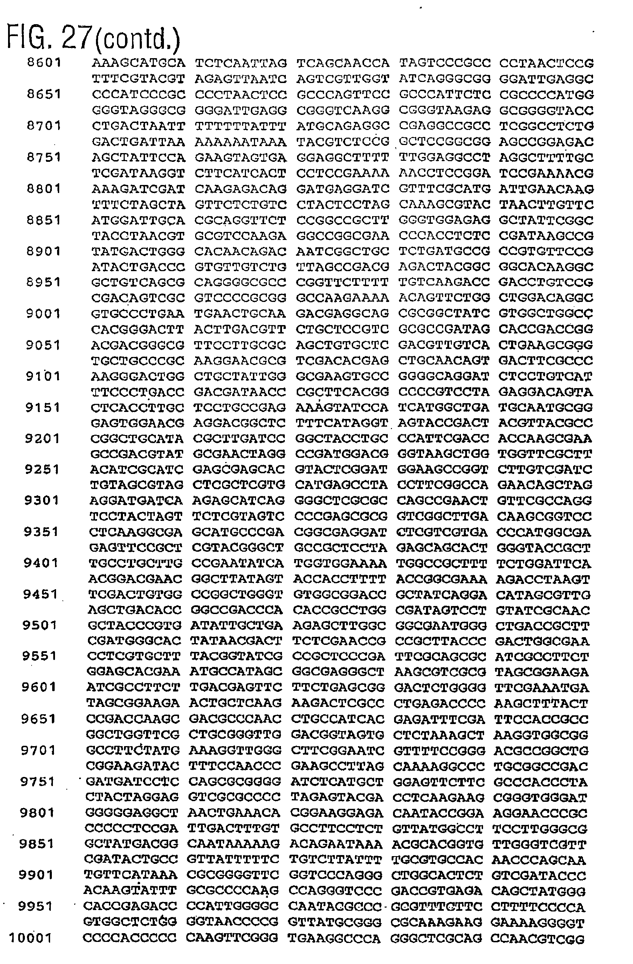

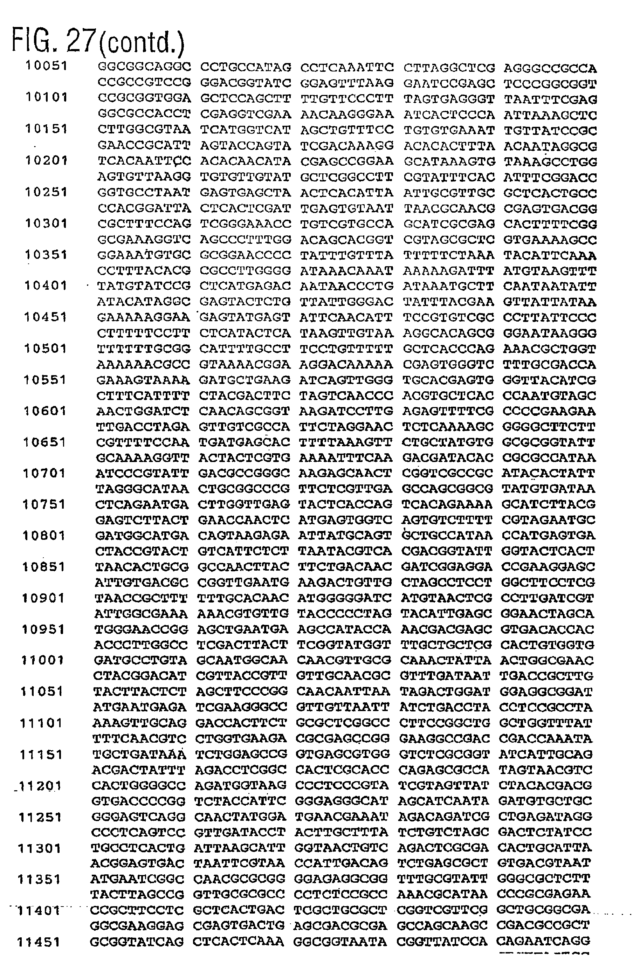

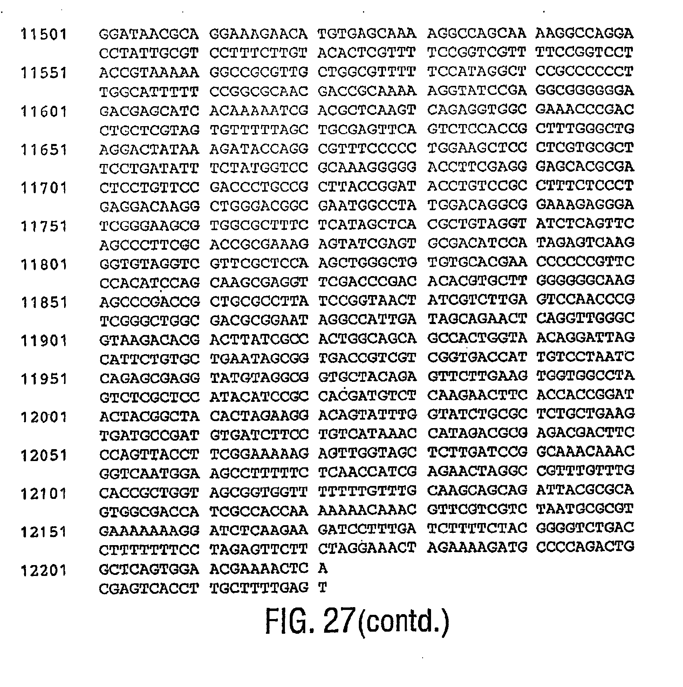

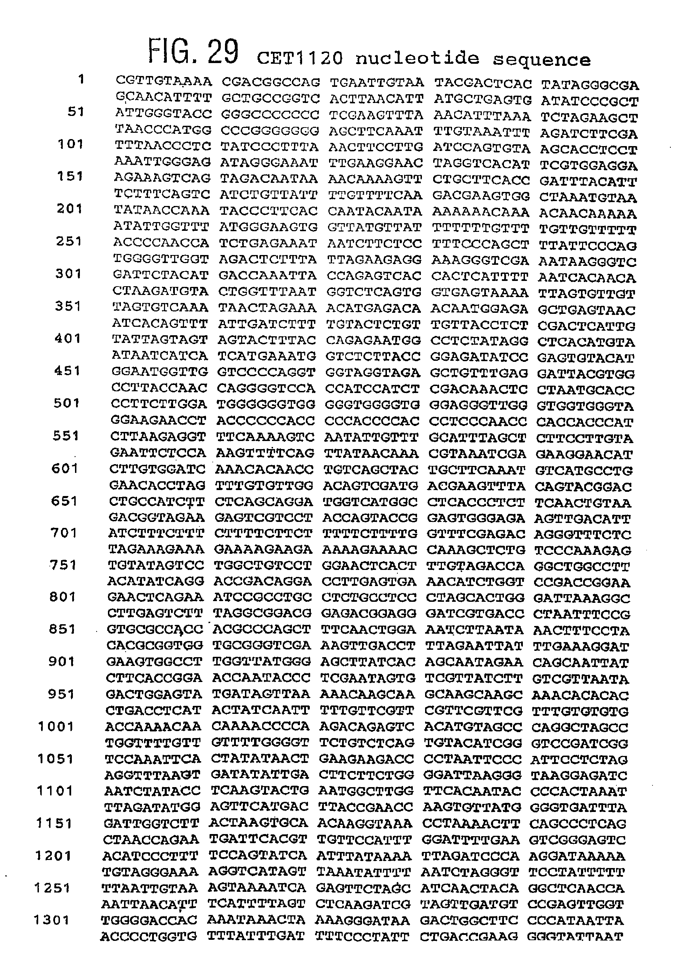

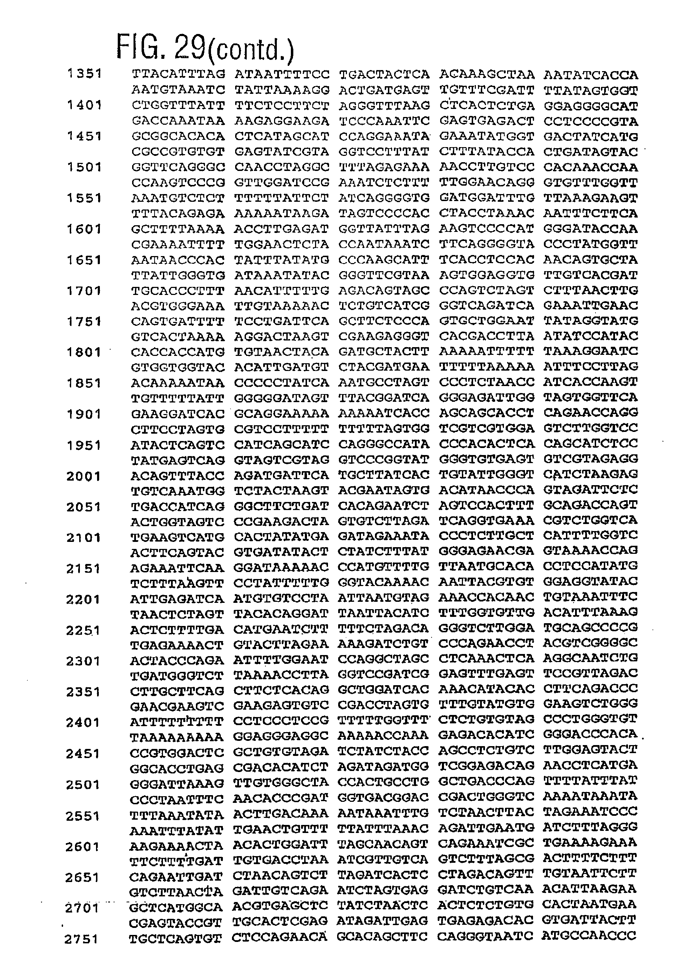

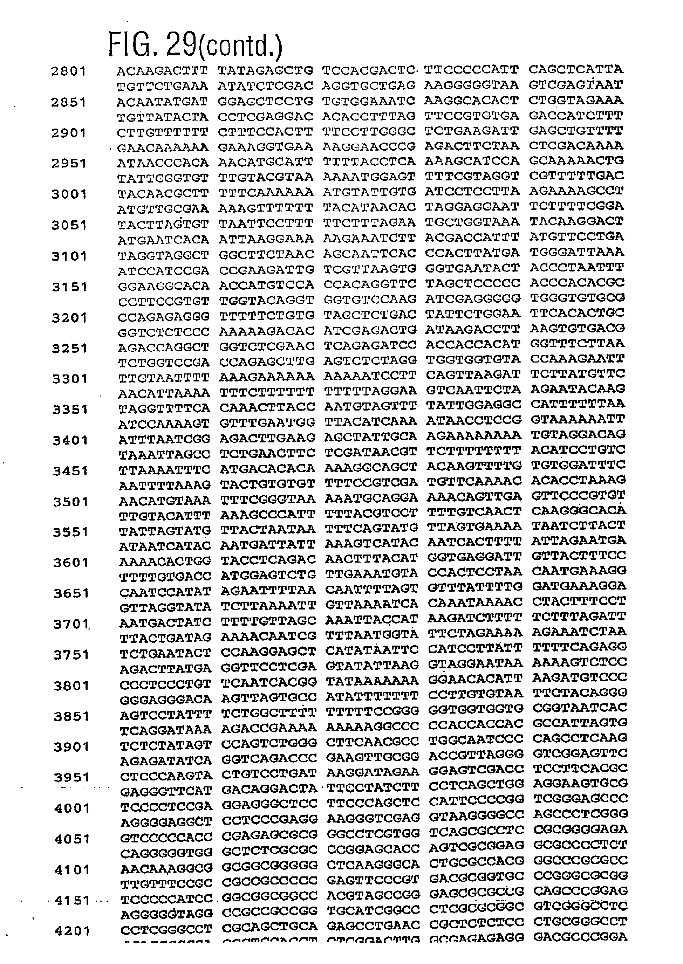

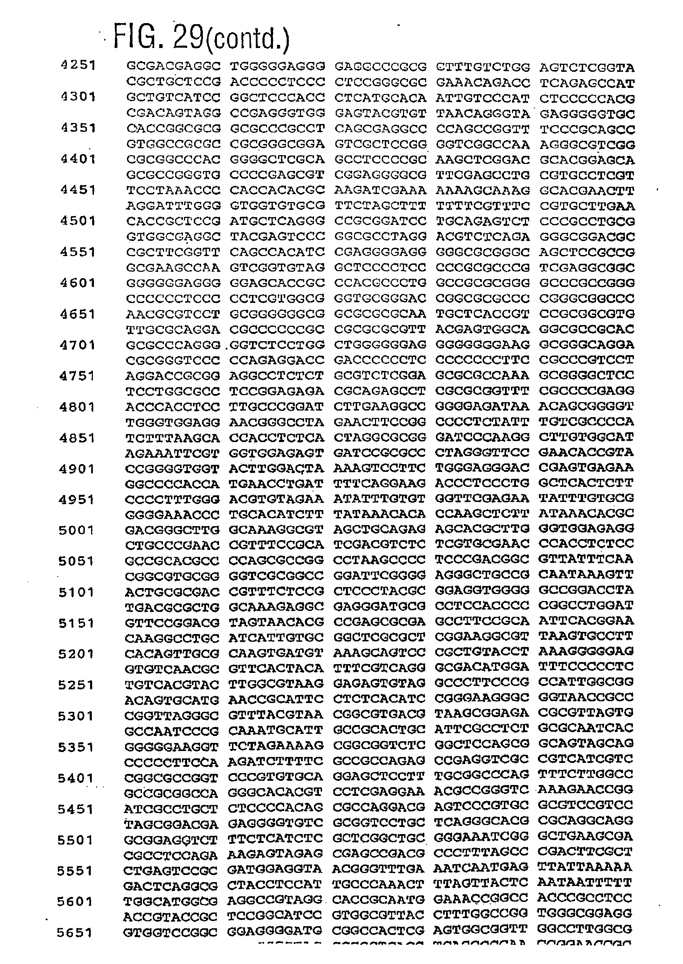

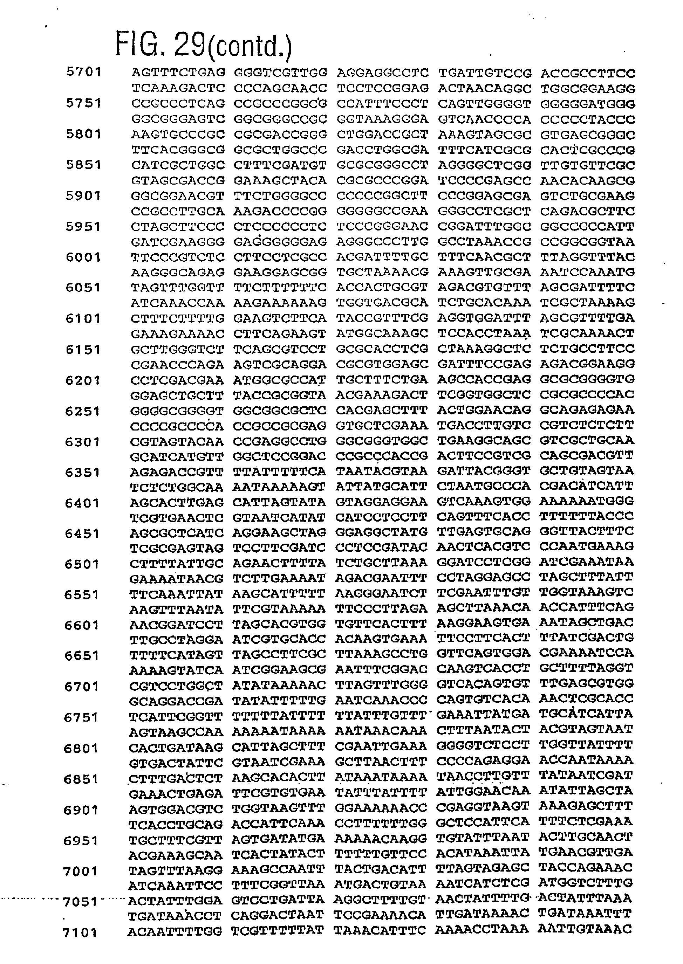

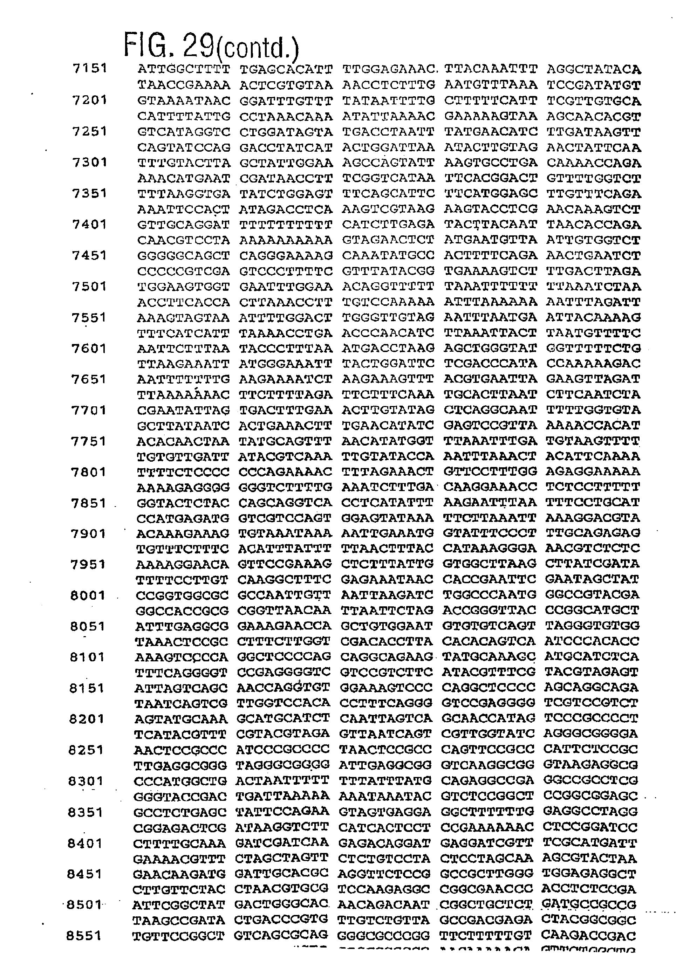

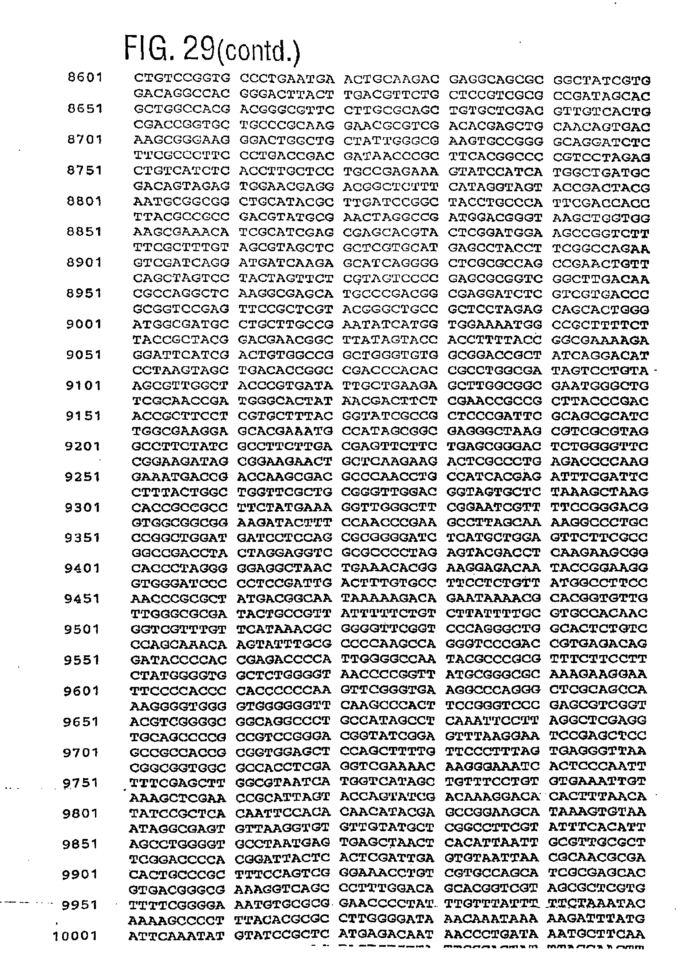

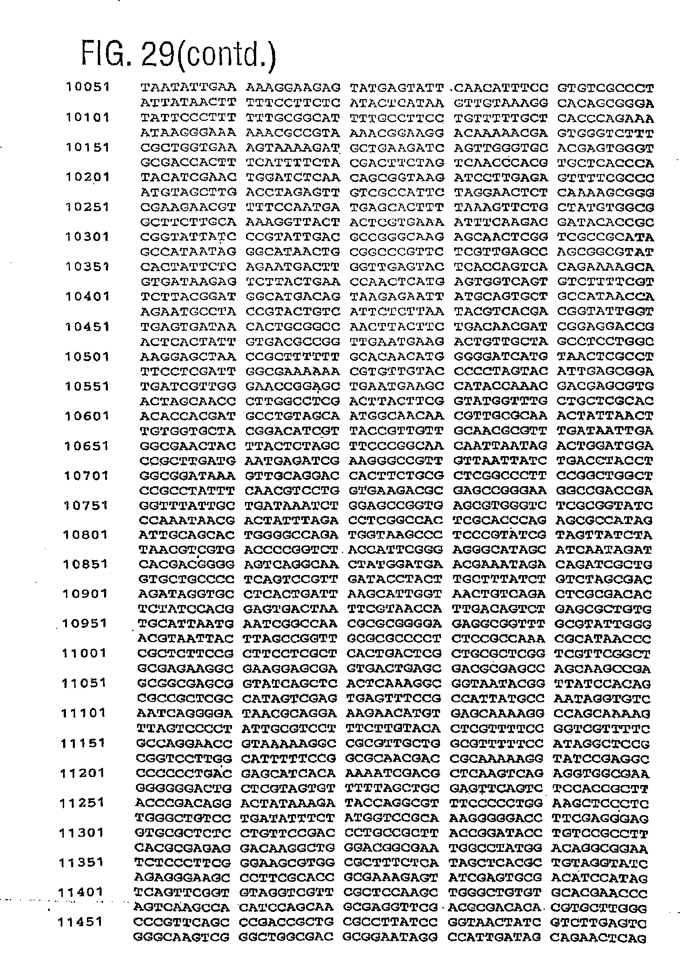

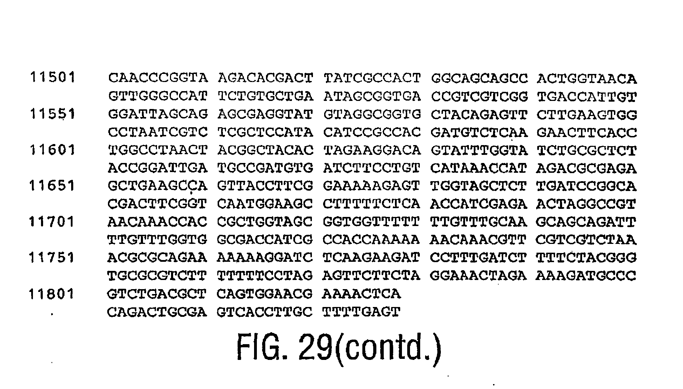

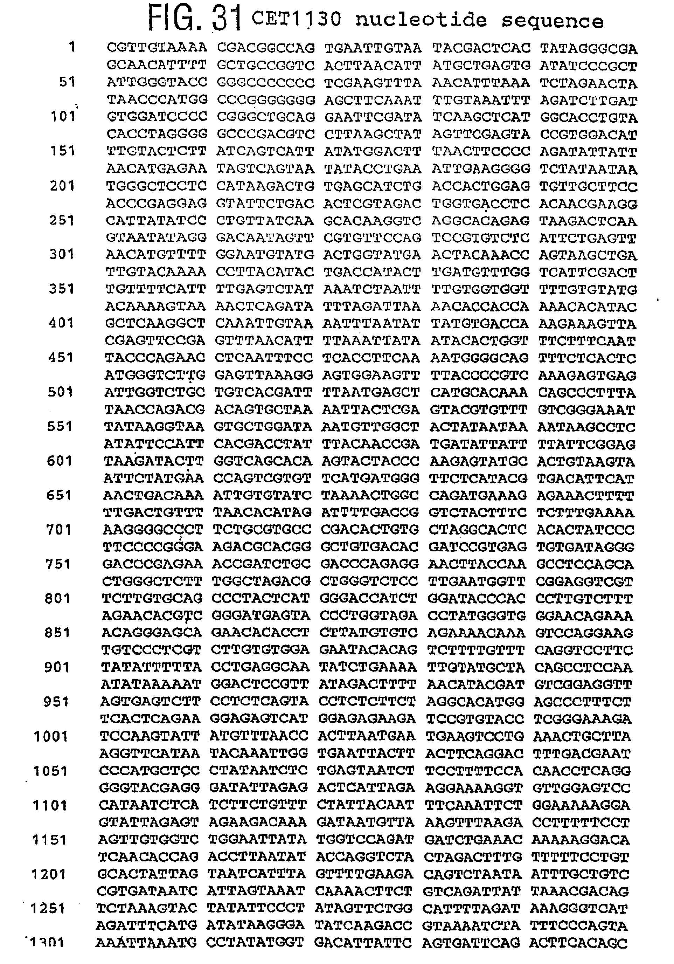

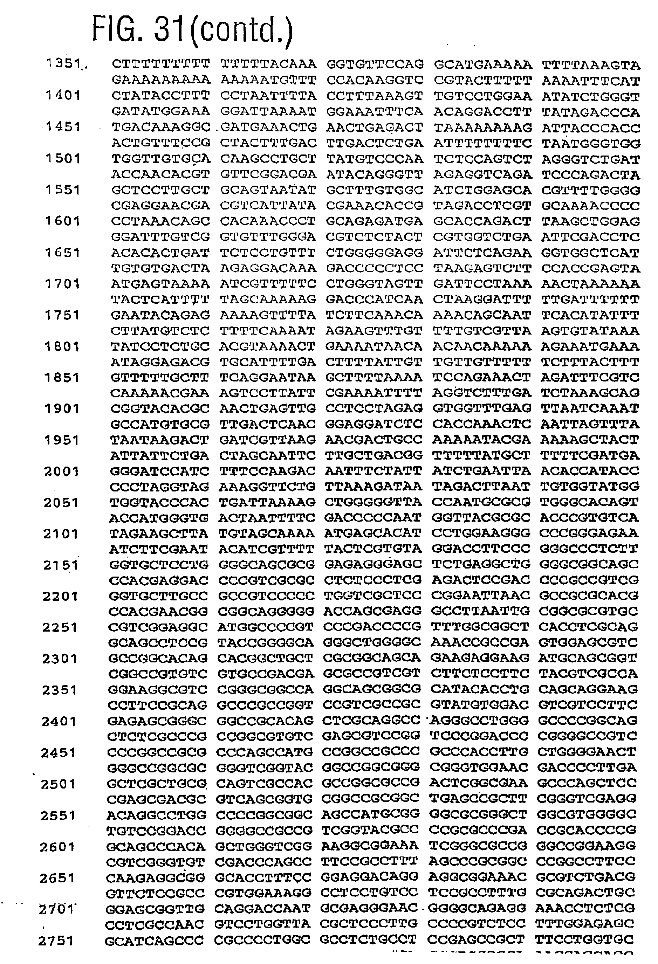

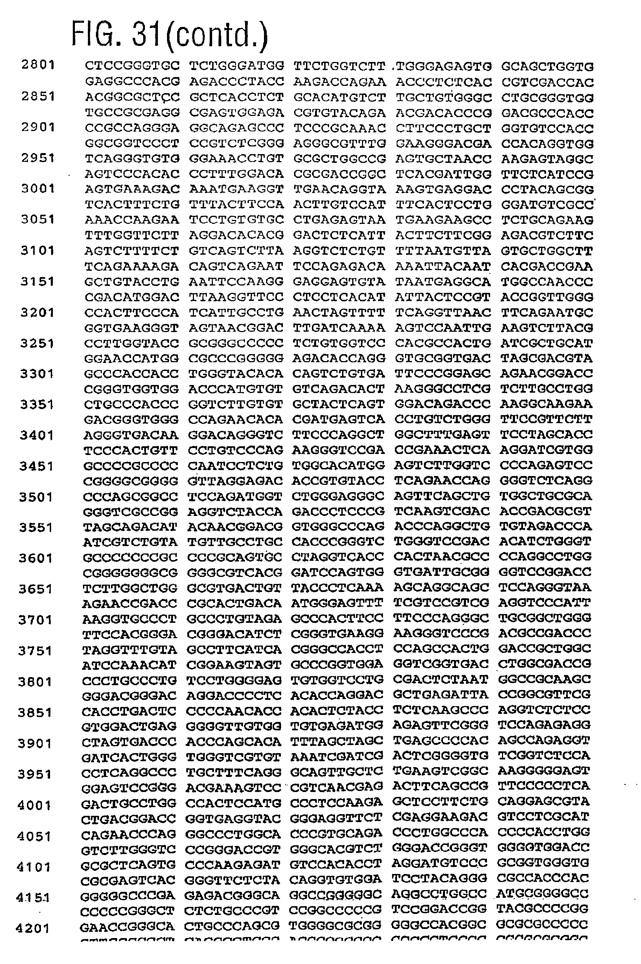

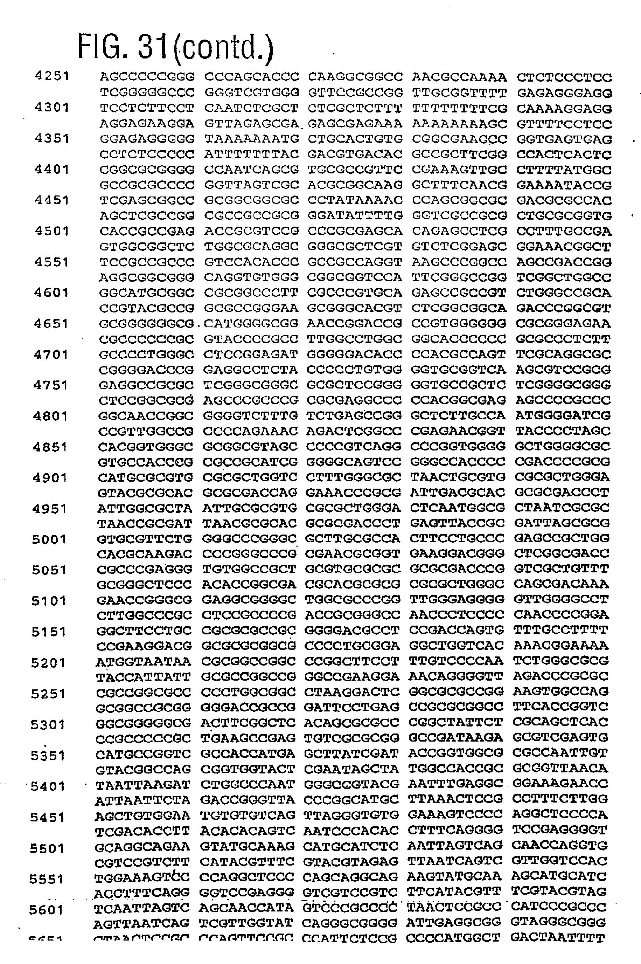

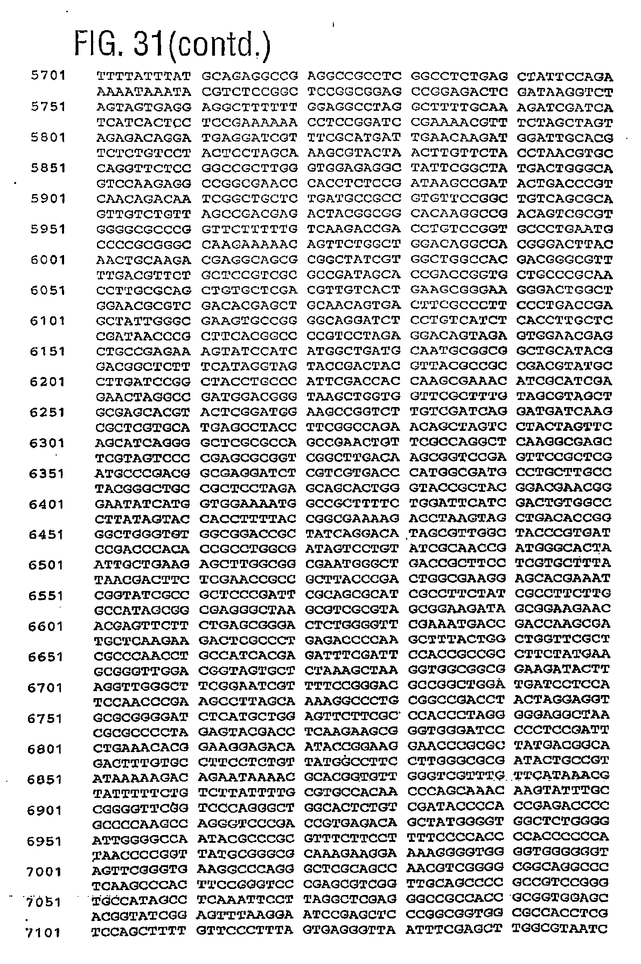

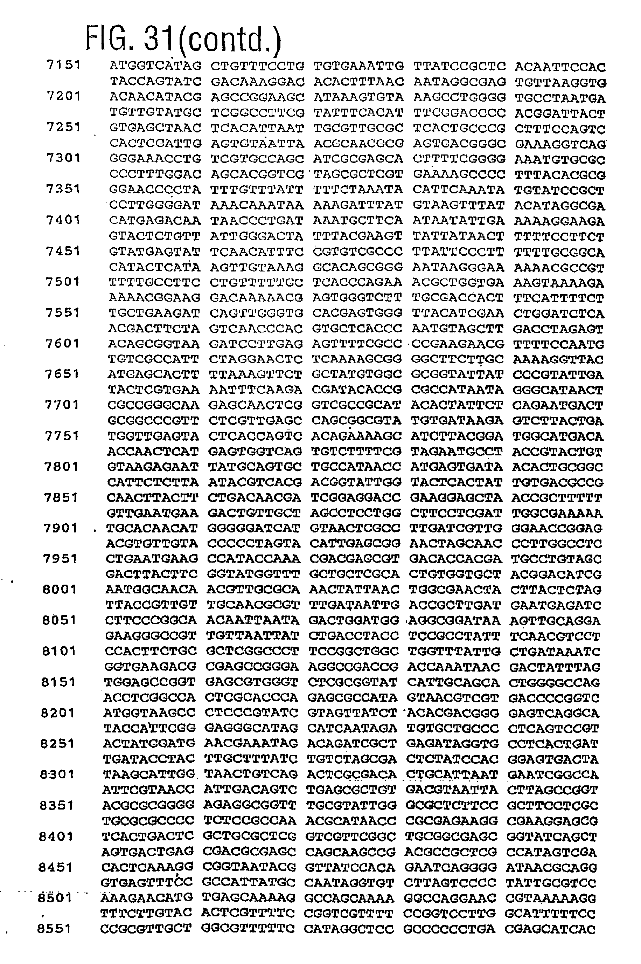

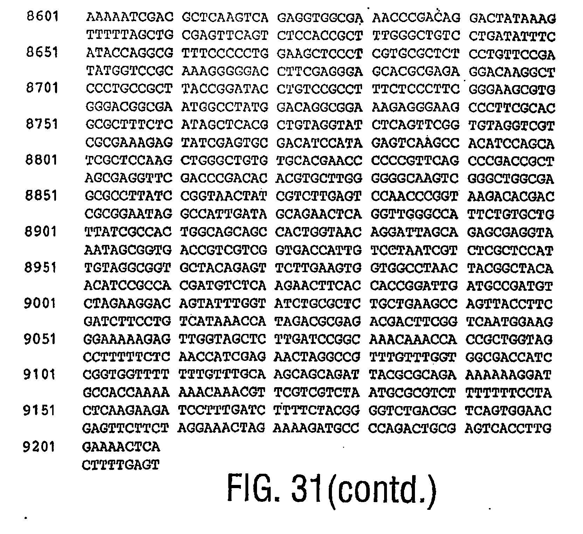

The following section of the description consists of numbered paragraphs simply providing statements of the invention already described herein. The numbered paragraphs in this section are not claims. The claims are set forth below in the later section headed "claims".
1. An isolated polynucleotide comprising:
   a. an extended methylation-free CpG island
   b. an expressible nucleic acid terminated by a polyadenylation signal.
   c. a selectable marker operably linked to a promoter
   wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 2000bp of the proximal end of the selectable marker.
2. An isolated polynucleotide comprising:
   a. an extended methylation-free CpG island
   b. an expressible nucleic acid terminated by a polyadenylation signal
   c. a selectable marker operably linked to a promoter
   wherein both the CpG island and the selectable marker are operably-linked to the expressible nucleic acid, and the components are positioned in the order: extended methylation-free CpG island, expressible nucleic acid, selectable marker, In the 5' to 3' orientation with respect to the sense strand of the expressible nucleic acid, and the polyadenylation signal at the 3' end of the expressible nucleic acid Is within 1500bp of the proximal end of the selectable marker.
3. An isolated polynucleotide comprising:
   a. an extended methylation-free CpG island
   b. an expressible nucleic acid terminated by a polyadenylation signal
   c. a selectable marker operably linked to a promoter
   wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 1000bp of the proximal end of the selectable marker.
4. An isolated polynucleotide comprising:
   a. an extended methylation-free CpG island
   b. an expressible nucleic acid terminated by a polyadenylation signal
   c. a selectable marker operably linked to a promoter
   wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 500 bp of the proximal end of the selectable marker.
5. The isolated polynucleotide of any one of the above wherein the selectable marker is an antibiotic resistance gene.
6. The isolated polynucleotide of 5, wherein the antibiotic resistance gene is obtained from a *Streptomyces* species.
7. The isolated polynucleotide of either of 6 wherein the antibiotic resistance gene is a puromycin resistance gene.
8. The isolated polynucleotide of 7 wherein the puromycin resistance gene is the puromycin N-acetyl transferase gene from *Streptomyces alboniger.*
9. The isolated polynucleotide of 8 wherein the puromycin resistance gene is a modified puromycin N-acetyl transferase gene from *Streptomyces alboniger.*
10. The isolated polynucleotide of 9 comprising the sequence of Fig 14.
11. The isolated polynucleotide of 6 wherein the antibiotic resistance gene is a neomycin resistance gene.
12. The isolated polynucleotide of 11 wherein the neomycin resistance gene is the aminoglycoside phosphotransferase gene from *Streptomyces fradiae.*
13. The isolated polynucleotide of 6 wherein the antibiotic resistance gene is a hygromycin resistance gene.
14. The isolated polynucleotide of 13 wherein the antibiotic resistance gene is the hygromycin phosphotransferase gene from *Streptomyces hygroscopicus.*
15. The isolated polynucleotide of 6 wherein the antibiotic resistance gene is a bleomycin resistance gene.
16. The isolated polynucleotide of 15 wherein the bleomycin resistance gene is the bleomycin binding protein from *Streptomyces verticillus.*
17.The isolated polynucleotide of 15 wherein the bleomycin resistance gene is the bleomycin N-acetyltransferase from *Streptomyces verticillus.*
18. The isolated polynucleotide of 6 wherein the antibiotic resistance gene is a blasticidin resistance gene.
19.The isolated polynucleotide of 18 wherein the blasticidin resistance gene is the blasticidin S-acetyltransferase gene from *Streptomyces verticillum.*
20.The isolated polynucleotide of 5, wherein the antibiotic resistance gene is the aminocyclitol phosphotransferase from *Escherichia coli.*
21.The isolated polynucleotide of 5, wherein the antibiotic resistance gene is the neomycin phosphotransferase gene from transposon Tn5.
22.The isolated polynucleotide of any one of the above wherein the extended methylation-free CpG island comprises an 8kb DNA fragment spanning the human hnRNP A2 gene.
23.The isolated polynucleotide of any one of 1-21 wherein the extended methylation-free CpG island comprises an 8kb DNA fragment spanning the murine hnRNP A2 gene.
24. The isolated polynucleotide of 23 wherein the extended methylation-free CpG island comprises nucleotides 1-7898 of the sequence of Figure 19.
25.The isolated polynucleotide of any one of 1-21, wherein the extended methylation-free CpG island comprises a 2.0 kb DNA fragment spanning the human β-actin CpG island/ promoter region and a 1.8 kb DNA fragment spanning the human PDCD2 CpG island/ promoter region.
26.A vector comprising the polynucleotide of any one of the previous claims.
27.A vector so constructed as to deliver, when linearised and integrated Into a chromosome, the polynucleotide of any one of 1-25.
28.The vector of 26, wherein the vector is an episomal vector.
29. The vector of 26, wherein the vector is an integrating vector.
30. The vector of 26, wherein the vector is a plasmid.
31.The vector of any one of 26-30, wherein the expressible nucleic acid is a therapeutic nucleic acid.
32. The vector of any one of 26-30, wherein the expressible nucleic acid encodes a recombinant protein for expression in an *In vitro* cell culture system.
33. A vector comprising:
   a. an extended methylation-free CpG island
   b. a multiple cloning site
   c. an antibiotic resistance gene obtained from a *streptomyces* species
   wherein both the CpG island and the selectable marker are operably-linked to the expressible nucleic acid, and the components are positioned in the order: extended methylation-free CpG island, multiple cloning site, selectable marker, in the 5' to 3' orientation with respect to the sense strand of the expressible nucleic acid, and the multiple cloning site is within 2000bp of the proximal end of the selectable marker.
34. The vector of 33 wherein the multiple cloning site is further operably linked to a promoter.
35. The vector of 34 wherein said promoter is a cytomegalovirus immediate/early promoter.
36. The vector of any one of 26-31 comprising nucleotides 1-10551 of the sequence of Figure 10.
37.The vector CET 710.
38. The vector of any one of 26-31 comprising nucleotides 1-13545 of the sequence of Figure 12.
39. The vector CET 720.
40. The vector CET 740.
41. The vector CET 760.
42. The vector CET 780.
43. The vector CET 820.
44. The vector CET 823.
45. The vector comprising nucleotides 1-12039 of the sequence of Figure 21.
46. The vector CET 1010
47. The vector comprising nucleotides 1-11646 of the sequence of Figure 23.
48. The vector CET 1020.
49. The vector comprising nucleotides 1-9027 of the sequence of Figure 25.
50.The vector CET 1030
51. The vector comprising nucleotides 1-12221 of the sequence of Figure 27.
52. The vector CET 1110
53. The vector comprising nucleotides 1-11828 of the sequence of Figure 29.
54. The vector CET 1120
55.The vector comprising nucleotides 1-9209 of the sequence of Figure 31.
56. The vector CET 1130
57. A host cell transfected with the vector of any of 26-56.
58. Use of the polynucleotide of any of 1-25, the vector of 26-56, or the host cell of 57 to obtain expression of an expressible nucleic acid.
59. Use of the polynucleotide of any of 1-25, the vector of 26-56, or the host cell of 57 in cell culture system to obtain expression of a desired gene product.
60. Use of the polynucleotide of any of 1-25, the vector of 26-56, or the host cell of claim 57 for therapy
61. Use of the polynucleotide of any of 1-25, the vector of 26-567, or the host cell of 57 for the manufacture of a composition for use in gene therapy.
62. A method of treatment, comprising the administering to a patient in need of such treatment a pharmaceutically effective amount of the polynucleotide of any of 1-25, the vector of 26-56, or the host cell of 57.
63. A pharmaceutical composition comprising the polynucleotide of any of 1-25, the vector of 26-56, or the host cell of 57 in combination with a pharmaceutically acceptable excipient.
64.A non-human transgenic animal comprising an artificially introduced extended methylation-free CpG island element and an artificially introduced selectable marker, wherein both the CpG island and the selectable marker are operably-linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 2000bp of the proximal end of the selectable marker.

## Claims

1. An isolated linear polynucleotide for integration into a chromosome comprising:
a. an extended methylation-free CpG island
b. an expressible nucleic acid terminated by a polyadenylation signal
c. a selectable marker operably linked to a promoter
wherein both the CpG island and the selectable marker are operably linked to the expressible nucleic acid, and the components are positioned in the order: extended methylation-free CpG island, expressible nucleic acid, selectable marker, in the 5' to 3' orientation with respect to the sense strand of the expressible nucleic acid, and the polyadenylation signal at the 3' end of the expressible nucleic acid is within 2000bp of the proximal end of the selectable marker.

2. An isolated linear polynucleotide according to claim 1, wherein the polyadenylation signal at the 3' end of the expressible nucleic acid is within 1500bp of the proximal end of the selectable marker.

3. An isolated linear polynucleotide according to claim 2, wherein the polyadenylation signal at the 3' end of the expressible nucleic acid is within I000bp of the proximal end of the selectable marker.

4. An isolated linear polynucleotide according to claim 3, wherein the polyadenylation signal at the 3' end of the expressible nucleic acid is within 500 bp of the proximal end of the selectable marker.

5. An isolated linear polynucleotide according to any one of the above claims, wherein the selectable marker is an antibiotic resistance gene.

6. An isolated linear polynucleotide according to claim 5, wherein the antibiotic resistance gene is obtained from a *Streptomyces* species.

7. An isolated linear polynucleotide according to claim 5, wherein the antibiotic resistance gene is selected from the group consisting of :
(i) a puromycin resistance gene, optionally the puromycin N-acetyl transferase gene or a modified purornycin N-acetyl transferase gene from *Streptomyces alboniger;*
(ii) a neomycin resistance gene, optionally the aminoglycoside phosphotransferase gene from *Streptomyces fradiae;*
(iii) a hygromycin resistance gene, optionally the hygromycin phosphotransferase gene from *Streptomyces hygroscopicus;*
(iv) a bleomycin resistance gene, optionally the bleomycin binding protein or the bleomycin N-acetyltransferase from *Streptomyces verticillus;*
(v) a blasticidin resistance gene, optionally the the blasticidin S-acetyltransferase gene from *Streptomyces verticillum.*

8. An isolated linear polynucleotide according to claim 5, wherein the antibiotic resistance gene is the aminocyclitol phosphotransferase from Escherichia coli.

9. An isolated linear polynucleotide according to claim 5, wherein the antibiotic resistance gene is the neomycin phosphotransferase gene from transposon Tn5.

10. An isolated linear polynucleotide according to any one of the above claims, wherein the extended methylation-free CpG island comprises an 8kb DNA fragment spanning the human hnRNP A2 gene.

11. An isolated linear polynucleotide according to any one of claims 1 to 9, wherein the extended methylation-free CpG island comprises an 8kb DNA fragment spanning the murine hnRNP A2 gene.

12. An isolated linear polynucleotide according to claim 11, wherein the extended methylation-free CpG island comprises nucleotides 1-7898 of the
sequence of Figure 19.

13. An isolated linear polynucleotide according to any one of claims 1 to 9, wherein the extended methylation-free CpG island comprises a 2.0 kb DNA fragment spanning the human β-actin CpG island/ promoter region and a 1.8 kb DNA fragment spanning the human PDCD2 CpG island/ promoter region.

14. An isolated linear polynucleotide according to any one of claims 1 to 13 for use as a medicament.

15. A pharmaceutical composition comprising the polynucleotide of any one of claims 1 to 13.

16. Use of the isolated polynucleotide according to any one of claims 1 to 13 to obtain expression of a desired gene product.

17. Use of the isolated polynucleotide according to claim 16, wherein the desired gene product is a therapeutic protein.

## Patentansprüche

1. Isoliertes, lineares Polynucleotid zur Integration in ein Chromosom, wobei das Polynucleotid folgendes umfasst:
a) eine verlängerte, nicht-methylierte CpG-Insel;
b) eine exprimierbare Nucleinsäure, terminiert durch ein Polyadenylationssignal;
c) einen wählbaren Marker, der funktionsfähig mit einem Promotor verknüpft ist,
wobei sowohl die CpG-Insel als auch der wählbare Marker funktionsfähig mit der exprimierbaren Nucleinsäure verknüpft sind, und wobei die Komponenten in der folgenden Reihenfolge angeordnet sind: verlängerte, nicht-methylierteCpG-Insel, exprimierbare Nucleinsäure, wählbarer Marker, in der 5` bis 3` Ausrichtung in Bezug auf den Sinnstrang der exprimierbaren Nucleinsäure, und wobei das Polyadenylationssignal an dem 3' Ende der exprimierbaren Nucleinsäure innerhalb von 2000 bp des proximalen Endes des wählbaren Markers liegt.

2. Isoliertes, lineares Polynucleotid nach Anspruch 1, wobei das Polyadenylationssignal an dem 3' Ende der exprimierbaren Nucleinsäure innerhalb von 1500 bp des proximalen Endes des wählbaren Markers liegt.

3. Isoliertes, lineares Polynucleotid nach Anspruch 1, wobei das Polyadenylationssignal an dem 3' Ende der exprimierbaren Nucleinsäure innerhalb von 1000 bp des proximalen Endes des wählbaren Markers liegt.

4. Isoliertes, lineares Polynucleotid nach Anspruch 1, wobei das Polyadenylationssignal an dem 3' Ende der exprimierbaren Nucleinsäure innerhalb von 500 bp des proximalen Endes des wählbaren Markers liegt.

5. Isoliertes, lineares Polynucleotid nach einem der vorstehenden Ansprüche, wobei es sich bei dem wählbaren Marker um ein Antibiotika-Resistenzgen handelt.

6. Isoliertes, lineares Polynucleotid nach Anspruch 5, wobei das Antibiotika-Resistenzgen aus einer *Streptomyces-Spezies* erhalten wird.

7. Isoliertes, lineares Polynucleotid nach Anspruch 5, wobei das Antibiotika-Resistenzgen aus der Gruppe ausgewählt wird, die folgendes umfasst:
(i) ein Puromycin-Resistenzgen, optional das Puromycin-N-Acetyltransferasegen oder ein modifiziertes Puromycin-N-Acetyltransferasegen aus *Streptomyces alboniger;*
(ii) ein Neomycin-Resistenzgen, optional das Aminoglycosid-Phosphotransferasegen aus *Streptomyces fradiae;*
(iii) ein Hygromycin-Resistenzgen, optional das Hygromycin-Phosphotransferasegen aus *Streptomyces hygroscopicus;*
(iv) ein Bleomycin-Resistenzgen, optional das Bleomycin-Bindungsprotein oder die Bleomycin-N-Acetyltransferase aus *Streptomyces verticillus;*
(v) ein Blasticidin-Resistenzgen, optional das Blasticidin-S-Acetyltransferasegen aus *Streptomyces verticillum.*

8. Isoliertes, lineares Polynucleotid nach Anspruch 5, wobei es sich bei dem Antibiotika-Resistenzgen um die Aminocyclitol-Phosphotransferase aus Escherichia coli handelt.

9. Isoliertes, lineares Polynucleotid nach Anspruch 5, wobei es sich bei dem Antibiotika-Resistenzgen um das Neomycin-Phosphotransferasegen aus Transposon Tn5 handelt.

10. Isoliertes, lineares Polynucleotid nach einem der vorstehenden Ansprüche, wobei die verlängerte, nicht-methylierteCpG-Insel ein 8kb DNA-Fragment umfasst, welches das menschliche hnRNP A2-Gen umspannt.

11. Isoliertes, lineares Polynucleotid nach einem der Ansprüche 1 bis 9, wobei die verlängerte, nicht-methylierteCpG-Insel ein 8kb DNA-Fragment umfasst, welches das mausartige hnRNP A2-Gen umfasst.

12. Isoliertes, lineares Polynucleotid nach Anspruch 11, wobei die verlängerte, nicht-methylierte CpG-Insel die Nucleotide 1-7898 der Sequenz aus Abbildung 19 umfasst.

13. Isoliertes, lineares Polynucleotid nach Anspruch 11, wobei die verlängerte, nicht-methylierte CpG-Insel ein 2,0kb DNA-Fragment umfasst, welches die menschliche β-Actin CpG-Insel/Promotor-Regionumspannt, und ein 1,8kb DNA-Fragment, welches die menschliche PDCD2 CpG-Insel/Promoor-Region umspannt.

14. Isoliertes, lineares Polynucleotid nach einem der Ansprüche 1 bis 13 zur Verwendung als ein Arzneimittel.

15. Pharmazeutische Zusammensetzung, welche die Polynucleotide nach einem der Ansprüche 1 bis 13 umfasst.

16. Einsatz des isolierten Polynucleotids nach einem der Ansprüche 1 bis 13, um eine Expression eines gewünschten Genprodukts zu erhalten.

17. Einsatz des isolierten Polynucleotids nach Anspruch 16, wobei es sich bei dem gewünschten Genprodukt um ein therapeutisches Protein handel.

## Revendications

1. Polynucléotide linéaire isolé pour l'intégration dans un chromosome comprenant :
a. un îlot CpG étendu exempt de méthylation
b. un acide nucléique exprimable terminé par un signal de polyadénylation
c. un marqueur sélectionnable lié de manière opérationnelle à un promoteur,
dans lequel l'îlot CpG et le marqueur sélectionnable sont tous deux liés de manière opérationnelle à l'acide nucléique exprimable, et les composants sont positionnés dans l'ordre : îlot CpG étendu exempt de méthylation, acide nucléique exprimable, marqueur sélectionnable, dans l'orientation 5' vers 3' par rapport au brin sens de l'acide nucléique exprimable, et le signal de polyadénylation à l'extrémité 3' de l'acide nucléique exprimable se situe dans les 2000 pb de l'extrémité proximale du marqueur sélectionnable.

2. Polynucléotide linéaire isolé selon la revendication 1, dans lequel le signal de polyadénylation à l'extrémité 3' de l'acide nucléique exprimable se situe dans les 1500 pb de l'extrémité proximale du marqueur sélectionnable.

3. Polynucléotide linéaire isolé selon la revendication 2, dans lequel le signal de polyadénylation à l'extrémité 3' de l'acide nucléique exprimable se situe dans les 1000 pb de l'extrémité proximale du marqueur sélectionnable.

4. Polynucléotide linéaire isolé selon la revendication 3, dans lequel le signal de polyadénylation à l'extrémité 3' de l'acide nucléique exprimable se situe dans les 500 pb de l'extrémité proximale du marqueur sélectionnable.

5. Polynucléotide linéaire isolé selon l'une quelconque des revendications ci-dessus, dans lequel le marqueur sélectionnable est un gène de résistance aux antibiotiques.

6. Polynucléotide linéaire isolé selon la revendication 5, dans lequel le gène de résistance aux antibiotiques est obtenu à partir d'une espècede Streptomyces.

7. Polynucléotide linéaire isolé selon la revendication 5, dans lequel le gène de résistance aux antibiotiques est sélectionné parmi le groupe constitué de :
(i) un gène de résistance à la puromycine, en option le gène de puromycine N-acétyltransférase ou un gène de puromycine N-acétyltransférase modifié provenant de Streptomyces alboniger ;
(ii) un gène de résistance à la néomycine, en option le gène d'aminoglycoside phosphotransférase provenant de Streptomyces fradiae ;
(iii) un gène de résistance à l'hygromycine, en option le gène d'hygromycine phosphotransférase provenant de Streptomyces hygroscopicus ;
(iv) un gène de résistance à la bléomycine, en option la protéine de liaison à la bléomycine ou la bléomycineN-acétyltransférase provenantde Streptomyces verticillus ;
(v) un gène de résistance à la blasticidine, en option le gène de blasticidine S-acétyltransférase provenantde Streptomyces verticillum.

8. Polynucléotide linéaire isolé selon la revendication 5, dans lequel le gène de résistance aux antibiotiques est l'aminocyclitol phosphotransférase provenant d'Escherichia coli.

9. Polynucléotide linéaire isolé selon la revendication 5, dans lequel le gène de résistance aux antibiotiques est le gène de néomycine phosphotransférase provenant du transposon Tn5.

10. Polynucléotide linéaire isolé selon l'une quelconque des revendications ci-dessus, dans lequel l'îlot CpG étendu exempt de méthylation comprend un fragment d'ADN de 8 kb couvrant le gène hnRNP A2 humain.

11. Polynucléotide linéaire isolé selon l'une quelconque des revendications 1 à 9, dans lequel l'îlot CpG étendu exempt de méthylation comprend un fragment d'ADN de 8 kb couvrant le gène hnRNP A2 murin.

12. Polynucléotide linéaire isolé selon la revendication 11, dans lequel l'îlot CpG étendu exempt de méthylation comprend les nucléotides 1 à 7898 de la séquence de la Figure 19.

13. Polynucléotide linéaire isolé selon l'une quelconque des revendications 1 à 9, dans lequel l'îlot CpG étendu exempt de méthylation comprend un fragment d'ADN de 2,0 kb couvrant la région promotrice/îlot CpG de β-actine humaine et un fragment d'ADN de 1,8 kb couvrant la région promotrice/îlot CpG de PDCD2 humain.

14. Polynucléotide linéaire isolé selon l'une quelconque des revendications 1 à 13 destiné à être utilisé en tant que médicament.

15. Composition pharmaceutique comprenant le polynucléotide selon l'une quelconque des revendications1 à 13.

16. Utilisation du polynucléotide isolé selon l'une quelconque des revendications 1 à 13 pour obtenir l'expression d'un produit génique souhaité.

17. Utilisation du polynucléotide isolé selon la revendication16, dans laquelle le produit génique souhaité est une protéine thérapeutique.
